# EUROPEAN PATENT APPLICATION

(11) **EP 4 385 991 A1**
(43) Date of publication of application: **19.06.2024**
(21) Application number: 22856152.8
(22) Date of filing: 08.08.2022
(51) Int. Cl.: C07D 417/14, A61K 31/497

(54) **COMPOUND HAVING SHP2 PROTEIN DEGRADING ACTIVITY, AND MEDICAL USES THEREOF**

(30) Priority: 09.08.2021 KR 20210104276
(71) Applicant: Ubix Therapeutics, Inc., Seoul 05836 (KR)
(72) Inventor: LEE, Song Hee, Seoul 05823 (KR); RYU, Je Ho, Seongnam-si, Gyeonggi-do 13470 (KR); LEE, Ji Hye, Incheon 22008 (KR); AHN, Jung Min, Incheon 21984 (KR); BAE, Onnuri, Incheon 22001 (KR); HAN, Jeong Hwa, Incheon 21562 (KR); LIM, Ye Seul, Seoul 05384 (KR); KIM, Ji Young, Seongnam-si, Gyeonggi-do 13121 (KR); LEE, Soo Hyun, Anyang-si, Gyeonggi-do 14074 (KR); PARK, Ji Youn, Seongnam-si, Gyeonggi-do 13236 (KR)
(74) Representative: Engelhard, Markus
(86) International application number: PCT/KR2022/011797
(87) International publication number: WO 2023/018155

(57) **Abstract**

The present disclosure provides a compound having a specific chemical structure or a pharmaceutically acceptable salt thereof having excellent activity in terms of degrading SHP2. The present disclosure also provides a composition comprising such a compound or a pharmaceutically acceptable salt thereof. The present disclosure also provides a medical use of a compound according to the present disclosure, a salt thereof, and a composition comprising the same for the treatment or prophylaxis of SHP2-related diseases (e.g., cancer). The present disclosure also provides a method for treating or preventing a SHP2-related disease (e.g., cancer) comprising administering to a subject in need thereof an effective amount of a compound according to the present disclosure, a salt thereof, or a composition comprising the same.

## Description

### [Technical Field]

The present disclosure relates to a group of compounds having SHP2 proteolytic activity. In particular, the present disclosure relates to a group of compounds having a specific structure and having very excellent activity of degrading SHP2 protein. The present disclosure also relates to useful methods of treating diseases related to SHP2 protein using such compounds. That is, the present disclosure relates to the medical use of the compounds according to the present disclosure for treating or preventing SHP2 protein related diseases.

### [Background Art]

Src homology 2 domain-containing phosphatase (SHP2) is a protein tyrosine phosphatase. Mutations in SHP2 are prevalent in Noonan syndrome and LEOPARD syndrome. Activated mutations in SHP2 have also been identified in childhood myelomonocytic leukemia, myelodysplastic syndrome, B-cell acute lymphoblastic leukemia and acute myeloid leukemia. Somatic activating mutations in SHP2 are associated with several types of solid tumors, including lung adenocarcinoma, colon cancer, neuroblastoma, glioblastoma, melanoma, hepatocellular carcinoma, prostate cancer, and breast cancer (Bentires-Alj et al., Cancer. Res. 2004, 64, 8816-8820).

It has been demonstrated that SHP2 is involved in various signaling processes such as RAS-ERK, JAK-STAT, PI3K-AKT, NF-κB and mTOR pathways within cancer cells. In the RAS-ERK pathway, SHP2 acts as a positive regulator in the upstream to promote RAS-RAF-MEK-ERK kinase cascade signaling. Therefore, SHP2 inhibition inhibits the dephosphorylation of ERK and the oncogenic function of the RAS-RAF-MEK-ERK pathway, resulting in cell growth inhibition and apoptosis in cancer cells. SHP2 also participates in the programmed cell death pathway (PD-1/PD-L1) and contributes to immune evasion by inhibiting T cell activation. In summary, SHP2 is a very attractive cancer therapeutic target. For example, the SHP2 inhibitor SHP099 has been shown to selectively block SHP2 phosphatase activity and inhibit cancer cell growth *in vitro* and tumor growth in xenograft mouse models (Chen et al., Nature 2016, 535, 148-152; Garcia Fortanet et al., J. Med. Chem. 2016, 59, 7773-7782).

Allosteric SHP2 inhibitors have been shown to be effective in a preclinical model of Kirsten rat sarcoma (KRAS)-mutant human cancer. Ablation of the SHP2 protein may also provide an alternative and perhaps even more effective strategy for inhibiting SHP2 activity. For example, it has been demonstrated that SHP2 inactivation by CRISPR-Cas9 induces senescence and inhibits tumor growth in xenograft models of KRAS-mutant tumors. These findings provide evidence that the removal of SHP2 protein from tumor cells can be an effective therapeutic strategy for human cancers, particularly cancers with KRAS-mutations.

### [Disclosure]

### [Technical Problem]

Therefore, a problem to be solved by the present invention is to provide a compound having Src homology 2 domain-containing phosphatase (SHP2) degrading activity, a pharmaceutical composition comprising the same as an active ingredient, and a medical use for the treatment or prevention of SHP2-related diseases (preferably, cancer or tumor).

Another problem to be solved by the present invention is to provide a method for treating or alleviating SHP2-related diseases (preferably, cancer or tumor), characterized in that it degrades SHP2 and consequently lowers SHP2 activity, and it comprises administering to a patient in need of treatment, improvement or prevention of SHP2 related diseases the compound according to the present invention.

### [Technical Solution]

### Compounds of the present invention

In order to solve the above problem, one embodiment of the present invention provides a compound represented by the following Chemical Formula 1 or a pharmaceutically acceptable salt thereof. 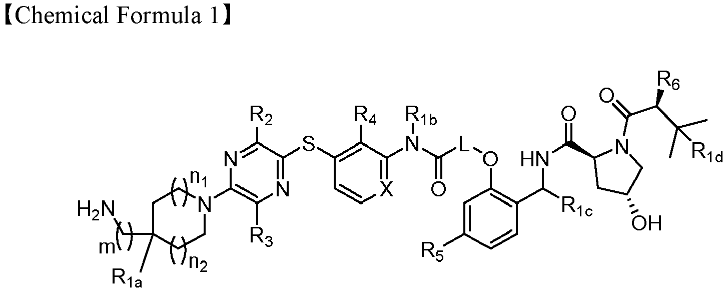 In the Chemical Formula 1,
R₁ₐ, R_{1b}, R_{1c}, and R_{1d} are each independently H or C₁₋₆alkyl (preferably methyl),
R₂ is H, C₁₋₆alkyl, or -NH₂,
R₃ is H, C₁₋₆alkyl, or C₁₋₆hydroxyalkyl,
R₄ is H, C₁₋₆alkyl, halogen (preferably Cl), or C₁₋₆haloalkyl,
R₅ is H, C₁₋₆alkyl, C₁₋₆alkynyl, halogen, -CN, or heteroaryl (preferably thiazole), wherein one or more hydrogens in the heteroaryl are optionally substituted with C₁₋₆alkyl (preferably methyl), halogen, or C₁₋₃haloalkyl,
R₆ is -NHC(O)R₇ or heteroaryl, wherein one or more hydrogens in the heteroaryl are optionally substituted with C₁₋₆alkyl, halogen, or C₁₋₃haloalkyl,
R₇ is C₁₋₆alkyl (preferably methyl), C₁₋₆haloalkyl, C₃₋₁₀cycloalkyl (preferably cyclopropyl), or heterocycle, wherein one or more hydrogens in the cycloalkyl and heterocycle are each independently and optionally substituted with C₁₋₆alkyl, halogen (preferably F), or -CN,
X is CH or N,
n₁ and n₂ is each independently 0, 1, or 2,
m is 0 or 1,
L is the following Chemical Formula 2, 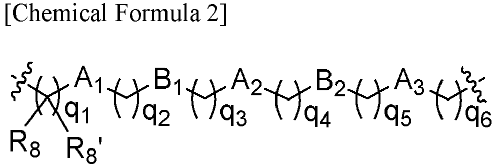 in the Chemical Formula 2,
   A₁, A₂, and A₃ are each independently direct bond, -O-, -N(R₉)- -C(O)-, -C(O)N(R₉)-, or -N(R₉)C(O)-,
   B₁ and B₂ are each independently direct bond, C₃₋₁₀cycloalkyl, heterocycle (preferably azetidine, pyrrolidine, piperidine, piperazine, azaspiro[3.5]nonane, diazabicyclo[3.2.1]octane), aryl, or heteroaryl (preferably pyridine, pyrimidine, pyridazine, pyrazine), wherein one or more hydrogens in the cycloalkyl, heterocycle, aryl, or heteroaryl are optionally substituted with C₁₋₆alkyl (preferably methyl), halogen, C₁₋₆haloalkyl, -OH, =O, or -CO₂R₉,
   R₈ and R₈' are each independently H or C₁₋₆alkyl, or R₈ and R₈' are linked to each other to form C₃₋₁₀cycloalkyl (preferably cyclopropyl),
   R₉ is H or C₁₋₆alkyl (preferably methyl),
   q₁, q₂, q₃, q₄, and q₅ are each independently an integer of from 0 to 10.

The compounds according to the present invention are PROTAC (Proteolysis-targeting chimera) compounds of 'SHP2 binding ligand - linker - E3 ligase ligand' ('Moiety located to the left of L - linker (L) - Moiety located to the right of L' in Chemical Formula 1). The present inventors have introduced various E3 ligase ligands, such as CRBN ligand, VHL ligand, and IAP ligand, to the SHP2 binding moiety of Chemical Formula 1 of the present invention. Surprisingly, among these ligands, only the VHL ligand represented by Chemical Formula 1 of the present invention had excellent effects. In addition, even in the case of using a VHL ligand, the effect was particularly excellent when the linker (L in Chemical Formula 1) was connected to a phenyl group in the VHL ligand. Although the present invention is based on this peculiar and surprising discovery, the present invention is not limited to these theoretical mechanisms.

The present inventors evaluated various substituents. In the case of the structure such as Chemical Formula 1 of the present disclosure, particularly, in the case of the preferred structure to be described later, excellent compounds in various aspects including SHP2 degradation activity could be obtained. That is, the present invention provides novel compounds having excellent SHP2 degradation activity, (metabolism) stability, etc., and excellent physicochemical properties (cLogP value, water solubility, cell membrane permeability) as an active ingredient.

As used herein, the terms "substituent", "radical", "group", "moiety", and "fragment" may be used interchangeably.

If a substituent is described as "optionally substituted", the substituent may be (1) unsubstituted or (2) substituted with one or more of the defined substituents. If the substitutable position is unsubstituted, the default substituent is hydrogen.

As used herein, the term "alkyl" means a saturated straight chain or branched non-cyclic hydrocarbon, unless the context clearly dictates otherwise, having from 1 to 10 carbon atoms. "Lower alkyl" means alkyl having from 1 to 4 carbon atoms. Representative saturated straight chain alkyls include -methyl, -ethyl, -n-propyl, -n-butyl, -n-pentyl, -n-hexyl, -n-heptyl, -n-octyl, - n-nonyl and -n-decyl, while saturated branched alkyls include -isopropyl, - sec-butyl, -isobutyl, - tert-butyl, -isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like. In a preferred embodiment of the present invention, alkyl is methyl.

As used herein, the term "alkynyl" means a straight chain or branched non-cyclic hydrocarbon, unless the context clearly dictates otherwise, having 2 to 10 carbon atoms and containing at least one carbon-carbon triple bond. Representative linear or branched (C₂-C₁₀)alkynyl is -acetylenyl, -propynyl, -1-butynyl, -2-butynyl, -1-pentynyl, -2-pentynyl, -3- methyl-1-butynyl, -4-pentynyl, -1-hexynyl, -2-hexynyl, -5-hexynyl, -1-heptynyl, -2-heptynyl, -6-heptynyl, -1-octynyl, -2-octynyl, -7-octynyl, -1-noninyl, -2-noninyl, -8-noninyl, -1-decynyl, -2-decynyl, and -9-decynyl. These alkynyl groups may be optionally substituted.

As used herein, if the term "C₁₋₆", "C1-6", or "C1-C6" is used, it means the number of carbon atoms is from 1 to 6. For example, C₁₋₆alkyl means an alkyl which carbon number is any integer of from 1 to 6.

As used herein, the terms "halogen" and "halo" mean fluorine, chlorine, bromine or iodine. In a preferred embodiment of the present invention, the halogen is chlorine or fluorine.

As used herein, the term "haloalkyl" means an alkyl group, wherein one or more hydrogen atoms are substituted with halogen atoms. For example, the haloalkyl includes -CF₃, -CHF₂, -CH₂F, -CBr₃, -CHBr₂, -CH₂Br, -CC1₃, -CHC1₂, -CH₂CI, -CI₃, -CHI₂, -CH₂I, -CH₂-CF₃, -CH₂-CHF₂, - CH₂-CH₂F, -CH₂-CBr₃, -CH₂-CHBr₂, -CH₂-CH₂Br, -CH₂-CC1₃, -CH₂-CHC1₂, -CH₂-CH₂CI, - CH₂-CI₃, -CH₂-CFH₂, -CH₂-CH₂I, and the like, wherein alkyl and halogen are as described above. In a preferred embodiment of the present invention, haloalkyl is -CF₃.

As used herein, the term "hydroxyalkyl" means linear or branched C₁₋₁₀alkyl substituted with one or more hydroxy groups. Examples of hydroxyalkyl group include hydroxymethyl, hydroxyethyl, hydroxypropyl and hydroxybutyl, but is not limited thereto.

As used herein, the term "cycloalkyl" means a monocyclic or polycyclic saturated ring having carbon and hydrogen atoms and having no carbon-carbon multiple bonds. Examples of monocyclic rings include, but are not limited to, (C₃-C₇)cycloalkyl groups, including cyclopropyl, cyclobutyl, cyclopentyl, cyclohexyl, and cycloheptyl. Examples of polycyclic rings include, but are not limited to, fused bicyclic rings such as octahydropentalene and decahydronaphthalene; spiro rings such as spiro[3.3]heptane, spiro[3.4]octane, spiro[3.5]nonane, spiro[4.4]nonane, spiro[4.5]decane, and spiro[5.5]undecane; and bridged bicycle rings such as bicyclo[2.1.1]hexane, bicyclo[2.2.1]heptane, and bicyclo[2.2.2]octane. A cycloalkyl group can be unsubstituted or optionally substituted. In a preferred embodiment of the present invention, cycloalkyl is cyclopropyl.

The term "heterocycle" or "heterocycloalkyl" means a 5- to 7-membered monocyclic, or 7- to 12-membered bicyclic, saturated heterocyclic ring which contains from 1 to 4 heteroatoms independently selected from nitrogen, oxygen and sulfur, and wherein the nitrogen and sulfur heteroatoms can be optionally oxidized, and the nitrogen heteroatom can be optionally quaternized. Heterocycles include heteroaryls as defined above. Representative heterocycles include oxiran, oxetan, tetrahydrofuran, tetrahydropyran, 1,4-dioxane, aziridine, azetidine, pyrrolidine, piperidine, piperazine, pyrrolidinone, hydantoine, valerolactam, thiirane, thietane, tetrahydrothiophene, tetrahydrothiopyra, morpholine, tetrahydropyridine, and tetrahydropyrimidine. Heterocycles include a bicyclic ring in which part of the heterocycle is fused to a benzene or cyclopenta-1,3-diene ring. The heterocycle can be attached via any heteroatom or carbon atom. In addition, heterocycles include fused bicyclic rings, spiro rings and bridged bicyclic rings in which one or more carbon atoms of the aforementioned polycyclic rings are replaced with nitrogen, oxygen or sulfur atoms. For example, when the heteroatom is nitrogen, these include, but are limited to, fused heterobicyclic rings such as octahydrocyclopenta[c]pyrrole, octahydropyrrolo[3,4-c]pyrrole, decahydroisoquinoline, and decahydro-2,6-naphthyridine; spiro rings such as 2-azaspiro[3.3]heptane, 2,6-diazaspiro[3.3]heptane, 2-azaspiro[3.4]octane, 2,6-diazaspiro[3.4]octane, 2-azaspiro[3.5]nonane, 2,7-diazaspiro[3.5]nonane, 2-azaspiro[4.4]nonane, 2,7-diazaspiro[4.4]nonane, 8-azaspiro[4.5]decane, 2,8-diazaspiro[4.5]decane, 3-azaspiro[5.5]undecane, and 3,9-diazaspiro[5.5]undecane; and bridged heterobicyclic rings such as 2-azabicyclo[2.1.1]hexane, 2-azabicyclo[2.2.1]heptane, 2,5-diazabicyclo[2.2.1]heptane, 2-azabicyclo[2.2.2]octane, and 2,5-diazabicyclo[2.2.2]octane. In a preferred embodiment of the present invention, heterocycle is any one selected from azetidine, diazetidine, piperidine, piperazine, azaspiro[3.5]nonane, and diazabicyclo[3.2.1]octane.

As used herein, the term "aryl" means a carbocyclic aromatic group containing from 5 to 10 ring atoms. Representative examples include, but are not limited to, phenyl, tolyl, xylyl, naphthyl, tetrahydronaphthyl, anthracenyl, fluorenyl, indenyl, and azulenyl. A carbocyclic aromatic group can be unsubstituted or optionally substituted.

As used herein, the term "heteroaryl" means an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are furan, 4H-pyran, pyrrole, imidazole, pyrazole, triazole, tetrazole, pyridine, pyrimidine, pyridazine, pyrazine, triazine, thiophene, oxazole, isoxazole, thiazole, isothiazole, oxadiazole, benzofuran, benzothiophene, quinoline, indole, benzoxazole, benzimidazole, benzothiazole, cinnoline, phthalazine, quinazoline, 1H-azepine, etc. In a preferred embodiment of the present invention, heteroaryl is thiazole, pyridine, or pyrazine.

In this specification, * or 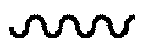 means connected to another moiety.

Preferably, in various aspects such as SHP2 degradation activity, (metabolism) stability, physicochemical properties, and the like, a preferred embodiment of the present invention provides a compound represented by the Chemical Formula 1 or a pharmaceutically acceptable salt thereof, wherein
R₁ₐ, R_{1b}, R_{1c}, and R_{1d} are each independently H or C₁₋₆alkyl (preferably methyl),
R₂ is H, C₁₋₆alkyl, or -NH₂,
R₃ is H, C₁₋₆alkyl, or C₁₋₆hydroxyalkyl,
R₄ is H, C₁₋₆alkyl, or halogen (preferably Cl),
R₅ is heteroaryl (preferably thiazole), wherein one or more hydrogens in the heteroaryl are optionally substituted with C₁₋₆alkyl (preferably methyl) or halogen,
R₆ is -NHC(O)R₇,
R₇ is C₁₋₆alkyl (preferably methyl) or C₃₋₁₀cycloalkyl (preferably cyclopropyl), wherein one or more hydrogens in the cycloalkyl are optionally substituted with C₁₋₆alkyl, halogen (preferably F), or -CN,
X is CH or N,
n₁ and n₂ are each independently 0 or 1,
m is 0 or 1,
L is the following Chemical Formula 2, 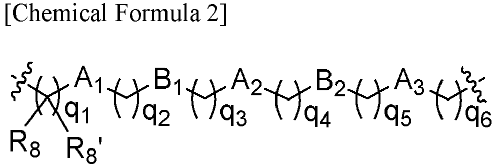 in the Chemical Formula 2,
   A₁, A₂, and A₃ are each independently direct bond, -O-, -N(R₉)- -C(O)-, -C(O)N(R₉)-, or -N(R₉)C(O)-,
   B₁ and B₂ are each independently direct bond, heterocycle (preferably azetidine, pyrrolidine, piperidine, piperazine, azaspiro[3.5]nonane, diazabicyclo[3.2.1]octane), or heteroaryl (preferably pyridine, pyrimidine, pyridazine, pyrazine), wherein one or more hydrogens in the heterocycle or heteroaryl are optionally substituted with C₁₋₆alkyl (preferably methyl), halogen, - OH, =O, or -CO₂R₉,
   R₈ and R₈' are each independently H or C₁₋₆alkyl, or R₈ and R₈' are linked to each other to form C₃₋₁₀cycloalkyl (preferably cyclopropyl),
   R₉ is H or C₁₋₆alkyl (preferably methyl),
   q₁, q₂, q₃, q₄, and q₅ are each independently an integer of from 0 to 10.

More preferably, in various aspects such as SHP2 degradation activity, (metabolism) stability, physicochemical properties, and the like, particularly in terms of SHP2 degradation activity, a more preferred embodiment of the present invention provides a compound represented by the Chemical Formula 1 or a pharmaceutically acceptable salt thereof, wherein
R₁ₐ, R_{1b}, R_{1c}, and R_{1d} are each independently H or C₁₋₆alkyl (preferably methyl),
R₂ is H or -NH₂,
R₃ is H,
R₄ is H or halogen (preferably Cl),
R₅ is thiazole, wherein one or more hydrogens in the thiazole are optionally substituted with C₁₋₆alkyl (preferably methyl),
R₆ is -NHC(O)R₇,
R₇ is C₁₋₆alkyl (preferably methyl) or cyclopropyl, wherein one or more hydrogens in the cyclopropyl are optionally substituted with halogen (preferably F) or -CN,
X is CH,
n₁ and n₂ are each independently 0 or 1,
m is 0 or 1,
L is the following Chemical Formula 2, 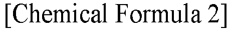 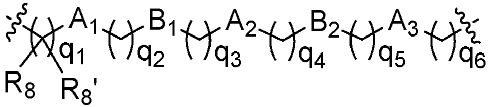 in the Chemical Formula 2,
   A₁, A₂, and A₃ are each independently direct bond, -O-, -N(R₉)- -C(O)-, -C(O)N(R₉)-, or -N(R₉)C(O)-,
   B₁ and B₂ are each independently direct bond, azetidine, pyrrolidine, piperidine, piperazine, azaspiro[3.5]nonane, diazabicyclo[3.2.1]octane, pyridine, pyrimidine, pyridazine, or pyrazine, wherein one or more hydrogens in the azetidine, pyrrolidine, piperidine, piperazine, azaspiro[3.5]nonane, diazabicyclo[3.2.1]octane, pyridine, pyrimidine, pyridazine, or pyrazine are optionally substituted with C₁₋₆alkyl (preferably methyl), -OH, =O, or -CO₂R₉,
   at least one of B₁ and B₂ is azetidine, pyrrolidine, piperidine, piperazine, azaspiro[3.5]nonane, diazabicyclo[3.2.1]octane, pyridine, pyrimidine, pyridazine, or pyrazine,
   R₈ and Rs' are each independently H or C₁₋₆alkyl, or R₈ and Rs' are linked to each other to form C₃₋₁₀cycloalkyl (preferably cyclopropyl),
   R₉ is H or C₁₋₆alkyl (preferably methyl),
   q₁, q₂, q₃, q₄, and q₅ are each independently an integer of from 0 to 10.

Preferably, in the SHP2 PROTAC compounds according to the present invention, the linker (L) is any one of the following linkers. When it has the following linker, SHP2 degradation activity is excellent, and it is more suitable for various purposes of the present invention.

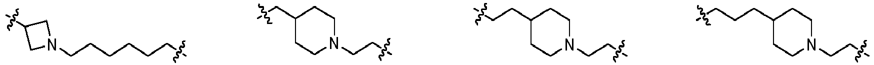

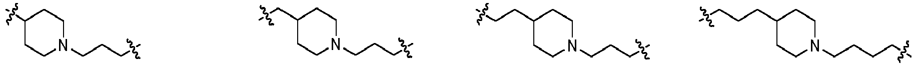

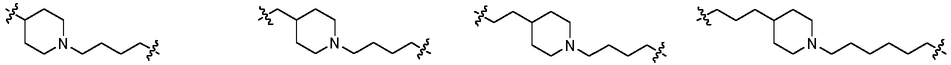

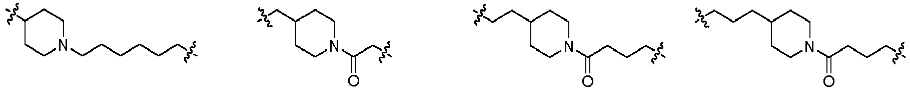

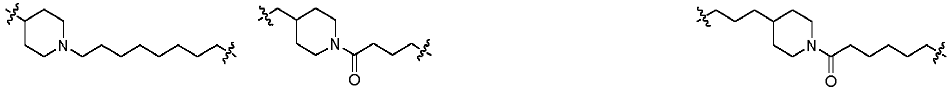

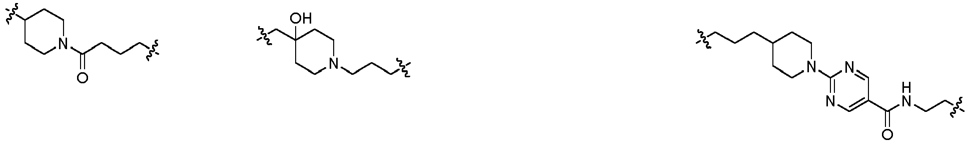

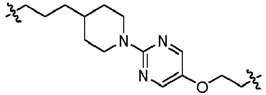

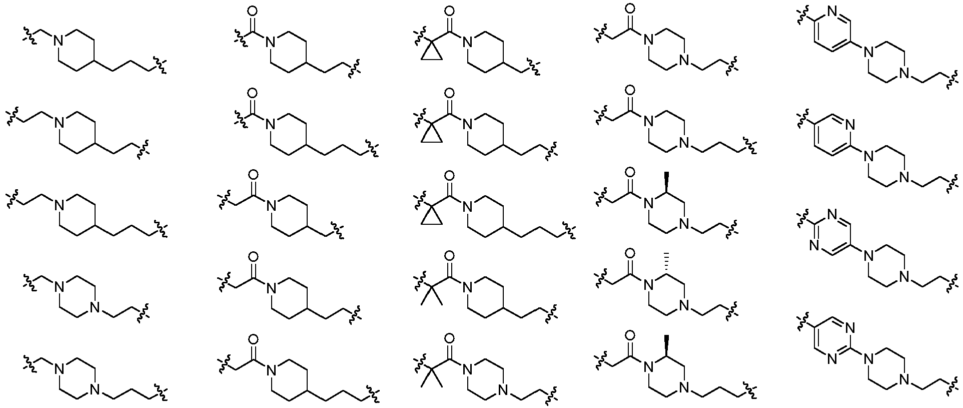

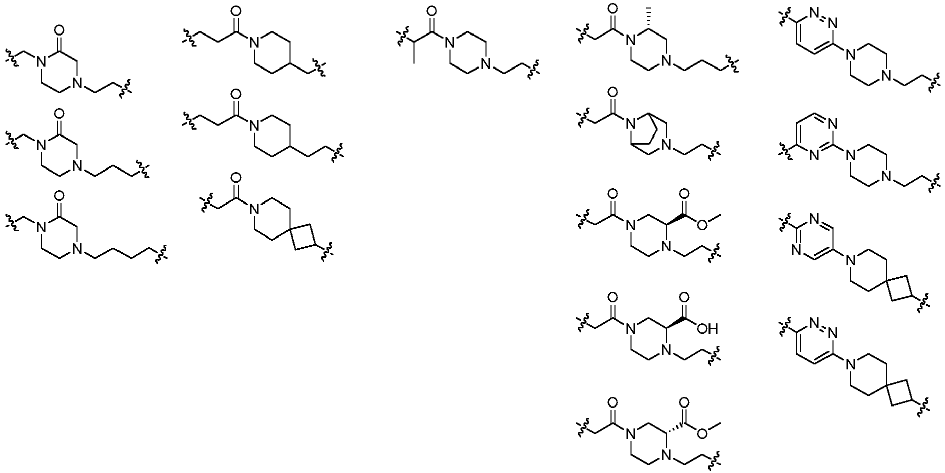

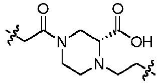

Non-limiting examples of the compound of Chemical Formula 1 according to the present disclosure are the compounds prepared in the Examples described below. Each example number corresponds to each compound number. For example, the number of the final compound prepared in Example 10 is Compound **10.**

Among the compounds, the compounds of Table 1 below were particularly preferable in terms of various aspects such as SHP2 degradation activity, cancer cell line cytotoxicity, (metabolic) stability, physicochemical properties, etc., in particular, in terms of SHP2 degrading activity.

**[Table 1]**

| Comp ound no. | IUPAC Name |
|---|---|
| 20 | ((2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-4-hydroxypiperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 29 | (2S,4R)-N-(2-((1-(3-((3-((5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 30 | (2S,4R)-N-(2-((1-(3-((3-((3-amino-5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 31 | (2S,4R)-N-(2-((1-(3-((3-((5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 32 | (2S,4R)-N-(2-((1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 33 | (2S,4R)-1-((S)-2-acetamido-3,3-dimethylbutanoyl)-N-(2-((1-(3-((3-((5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-4-hydroxypyrrolidine-2-carboxamide |
| 34 | (2S,4R)-N-(2-(2-(1-(3-((3-((5-(4-amino-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 35 | (2S,4R)-N-(2-(2-(1-(3-((3-((3-amino-5-(4-amino-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 36 | (2S,4R)-N-(2-(2-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 37 | (2S,4R)-N-(2-(2-(1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 38 | (2S,4R)-1-((S)-2-acetamido-3,3-dimethylbutanoyl)-N-(2-(2-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-4-hydroxypyrrolidine-2-carboxamide |
| 39 | (2S,4R)-N-(2-((1-(3-((3-((5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 40 | (2S,4R)-N-(2-(3-(1-(3-((3-((3-amino-5-(4-amino-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 41 | (2S,4R)-N-(2-(3-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 42 | (2S,4R)-N-(2-(3-(1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 43 | (2S,4R)-1-((S)-2-acetamido-3,3-dimethylbutanoyl)-N-(2-(3-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-4-hydroxypyrrolidine-2-carboxamide |
| 44 | (2S,4R)-N-(2-((1-(4-((3-((5-(4-(aminomethyl)-4-methylpiperidin-1-yl)pyrazin-2-yl)thio)-2-chlorophenyl)amino)-4-oxobutanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 45 | (2S,4R)-N-(2-(2-(1-(4-((3-((5-(4-(aminomethyl)-4-methylpiperidin-1-yl)pyrazin-2-yl)thio)-2-chlorophenyl)amino)-4-oxobutanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 50 | (2S,4R)-N-(2-((7-(3-((3-((5-(4-(aminomethyl)-4-methylpiperidin-1-yl)pyrazin-2-yl)thio)-2-chlorophenyl)amino)-3-oxopropanoyl)-7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 52 | (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 53 | (2S,4R)-N-(2-(2-(4-(3-((3-((5-(4-amino-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 54 | (2S,4R)-N-(2-(2-(4-(3-((3-((3-amino-5-(4-amino-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 55 | (2S,4R)-N-(2-(2-(4-(3-((3-((3-amino-5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 56 | (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(cyclopropanecarboxamido)-3,3-dimethylbutanoyl)-4-hydroxypyrrolidine-2-carboxamide |
| 57 | (2S,4R)-1-((S)-2-acetamido-3,3-dimethylbutanoyl)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-4-hydroxypyrrolidine-2-carboxamide |
| 58 | (2S,4R)-N-(2-(2-(4-(3-((3-((5-(4-(aminomethyl)-4-methylpiperidin-1-yl)pyrazin-2-yl)thio)-2-chlorophenyl)amino)-2-methyl-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 60 | (2S,4R)-N-(2-(3-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 61 | (2S,4R)-1-((S)-2-acetamido-3,3-dimethylbutanoyl)-N-(2-(3-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)propoxy)-4-(4-methylthiazol-5-yl)benzyl)-4-hydroxypyrrolidine-2-carboxamide |
| 62 | (2S,4R)-N-(2-(2-((R)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 63 | (2S,4R)-N-(2-(2-((S)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 64 | (2S,4R)-N-(2-(3-((S)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 66 | (S)-methyl 4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocydopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate |
| 67 | methyl (R)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocydopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate |
| 68 | (2S,4R)-N-(2-(2-(8-(3-((3-((5-(4-(aminomethyl)-4-methylpiperidin-1-yl)pyrazin-2-yl)thio)-2-chlorophenyl)amino)-3-oxopropanoyl)-3,8-diazabicyclo[3.2.1]octan-3-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 76 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 77 | (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 78 | (2S,4R)-N-(2-(4-(4-(2-((3-((5-(4-(aminomethyl)-4-methylpiperidin-1 -yl)pyrazin-2-yl)thio)-2-chlorophenyl)amino)-2-oxoethyl)-3-oxopiperazin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 79 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(cydopropanecarboxamido)-3,3-dimethylbutanoyl)-4-hydroxypyrrolidine-2-carboxamide |
| 80 | (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)propoxy)-4-(4-methylthiazol-5-yl)benzyl)-1-((S)-2-(cyclopropanecarboxamido)-3,3-dimethylbutanoyl)-4-hydroxypyrrolidine-2-carboxamide |
| 83 | N-(3-((5-(4-(aminomethyl)-4-methylpiperidin-1-yl)pyrazin-2-yl)thio)-2-chlorophenyl)-5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-2-carboxamide |
| 84 | N-(3-((5-(4-(aminomethyl)-4-methylpiperidin-1-yl)pyrazin-2-yl)thio)-2-chlorophenyl)-2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-5-carboxamide |
| 85 | N-(3-((5-(4-(aminomethyl)-4-methylpiperidin-1-yl)pyrazin-2-yl)thio)-2-chlorophenyl)-6-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyridazine-3-carboxamide |

As used herein, the term "pharmaceutically acceptable salt(s)" refers to a salt prepared from active compounds according to the present disclosure with relatively non-toxic acids or bases, depending on the particular substituents of those compounds. When the compounds have a relatively acidic group, base-added salts can be obtained by contacting the neutral compounds with a sufficient amount of the desired base and a pure or inert solvent. Suitable pharmaceutically acceptable base addition salts include, but are not limited to sodium, potassium, calcium, aluminum, organic amino, magnesium salts and the like. When the compounds have a relatively basic group, acid-added salts can be obtained by contacting the neutral compounds with a sufficient amount of the desired acid and pure or inert solvent. Suitable pharmaceutically acceptable acid addition salts include salts derived from non-toxic organic acids including, but are not limited to, acetic acid, propionic acid, isobutyl acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and the like, and non-toxic inorganic acids including, but are not limited to, hydrochloric acid, hydrobromic acid, nitric acid, carbonic acid, monohydrogencarbonic acid, phosphoric acid, monohydrogenphosphric acid, dihydrogenphosphoric acid, sulfuric acid, monohydrogensulfuric acid, hydrogen iodide, phosphorous acid and the like. Also it includes a salt of amino acid such as arginate or its analogues, and it also includes analogues of organic acid such as glucuronic or galacturonic acid. Some specific compounds of this disclosure have both basic and acidic functionality for the conversion of compounds with a basic or acidic portion (addition) salts. Other examples of salts are well known through literature known in the art to which the present invention pertains.

As used herein, the phrase "compound(s) of this/the invention" includes any compound(s) of Chemical Formula 1, as well as clathrates, hydrates, solvates, or polymorphs thereof. And, even if the term "compound(s) of the invention" does not mention its pharmaceutically acceptable sat, the term includes salts thereof. In one embodiment, the compounds of this disclosure include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of Chemical Formula 1 according to the present disclosure or salts thereof are tautomeric isomers and/or stereoisomers (e.g., geometrical isomers and conformational isomers), such isolated isomers and their mixtures also are included in the scope of this disclosure. If the compounds of the present disclosure or salts thereof have an asymmetric carbon in their structures, their active optical isomers and their racemic mixtures also are included in the scope of this disclosure.

As used herein, the term "polymorph" refers to solid crystalline forms of a compound of this disclosure or complex thereof. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means a compound or its salt according to this disclosure that further includes a stoichiometric or non-stoichiometric amount of a solvent bound by non-covalent intermolecular forces. Preferred solvents are volatile, non-toxic, and acceptable for administration to humans in trace amounts.

As used herein, the term "hydrate" means a compound or its salt according to this disclosure that further includes a stoichiometric or non-stoichiometric amount of water bound by non-covalent intermolecular forces.

As used herein, the term "clathrate" means a compound or its salt in the form of a crystal lattice that contains spaces (e.g., channels) that have a guest molecule (e.g., a solvent or water) trapped within.

As used herein, the term "purified" means that when isolated, the isolate is greater than 90% pure, in one embodiment greater than 95% pure, in another embodiment greater than 99% pure and in another embodiment greater than 99.9% pure.

### Medical uses and methods of treatment of the compounds according to the present invention

The present invention further provides methods for treating a disease or condition in a subject having or susceptible to having such a disease or condition, by administering to the subject a therapeutically-effective amount of one or more compounds as described above. In one embodiment, the treatment is preventative treatment. In another embodiment, the treatment is palliative treatment. In another embodiment, the treatment is restorative treatment.

### 1. Diseases or conditions

The compounds of the present invention for degrading SHP2 are useful for various therapeutic or prophylactic uses (e.g., cancer). These compounds can be used to degrade SHP2 to lower SHP2 activity, and can also be used to treat SHP2-related diseases or to prevent exacerbation of these diseases. Accordingly, the present invention provides a method for degrading SHP2 in a cell. In this method the cells are contacted with an effective amount of a compound of the invention. In one embodiment, the cell is present in a subject. The method of the present invention comprises administering to a subject in need of treatment or prevention a pharmaceutical composition comprising a therapeutically or prophylactically effective amount of a compound according to the present invention.

In one embodiment, the present invention provides a method of degrading SHP2 in a cell of a SHP2-associated disease. For example, the present invention can be used to degrade SHP2 in cells of a subject having a SHP2-related disease, which will be described later, and consequently lower SHP2 activity. In another embodiment of the present invention, the present invention can be used to degrade SHP2 in cells of cancer, in particular leukemia, lymphoma, lung cancer, head and neck cancer, esophageal cancer, stomach cancer, colorectal cancer, pancreatic cancer, liver cancer, breast cancer, ovarian cancer, cervical cancer, bladder cancer, melanoma, neuroblastoma, glioma, sarcoma and so on.

In another embodiment, the present invention provides a method of treating a SHP2-related disease, comprising administering to a subject a therapeutically effective amount of a compound of Chemical Formula 1 or a pharmaceutically acceptable salt thereof. Such a method comprises administering to a subject in need of treatment an amount of a compound of the invention sufficient to degrade SHP2, i.e., a therapeutically effective amount. In such a method, a compound of the present invention may be administered to the subject in the form of a pharmaceutical composition described herein.

In the present invention, the SHP2-related disease is cancer. For example, it may be any one of the cancers described in Table 2 of International Patent Application Publication No. WO 2021/236775, but is not limited thereto. The disclosures of Table 2 of WO 2021/236775 are all incorporated herein by reference. In another embodiment, the cancer is a solid tumor. In another embodiment, the cancer is a hematologic cancer. Exemplary hematologic cancers include, but are not limited to, the cancers listed in Table 3 of WO 2021/236775. The disclosures of Table 3 of WO 2021/236775 are all incorporated herein by reference. In another embodiment, the hematologic cancer is acute lymphocytic leukemia, chronic lymphocytic leukemia (including B-cell chronic lymphocytic leukemia), or acute myeloid leukemia. In another embodiment, the cancer is a leukemia, e.g., a leukemia selected from acute monocytic leukemia, acute myeloid leukemia, chronic myelogenous leukemia, chronic lymphocytic leukemia and mixed lineage leukemia (MLL). In another embodiment, the cancer is NUT-midline carcinoma. In another embodiment, the cancer is multiple myeloma. In another embodiment, the cancer is lung cancer, such as small cell lung cancer (SCLC). In another embodiment, the cancer is neuroblastoma. In another embodiment, the cancer is Burkitt's lymphoma. In another embodiment, the cancer is cervical cancer. In another embodiment, the cancer is head and neck cancer. In another embodiment, the cancer is esophageal cancer. In another embodiment, the cancer is stomach cancer. In another embodiment, the cancer is pancreatic cancer. In another embodiment, the cancer is liver cancer. In another embodiment, the cancer is melanoma. In another embodiment, the cancer is ovarian cancer. In another embodiment, the cancer is colorectal cancer. In another embodiment, the cancer is prostate cancer. In another embodiment, the cancer is breast cancer.

In another embodiment, the cancer according to the present invention is selected from the group consisting of acute monocytic leukemia, acute myeloid leukemia, chronic myelogenous leukemia, chronic lymphocytic leukemia, mixed lineage leukemia, lymphoma, NUT-midline carcinoma, multiple myeloma, small cell lung cancer, non-small cell lung cancer, neuroblastoma, Burkitt's lymphoma, cervical cancer, head and neck cancer, esophageal cancer, stomach cancer, pancreatic cancer, liver cancer, melanoma, ovarian cancer, colorectal cancer, prostate cancer, breast cancer, bladder cancer, glioma, sarcoma, esophageal squamous cell carcinoma and papillary thyroid carcinoma.

That is, the present invention provides a medical use of the compound of Chemical Formula 1 or a pharmaceutically acceptable salt thereof for treating or preventing the above diseases.

### 2. Subjects

Suitable subjects to be treated according to the present invention include mammalian subjects. Mammals according to the present disclosure include, but are not limited to, human, canine, feline, bovine, caprine, equine, ovine, porcine, rodents, lagomorphs, primates, and the like, and encompass mammals *in utero.*

In one embodiment, the suitable subject to be treated according to the present invention is human.

### 3. Administration and Dosing

The compounds of the present invention are generally administered in a therapeutically effective amount.

As used herein, "effective amount" refers to an amount of a compound of the invention sufficient to slow or minimize the progression of a SHP2-related disease or to provide a therapeutic benefit in the treatment or management of a SHP2-related disease. "Effective amount" also refers to an amount sufficient to inhibit or reduce SHP2 activity, either *in vitro* or *in vivo.*

The compounds of the present invention can be administered by any suitable route in the form of a pharmaceutical composition adapted to such a route, and in a dose effective for the treatment intended. An effective dosage is typically in the range of about 0.001 to about 100 mg per kg body weight per day, preferably about 0.01 to about 50 mg/kg/day, in single or divided doses. Depending on age, species and disease or condition being treated, dosage levels below the lower limit of this range may be suitable. In other cases, still larger doses may be used without harmful side effects. Larger doses may also be divided into several smaller doses, for administration throughout the day.

### Pharmaceutical compositions of the compounds of the present invention

In another embodiment, the present invention provides a pharmaceutical composition comprising the compound of Chemical Formula 1 or a pharmaceutically acceptable salt thereof, and a pharmaceutically acceptable carrier or excipient. In one embodiment of the present invention, the pharmaceutical composition is used for the treatment or prevention of SHP2-related diseases, preferably cancer, which is described above.

The term "pharmaceutically acceptable" means suitable for use as a pharmaceutical preparation, and generally considered safe for such use. The term also means that it has been officially approved by the governing body of a country for this use, or is listed in the Korean Pharmacopoeia or the United States Pharmacopoeia.

### Pharmaceutical compositions, dosage forms and administration routes

For the treatment of the diseases or conditions referred to above, the compounds described herein or pharmaceutically acceptable salts thereof can be administered as follows:

### Oral administration

The compounds of the present invention may be administered orally, including by swallowing, so that the compound enters the gastrointestinal tract, or absorbed into the blood stream directly from the mouth (e.g., buccal or sublingual administration).

Suitable compositions for oral administration include solid, liquid, gel or powder formulations, and have a dosage form such as tablet, lozenge, capsule, granule or powder.

Compositions for oral administration may optionally be enteric coated and may exhibit delayed or sustained release through the enteric coating. That is, the composition for oral administration according to the present invention may be a formulation having an immediate or modified release pattern.

Liquid formulations can include solutions, syrups and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

In a tablet dosage form the amount of drug, active ingredient, present may be from about 0.05% to about 95% by weight, more typically from about 2% to about 50% by weight of the dosage form. In addition, tablets may contain a disintegrant, comprising from about 0.5% to about 35% by weight, more typically from about 2% to about 25% of the dosage form. Examples of disintegrants include, but are not limited to, lactose, starch, sodium starch glycolate, crospovidone, croscarmellose sodium, maltodextrin, or mixtures thereof.

Suitable lubricants, for use in a tablet, may be present in amounts from about 0.1% to about 5% by weight, and include, but are not limited to, talc, silicon dioxide, stearic acid, calcium, zinc or magnesium stearate, sodium stearyl fumarate and the like.

Suitable binders, for use in a tablet, include, but are not limited to, gelatin, polyethylene glycol, sugars, gums, starch, polyvinyl pyrrolidone, hydroxypropyl cellulose, hydroxypropylmethyl cellulose and the like. Suitable diluents, for use in a tablet, include, but are not limited to, mannitol, xylitol, lactose, dextrose, sucrose, sorbitol, microcrystalline cellulose and starch.

Suitable solubilizers, for use in a tablet, may be present in amounts from about 0.1% to about 3% by weight, and include, but are not limited to, polysorbates, sodium lauryl sulfate, sodium dodecyl sulfate, propylene carbonate, diethyleneglycol monoethyl ether, dimethyl isosorbide, polyethylene glycol (natural or hydrogenated) castor oil, HCOR^{™} (Nikkol), oleyl ester, Gelucire^{™}, caprylic/caprylic acid mono/diglyceride, sorbitan fatty acid esters, and Solutol HS^{™}.

### Parenteral Administration

Compounds of the present disclosure may be administered directly into the blood stream, muscle, or internal organs. Suitable means for parenteral administration include intravenous, intramuscular, subcutaneous intraarterial, intraperitoneal, intrathecal, intracranial, and the like. Suitable devices for parenteral administration include injectors (including needle and needle-free injectors) and infusion methods.

Compositions for parenteral administration may be formulated as immediate or modified release, including delayed or sustained release.

Most parenteral formulations are liquid compositions, and the liquid composition is an aqueous solution containing the active ingredient according to the present invention, a salt, a buffering agent, an isotonic agent, and the like.

Parenteral formulations may also be prepared in a dehydrated form (e.g., by lyophilization) or as sterile non-aqueous solutions. These formulations can be used with a suitable vehicle, such as sterile water. Solubility-enhancing agents may also be used in preparation of parenteral solutions.

### Topical Administration

Compounds of the present invention may be administered topically to the skin or transdermally. Formulations for this topical administration can include lotions, solutions, creams, gels, hydrogels, ointments, foams, implants, patches and the like. Pharmaceutically acceptable carriers for topical administration formulations can include water, alcohol, mineral oil, glycerin, polyethylene glycol and the like. Topical administration can also be performed by electroporation, iontophoresis, phonophoresis and the like.

Compositions for topical administration may be formulated as immediate or modified release, including delayed or sustained release.

### [Advantageous Effects]

The present disclosure provides compounds capable of exhibiting various pharmacological activities due to very good SHP2 degradation activity, pharmaceutical compositions comprising the compound as an active ingredient, their medical uses (especially cancer or tumors), and methods of treatment or prevention comprising administering the compound to a subject in need of such treatment or prevention. The compound according to the present invention or a pharmaceutically acceptable salt thereof is excellent in various aspects such as SHP2 degradation activity, (metabolism) stability, physicochemical properties, etc., and in particular, SHP2 degradation activity is much superior to that of compounds having a similar structure.

### [Mode for Invention]

Hereinafter, the present invention is described in considerable detail with examples to help those skilled in the art understand the present invention. However, the following examples are offered by way of illustration and are not intended to limit the scope of the invention. It is apparent that various changes may be made without departing from the spirit and scope of the invention or sacrificing all of its material advantages.

### Preparation of compounds of the present invention

Hereinafter, the synthesis process of some compounds of the present invention will be described, and the other compounds not mentioned below can be prepared by substituting starting materials, intermediates and/or reactants in a similar manner.

### Intermediate 1. Tert-butyl (1-(5-((3-amino-2-chlorophenyl)thio)pyrazin-2-yl)-4-methylpiperidin-4-yl)carbamate

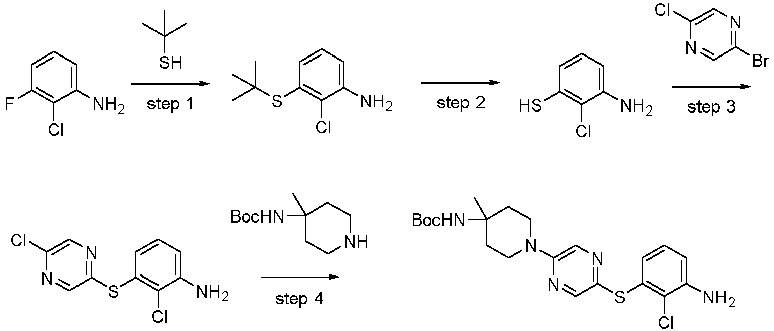

### Step 1: Synthesis of 3-(tert-butylthio)-2-chloroaniline

2-chloro-3-fluoroaniline (3 g, 20.60 mmol), 2-methylpropane-2-thiol (7 ml, 61.81 mmol), and cesium carbonate (13 g, 41.20 mmol) were suspended in DMF (40 ml) and then stirred at 120 °C for 16 hours. The reaction solution was diluted with EtOAc (400 ml) and washed with distilled water (150 ml x 3) and brine (150 ml x 3). The organic layer was dried over anhydrous sodium sulfate, filtered and concentrated under reduced pressure to obtain 4.44 g (quant.) of a brown oil.

### Step 2: Synthesis of 3-amino-2-chlorobenzenethiol

3-(tert-butylthio)-2-chloroaniline (4.44 g, 20.58 mmol) was suspended in conc. HCl (38 ml) and then stirred at 90 °C for 6 hours. After cooling the reaction solution, the resulting solid was filtered and dried to obtain 2.39 g (73%) of an ivory solid.

### Step 3: Synthesis of 2-chloro-3-((5-chloropyrazin-2-yl)thio)aniline

3-amino-2-chlorobenzenethiol (1.34 g, 8.39 mmol), 2-bromo-5-chloropyrazine (1.6 g, 8.39 mmol), Pd₂(dba)₃ (77 mg, 0.08 mmol), Xantphos (97 mg, 0.17 mmol), and DIPEA (2.9 mg, 16.79 mmol) were suspended in 1,4-dioxane (15 ml) and then then stirred at 95 °C for 1 hour. After filtration of the reaction solution, the filtrate was concentrated under reduced pressure to obtain a residue. The obtained residue was subjected to MPLC (3-15% EtOAc/Hexane) to give 1.24 g (54%) of an ivory solid.

### Step 4: Synthesis of tert-butyl (1-(5-((3-amino-2-chlorophenyl)thio)pyrazin-2-yl)-4-methylpiperidin-4-yl)carbamate

2-chloro-3-((5-chloropyrazin-2-yl)thio)aniline (120 mg, 0.44 mmol), tert-butyl (4-methylpiperidin-4-yl)carbamate (113 mg, 0.53 mmol), and DIPEA (0.35 ml, 1.99 mmol) were suspended in NMP (0.48 ml) and then then stirred at 130 °C for 16 hours. Distilled water (15 ml) was added to the reaction solution, followed by extraction with EtOAc (15 ml), and the organic layer was washed with brine (15 ml). Then, it was dried over anhydrous sodium sulfate, filtered, and concentrated under reduced pressure to obtain a residue. The obtained residue was subjected to MPLC (0-33% EtOAc/Hexane) to give 175 mg (88%) of an ivory solid.

### Intermediate 2. Tert-butyl (1-(6-amino-5-((3-amino-2-chlorophenyl)thio)pyrazin-2-yl)-4-methylpiperidin-4-yl)carbamate

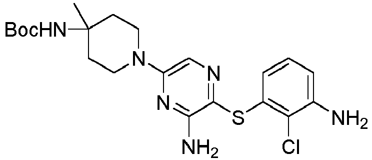

Intermediate 2 was synthesized in the same manner as in Intermediate 1, using 3-bromo-6-chloropyrazin-2-amine instead of 2-bromo-5-chloropyrazine.

### Intermediate 3. Tert-butyl ((1-(5-((3-amino-2-chlorophenyl)thio)pyrazin-2-yl)-4-methylpiperidin-4-yl)methyl)carbamate

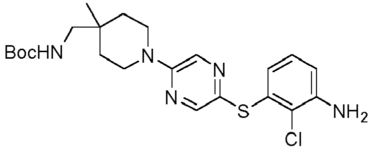

Intermediate 3 was synthesized in the same manner as in Intermediate 1, using tert-butyl ((4-methylpiperidin-4-yl)methyl)carbamate instead of tert-butyl (4-methylpiperidin-4-yl)carbamate.

### Intermediate 4. Tert-butyl ((1-(6-amino-5-((3-amino-2-chlorophenyl)thio)pyrazin-2-yl)-4-methylpiperidin-4-yl)methyl)carbamate

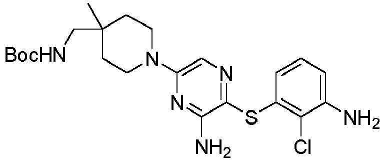

Intermediate 4 was synthesized in the same manner as in Intermediate 1, using 3-bromo-6-chloropyrazin-2-amine and tert-butyl ((4-methylpiperidin-4-yl)methyl)carbamate instead of 2-bromo-5-chloropyrazine and tert-butyl (4-methylpiperidin-4-yl)carbamate, respectively.

### Intermediate 5. Tert-butyl ((1-(5-((2-chloro-3-(methylamino)phenyl)thio)pyrazin-2-yl)-4-methylpiperidin-4-yl)methyl)carbamate

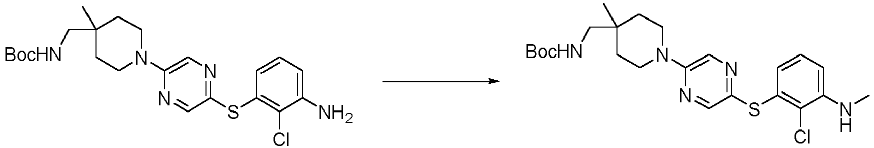

Tert-butyl ((1-(5-((3-amino-2-chlorophenyl)thio)pyrazin-2-yl)-4-methylpiperidin-4-yl)methyl)carbamate (Intermediate 3, 100 mg, 0.22 mmol) and 37% formaldehyde (87 µl, 1.08 mmol) were suspended in DCM (2 ml). Then, acetic acid (66 µl, 0.86 mmol), and sodium cyanoborohydride (27 mg, 0.43 mmol) were added and stirred at room temperature for 1 hour. A saturated aqueous solution of NaHCOs (10 ml) was added to the reaction solution, followed by extraction with DCM (10 ml x 2). The organic layer was dried over anhydrous sodium sulfate, filtered and concentrated under reduced pressure to obtain a residue. The obtained residue was subjected to MPLC (1-30% EtOAc/Hexane) to give 65.9 mg (64%) of an ivory solid.

### Intermediate 6. Tert-butyl (R)-((1-(5-((3-amino-2-chlorophenyl)thio)pyrazin-2-yl)pyrrolidin-3-yl)methyl)carbamate

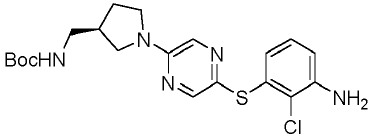

Intermediate 6 was synthesized in the same manner as in Intermediate 1, using tert-butyl (S)-(pyrrolidin-3-ylmethyl)carbamate instead of tert-butyl (4-methylpiperidin-4-yl)carbamate.

### Intermediate 7. (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide

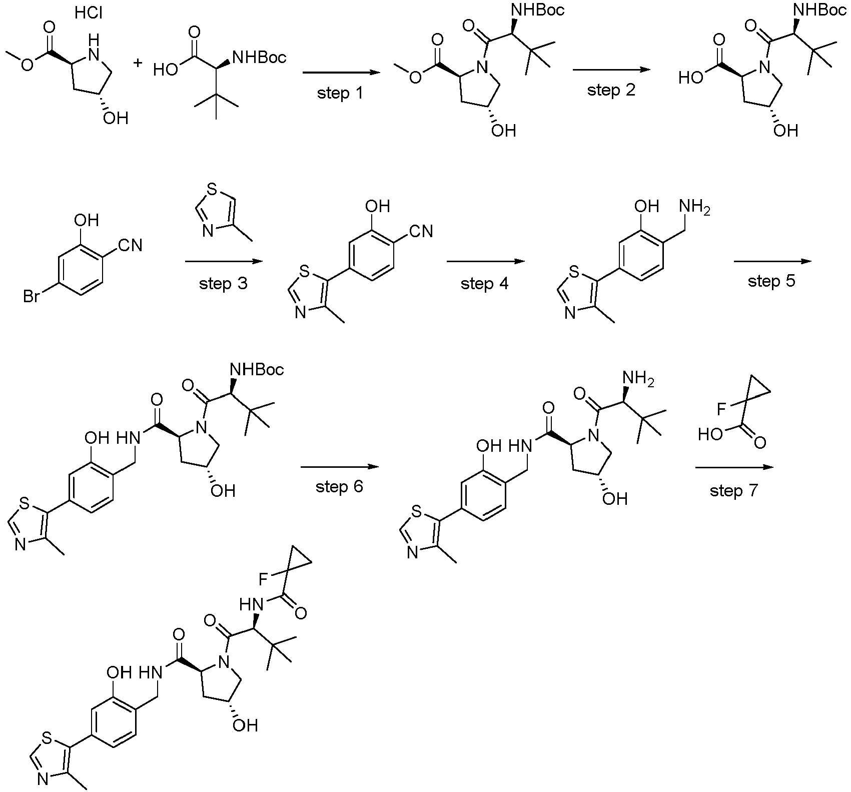

### Step 1: Synthesis of (2S,4R)-methyl 1-((S)-2-((tert-butoxycarbonyl)amino)-3,3-dimethylbutanoyl)-4-hydroxypyrrolidine-2-carboxylate

(2S,4R)-methyl 4-hydroxypyrrolidine-2-carboxylate hydrochloride (7.85 g 43.23 mmol), (S)-2-((tert-butoxycarbonyl)amino)-3,3-dimethylbutanoic acid (10 g, 43.23 mmol), EDCI (16.57 g, 86.46 mmol), HOBt (7 g, 86.46 mmol), and DIPEA (45 ml, 259.38 mmol) were suspended in DMF (216 ml) and then stirred at room temperature for 16 hours. The reaction solution was washed with a saturated aqueous solution of NH₄Cl (300 ml) and a saturated aqueous solution of NaHCOs (300 ml), and then extracted with EtOAc (150 ml x 2). The organic layer was washed with brine (150 ml x 2) and dried over anhydrous sodium sulfate. Then, the residue obtained by filtration and concentration under reduced pressure was subjected to MPLC (0-5% MeOH/DCM) to obtain 11 g (71%) of an ivory solid.

### Step 2: Synthesis of (2S,4R)-1-((S)-2-((tert-butoxycarbonyl)amino)-3,3-dimethylbutanoyl)-4-hydroxypyrrolidine-2-carboxylic acid

(2S,4R)-methyl 1-((S)-2-((tert-butoxycarbonyl)amino)-3,3-dimethylbutanoyl)-4-hydroxypyrrolidine-2-carboxylate (781 mg, 2.18 mmol) and lithium hydroxide (366 mg, 8.72 mmol) were suspended in water : THF = 1 : 1 (10 ml) and then stirred at room temperature for 3 hours. The reaction solution was adjusted to pH 1-2 using 1N HCl aqueous solution, and then extracted with EtOAc (15 ml x 2). Then, the organic layer was dried over anhydrous sodium sulfate, filtered, and concentrated under reduced pressure to obtain 680 mg (91%) of a white solid.

### Step 3: Synthesis of 2-hydroxy-4-(4-methylthiazol-5-yl)benzonitrile

4-bromo-2-hydroxybenzonitrile (2 g, 10.15 mmol) and 4-methylthiazole (1.83 ml, 20.20 mmol) were suspended in DMAC (40 ml), and potassium acetate (1.98 g, 20.20 mmol) and Pd(OAc)₂ (45 mg, 0.20 mmol) were added thereto and heated to reflux for 16 hours. Distilled water (100 ml) was added to the reaction solution, followed by extraction with EtOAc (50 ml x 2). The organic layer was washed with brine (50 ml x 2) and dried over anhydrous magnesium sulfate. After filtration and concentration under reduced pressure, the obtained residue was subjected to MPLC (0-50% EtOAc/Hexane) to obtain 980 mg (45%) of an ivory solid.

### Step 4: Synthesis of 2-(aminomethyl)-5-(4-methylthiazol-5-yl)phenol

2-hydroxy-4-(4-methylthiazol-5-yl)benzonitrile (864 mg, 3.99 mmol) was suspended in THF (22 ml). Then, LiAlH₄ (2.0 M in THF, 8 ml, 15.98 mmol) was slowly added at 0 °C and heated to reflux for 3 hours. After lowering the temperature of the reaction solution to 0 °C, distilled water (0.5 ml) was slowly added thereto, followed by neutralization with Rochelle solution (20 ml). Then, it was filtered through a celite filter. The filtrate was subjected to MPLC (DCM : MeOH : NH₄OH = 15 : 1 : 0.1) to give 416 mg (47%) of a yellow solid.

### Step 5: Synthesis of tert-butyl ((S)-1-((2S,4R)-4-hydroxy-2-((2-hydroxy-4-(4-methylthiazol-5-yl)benzyl)carbamoyl)pyrrolidin-1-yl)-3,3-dimethyl-1-oxobutan-2-yl)carbamate

2-(aminomethyl)-5-(4-methylthiazol-5-yl)phenol (416 mg 1.89 mmol), (2S,4R)-1-((S)-2-((tert-butoxycarbonyl)amino)-3,3-dimethylbutanoyl)-4-hydroxypyrrolidine-2-carboxylic acid (650 mg, 1.89 mmol), HATU (789 mg, 2.08 mmol), and DIPEA (0.98 ml, 5.66 mmol) were suspended in DMF (6 ml) and then stirred at room temperature for 16 hours. Distilled water (25 ml) was added to the reaction solution, followed by extraction with EtOAc (15 ml x 2). The organic layer was washed with brine (15 ml x 2) and dried over anhydrous sodium sulfate. After filtration and concentration under reduced pressure, the obtained residue was subjected to MPLC (0-20% MeOH/DCM) to obtain 898 mg (87%) of a pale yellow solid.

### Step 6: Synthesis of (2S,4R)-1-((S)-2-amino-3,3-dimethylbutanoyl)-4-hydroxy-N-(2-hydroxy-4-(4-methylthiazol-5-yl)benzyl)pyrrolidine-2-carboxamide

Tert-butyl ((S)-1-((2S,4R)-4-hydroxy-2-((2-hydroxy-4-(4-methylthiazol-5-yl)benzyl)carbamoyl)pyrrolidin-1-yl)-3,3-dimethyl-1-oxobutan-2-yl)carbamate (885 mg, 1.62 mmol) was suspended in DCM (30 ml). 4 M HCl in dioxane (8 ml) was added thereto, and then stirred at room temperature for 2 hours. The reaction solution was neutralized by adding a saturated aqueous solution of NaHCOs (20 ml), followed by extraction with DCM (10 ml x 2). The organic layer was washed with brine (10 ml x 2), dried over anhydrous magnesium sulfate, filtered, and concentrated under reduced pressure. The resulting residue was subjected to MPLC (amine-silica, 0-10% MeOH/DCM) to obtain 522 mg (72%) of a yellow solid.

### Step 7: Synthesis of (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide

(2S,4R)-1-((S)-2-amino-3,3-dimethylbutanoyl)-4-hydroxy-N-(2-hydroxy-4-(4-methylthiazol-5-yl)benzyl)pyrrolidine-2-carboxamide (399 mg 0.89 mmol), 1-fluorocyclopropane-1-carboxylic acid (92.6 mg, 0.89 mmol), HATU (381 mg, 0.89 mmol), and DIPEA (0.8 ml, 4.47 mmol) were suspended in DMF (4 ml) and then stirred at room temperature for 16 hours. Distilled water (10 ml) was added to the reaction solution, followed by extraction with EtOAc (11 ml x 2). The organic layer was washed with brine (10 ml x 2), dried over anhydrous sodium sulfate, filtered, and concentrated under reduced pressure to obtain a residue. The obtained residue was subjected to MPLC (0-10% MeOH/DCM) to give 377 mg (79%) of a white solid.

### Intermediate 8. (2S,4R)-1-((S)-2-(cyclopropanecarboxamido)-3,3-dimethylbutanoyl)-4-hydroxy-N-(2-hydroxy-4-(4-methylthiazol-5-yl)benzyl)pyrrolidine-2-carboxamide

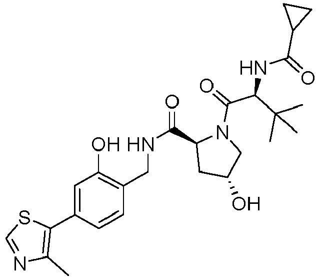

Intermediate 8 was synthesized in the same manner as in Intermediate 7, using cyclopropanecarboxylic acid instead of 1-fluorocyclopropane-1-carboxylic acid.

### Intermediate 9. (2S,4R)-1-((S)-2-acetamido-3,3-dimethylbutanoyl)-4-hydroxy-N-(2-hydroxy-4-(4-methylthiazol-5-yl)benzyl)pyrrolidine-2-carboxamide

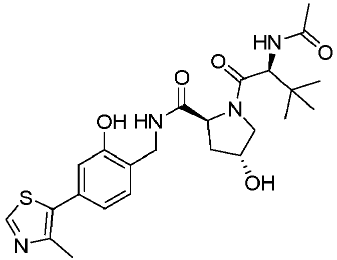

Intermediate 9 was synthesized in the same manner as in Intermediate 7, using acetic acid instead of 1-fluorocyclopropane-1-carboxylic acid.

### Intermediate 10. (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

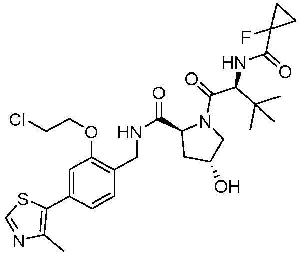

(2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 7, 350 mg, 0.657 mmol), and 1-bromo-2-chloroethane (0.2 ml, 2.628 mmol) were suspended in DMF (2 ml). Then, potassium carbonate (272 mg, 1.971 mmol), and potassium iodide (218 mg, 1.314 mmol) were added thereto and then stirred at 70 °C for 16 hours. Distilled water (20 ml) was added to the reaction solution, followed by extraction with EtOAc (20 ml x 2). The organic layer was washed with brine (20 ml x 2), dried over anhydrous magnesium sulfate, filtered and concentrated under reduced pressure to obtain a residue. The obtained residue was subjected to MPLC (0-5% MeOH/DCM) to give 234 mg (60%) of an ivory solid.

Intermediate 11-18 of Table 2 below were synthesized in the same manner as for the synthesis of Intermediate 10.

**[Table 2]**

| | |
|---|---|
| **Intermediate 11. (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide** | 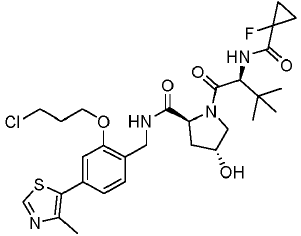 |
| **Intermediate 12. (2S,4R)-N-(2-(4-chlorobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide** | 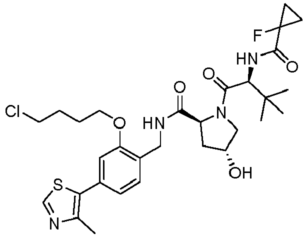 |
| **Intermediate 13. (2S,4R)-N-(2-((6-chlorohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide** | 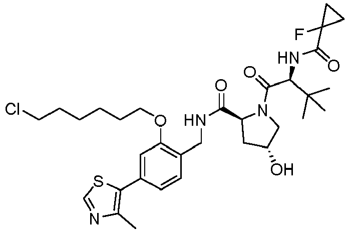 |
| **Intermediate 14. (2S,4R)-N-(2-((8-chlorooctyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide** | 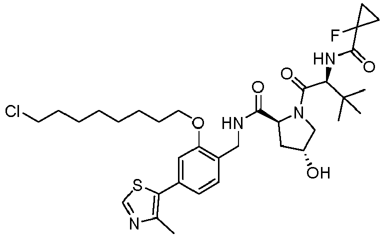 |
| **Intermediate 15. (2S,4R)-N-(2-(2-chloroethoxy)-4-(4-methylthiazol-5-yl)benzyl)-1-((S)-2-(cyclopropanecarboxamido)-3,3-dimethylbutanoyl)-4-hydroxypyrrolidine-2-carboxamide** | 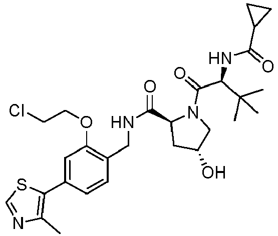 |
| **Intermediate 16. (2S,4R)-N-(2-(3-chloropropoxy)-4-(4-methylthiazol-5-yl)benzyl)-1-((S)-2-(cyclopropanecarboxamido)-3,3-dimethylbutanoyl)-4-hydroxypyrrolidine-2-carboxamide** | 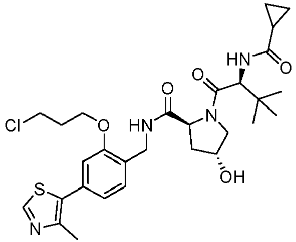 |
| **Intermediate 17. (2S,4R)-1-((S)-2-acetamido-3,3-dimethylbutanoyl)-N-(2-(2-chloroethoxy)-4-(4-methylthiazol-5-yl)benzyl)-4-hydroxypyrrolidine-2-carboxamide** | 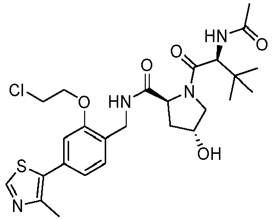 |
| **Intermediate 18. (2S,4R)-1-((S)-2-acetamido-3,3-dimethylbutanoyl)-N-(2-(3-chloropropoxy)-4-(4-methylthiazol-5-yl)benzyl)-4-hydroxypyrrolidine-2-carboxamide** | 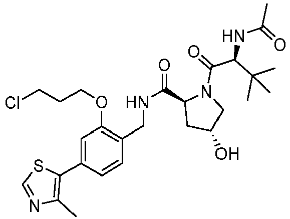 |

### Example 1. N-(3-((5-(4-(aminomethyl)-4-methylpiperidin-1-yl)pyrazin-2-yl)thio)-2-chlorophenyl)-1-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)piperidine-4-carboxamide

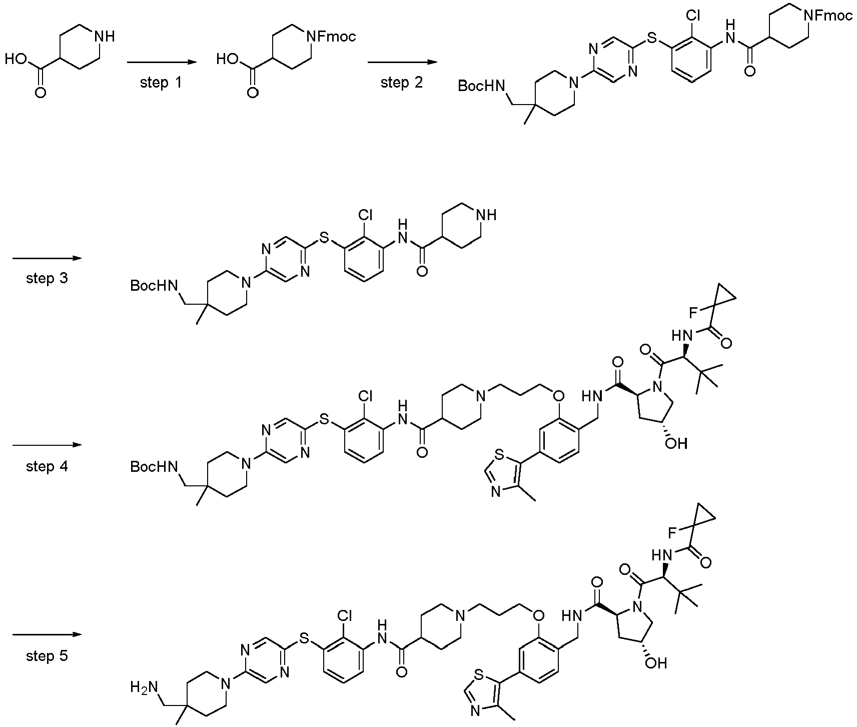

### Step 1: Synthesis of 1-(((9H-fluoren-9-yl)methoxy)carbonyl)piperidine-4-carboxylic acid

4-piperidinecarboxylic acid (1 g, 7.74 mmol), and Na₂CO₃ (2.5 g, 23.22 mmol) were suspended in water : 1,4-dioxane = 1 : 1 (64 ml) and then stirred at 0 °C for 10 minutes. After adding Fmoc-Cl (2 g, 7.74 mmol), the mixture was stirred at room temperature for 16 hours. The reaction solution was adjusted to pH = 6 using 1N HCl aqueous solution, and then extracted with EtOAc (30 ml x 2). The organic layer was dried over anhydrous sodium sulfate, filtered and concentrated under reduced pressure to obtain a residue. The obtained residue was subjected to MPLC (0-10% MeOH/DCM) to give 1.91 g (70%) of a white solid.

### Step 2: Synthesis of (9H-fluoren-9-yl)methyl 4-((3-((5-(4-(((tert-butoxycarbonyl)amino)methyl)-4-methylpiperidin-1-yl)pyrazin-2-yl)thio)-2-chlorophenyl)carbamoyl)piperidine-1-carboxylate

1-(((9H-fluoren-9-yl)methoxy)carbonyl)piperidine-4-carboxylic acid (758 mg, 2.16 mmol) was suspended in DCM (40 ml). Then, oxalyl chloride (0.24 ml, 2.80 mmol), and DMF (3 drops) were added thereto and then stirred at room temperature for 2.5 hours. The reaction solution was concentrated to obtain a yellow oil. After dissolving the obtained yellow oil in DCM (15 ml), tert-butyl ((1-(5-((3-amino-2-chlorophenyl)thio)pyrazin-2-yl)-4-methylpiperidin-4-yl)methyl)carbamate (Intermediate 3, 500 mg, 1.08 mmol), and DIPEA (0.75 ml, 4.31 mmol) were added and stirred at room temperature for 1 hour. Distilled water (20 ml) was added thereto, followed by extraction with DCM (15 ml x 2). The obtained organic layer was dried over anhydrous sodium sulfate, filtered and concentrated under reduced pressure to obtain a residue. The obtained residue was subjected to MPLC (0-5% MeOH/DCM) to give 908 mg (quant.) of an ivory solid.

### Step 3: Synthesis of tert-butyl ((1-(5-((2-chloro-3-(piperidine-4-carboxamido)phenyl)thio)pyrazin-2-yl)-4-methylpiperidin-4-yl)methyl)carbamate

(9H-fluoren-9-yl)methyl 4-((3-((5-(4-(((tert-butoxycarbonyl)amino)methyl)-4-methylpiperidin-1-yl)pyrazin-2-yl)thio)-2-chlorophenyl)carbamoyl)piperidine-1-carboxylate (908 mg, 1.14 mmol) was dissolved in DMF (7.2 ml). Then, piperidine (1.9 ml, 19.36 mmol) was slowly added and stirred at room temperature for 1 hour. Distilled water (20 ml) was added to the reaction solution, followed by extraction with EtOAc (20 ml x 2), and the organic layer was dried over anhydrous sodium sulfate. Then, it was filtered and concentrated under reduced pressure to obtain a residue. The obtained residue was subjected to MPLC (amine-silica, 0-100% EtOAc/Hexane, 0-5% MeOH/DCM) to obtain 462 mg (71%) of an ivory solid.

### Step 4: Synthesis of tert-butyl ((1-(5-((2-chloro-3-(1-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)piperidine-4-carboxamido)phenyl)thio)pyrazin-2-yl)-4-methylpiperidin-4-yl)methyl)carbamate

Tert-butyl ((1-(5-((2-chloro-3-(piperidine-4-carboxamido)phenyl)thio)pyrazin-2-yl)-4-methylpiperidin-4-yl)methyl)carbamate (16 mg, 0.03 mmol), and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11, 18.6 mg, 0.03 mmol) were suspended in DMF (0.04 ml). Then, potassium carbonate (12 mg, 0.08 mmol), and potassium iodide (9 mg, 0.06 mmol) were added and then stirred at 70 °C for 16 hours. Distilled water (1 ml) was added to the reaction solution, followed by extraction with EtOAc (2 ml x 2). The organic layer was washed with brine (1 ml x 2), dried over anhydrous magnesium sulfate, filtered and concentrated under reduced pressure to obtain a residue. The obtained residue was subjected to MPLC (0-10% MeOH/DCM) to give 20.8 mg (65%) of an ivory solid.

### Step 5: Synthesis of N-(3-((5-(4-(aminomethyl)-4-methylpiperidin-1-yl)pyrazin-2-yl)thio)-2-chlorophenyl)-1-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)piperidine-4-carboxamide

Tert-butyl ((1-(5-((2-chloro-3-(1-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)piperidine-4-carboxamido)phenyl)thio)pyrazin-2-yl)-4-methylpiperidin-4-yl)methyl)carbamate (20.8 mg, 0.02 mmol) was suspended in DCM (0.9 ml). Then, 4 M HCl in dioxane (0.04 ml) was added and then stirred at room temperature for 1 hour. The reaction solution was neutralized by adding a saturated aqueous solution of NaHCOs (10 ml), followed by extraction with DCM (5 ml x 2). The organic layer was washed with brine (5 ml x 2), dried over anhydrous magnesium sulfate, filtered, and concentrated under reduced pressure to obtain a residue. The obtained residue was subjected to MPLC (amine-silica, 0-5% MeOH/DCM) to obtain 15.4 mg (81%) of an ivory solid.

### Example 2. N-(3-((5-(4-(aminomethyl)-4-methylpiperidin-1-yl)pyrazin-2-yl)thio)-2-chlorophenyl)-1-(4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butyl)piperidine-4-carboxamide

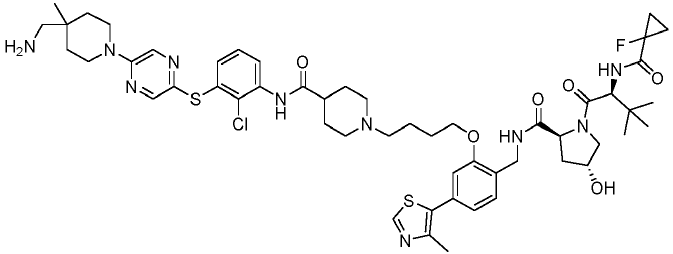

Example 2 was synthesized in the same way as in Example 1, using (2S,4R)-N-(2-(4-chlorobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 12) instead of (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11).

### Example 3. N-(3-((5-(4-(aminomethyl)-4-methylpiperidin-1-yl)pyrazin-2-yl)thio)-2-chlorophenyl)-1-(6-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)hexyl)piperidine-4-carboxamide

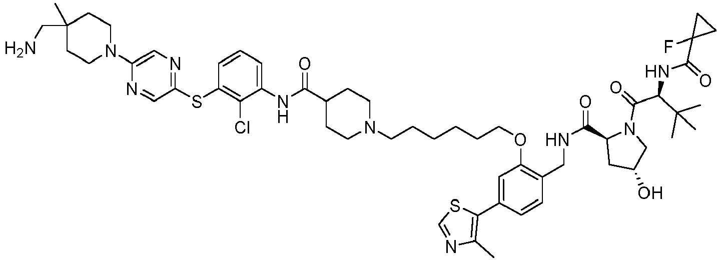

Example 3 was synthesized in the same way as in Example 1, using (2S,4R)-N-(2-((6-chlorohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 13) instead of (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11).

### Example 4. N-(3-((5-(4-(aminomethyl)-4-methylpiperidin-1-yl)pyrazin-2-yl)thio)-2-chlorophenyl)-1-(8-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)octyl)piperidine-4-carboxamide

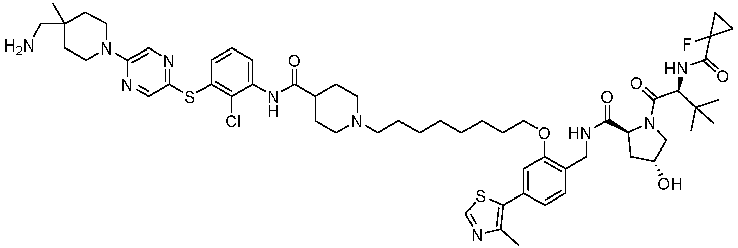

Example 4 was synthesized in the same way as in Example 1, using (2S,4R)-N-(2-((8-chlorooctyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 14) instead of (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11).

### Example 5. (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

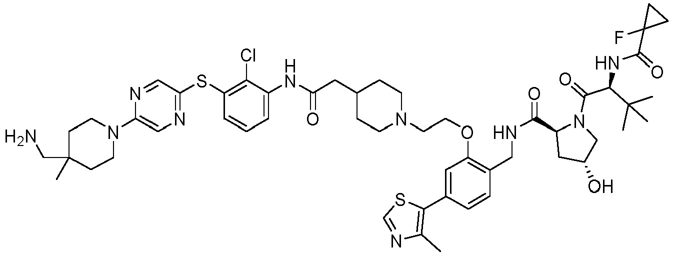

Example 5 was synthesized in the same way as in Example 1, using 2-(piperidin-4-yl)acetic acid and (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 6. (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

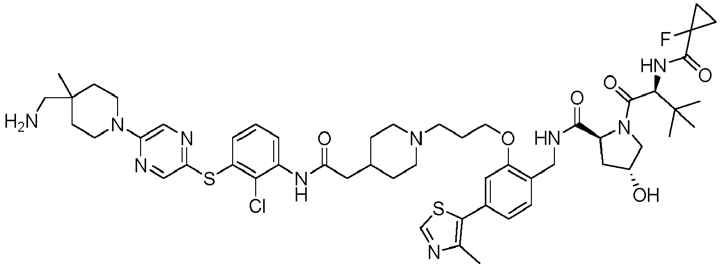

Example 6 was synthesized in the same way as in Example 1, using 2-(piperidin-4-yl)acetic acid instead of piperidine-4-carboxylic acid.

### Example 7. (2S,4R)-N-(2-(4-(4-(2-((3-((5-(4-(aminomethyl)-4-methylpiperidin-1-yl)pyrazin-2-yl)thio)-2-chlorophenyl)amino)-2-oxoethyl)piperidin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

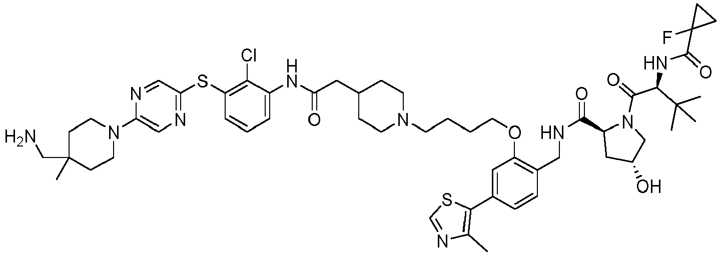

Example 7 was synthesized in the same way as in Example 1, using 2-(piperidin-4-yl)acetic acid and (2S,4R)-N-(2-(4-chlorobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 12) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 8. (2S,4R)-N-(2-(3-(4-(2-((3-((5-(4-amino-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

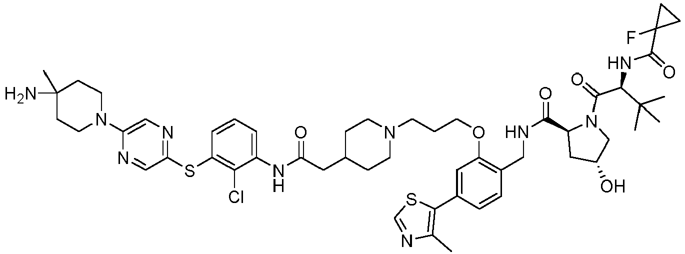

Example 8 was synthesized in the same way as in Example 1, using 2-(piperidin-4-yl)acetic acid and tert-butyl ((1-(5-((3-amino-2-chlorophenyl)thio)pyrazin-2-yl)-4-methylpiperidin-4-yl)methyl)carbamate (Intermediate 1) instead of piperidine-4-carboxylic acid and tert-butyl ((1-(5-((3-amino-2-chlorophenyl)thio)pyrazin-2-yl)-4-methylpiperidin-4-yl)methyl)carbamate (Intermediate 3), respectively.

### Example 9. (2S,4R)-N-(2-(3-(4-(2-((3-((3-amino-5-(4-amino-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

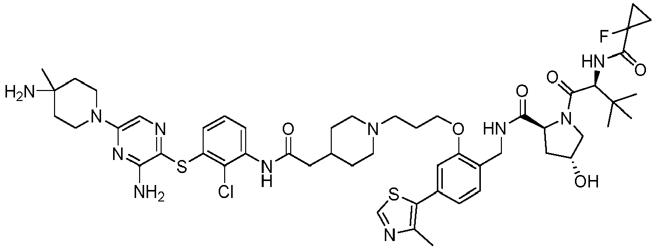

Example 9 was synthesized in the same way as in Example 1, using 2-(piperidin-4-yl)acetic acid and tert-butyl (1-(6-amino-5-((3-amino-2-chlorophenyl)thio)pyrazin-2-yl)-4-methylpiperidin-4-yl)carbamate (Intermediate 2) instead of piperidine-4-carboxylic acid and tert-butyl ((1-(5-((3-amino-2-chlorophenyl)thio)pyrazin-2-yl)-4-methylpiperidin-4-yl)methyl)carbamate (Intermediate 3), respectively.

### Example 10. (2S,4R)-N-(2-(3-(4-(2-((3-((3-amino-5-(4-(aminomethyl)-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

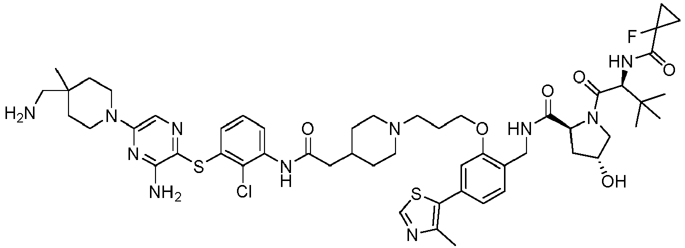

Example 10 was synthesized in the same way as in Example 1, using 2-(piperidin-4-yl)acetic acid and tert-butyl ((1-(6-amino-5-((3-amino-2-chlorophenyl)thio)pyrazin-2-yl)-4-methylpiperidin-4-yl)methyl)carbamate (Intermediate 4) instead of piperidine-4-carboxylic acid and tert-butyl ((1-(5-((3-amino-2-chlorophenyl)thio)pyrazin-2-yl)-4-methylpiperidin-4-yl)methyl)carbamate (Intermediate 3), respectively.

### Example 11. (2S,4R)-N-(2-(3-(4-(2-((3-((5-((R)-3-(aminomethyl)pyrrolidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

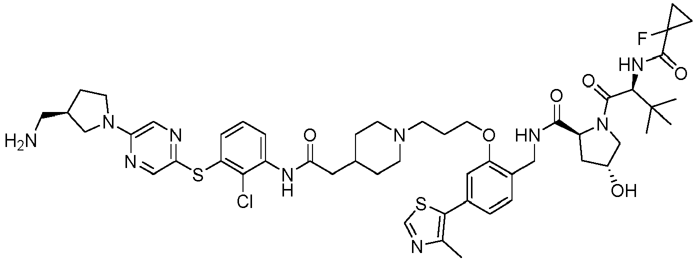

Example 11 was synthesized in the same way as in Example 1, using 2-(piperidin-4-yl)acetic acid and tert-butyl (R)-((1-(5-((3-amino-2-chlorophenyl)thio)pyrazin-2-yl)pyrrolidin-3-yl)methyl)carbamate (Intermediate 6) instead of piperidine-4-carboxylic acid and tert-butyl ((1-(5-((3-amino-2-chlorophenyl)thio)pyrazin-2-yl)-4-methylpiperidin-4-yl)methyl)carbamate (Intermediate 3), respectively.

### Example 12. (2S,4R)-1-((S)-2-acetamido-3,3-dimethylbutanoyl)-N-(2-(3-(4-(2-((3-((5-(4-(aminomethyl)-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-4-hydroxypyrrolidine-2-carboxamide

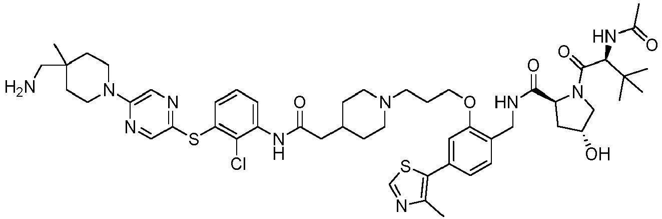

Example 12 was synthesized in the same way as in Example 1, using 2-(piperidin-4-yl)acetic acid and (2S,4R)-1-((S)-2-acetamido-3,3-dimethylbutanoyl)-N-(2-(3-chloropropoxy)-4-(4-methylthiazol-5-yl)benzyl)-4-hydroxypyrrolidine-2-carboxamide (Intermediate 18) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 13. (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

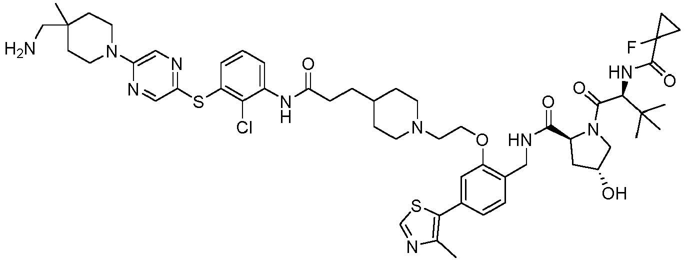

Example 13 was synthesized in the same way as in Example 1, using 3-(piperidin-4-yl)propanoic acid and (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 14. (2S,4R)-N-(2-(3-(4-(3-((3-((5-(4-(aminomethyl)-4-methylpiperidin-1-yl)pyrazin-2-yl)thio)-2-chlorophenyl)amino)-3-oxopropyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

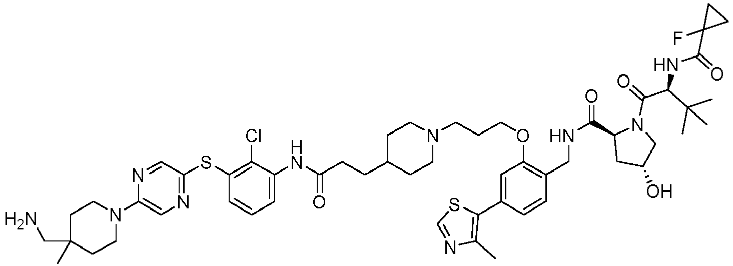

Example 14 was synthesized in the same way as in Example 1, using 3-(piperidin-4-yl)propanoic acid instead of piperidine-4-carboxylic acid.

### Example 15. (2S,4R)-N-(2-(4-(4-(3-((3-((5-(4-(aminomethyl)-4-methylpiperidin-1-yl)pyrazin-2-yl)thio)-2-chlorophenyl)amino)-3-oxopropyl)piperidin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

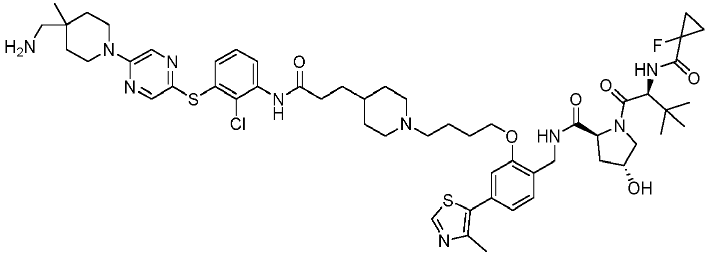

Example 15 was synthesized in the same way as in Example 1, using 3-(piperidin-4-yl)propanoic acid and (2S,4R)-N-(2-(4-chlorobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 12) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 16. (2S,4R)-N-(2-(2-(4-(4-((3-((5-(4-(aminomethyl)-4-methylpiperidin-1-yl)pyrazin-2-yl)thio)-2-chlorophenyl)amino)-4-oxobutyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

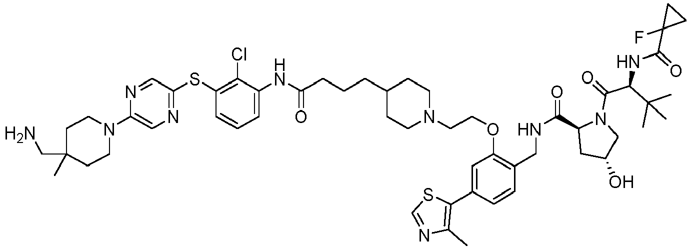

Example 16 was synthesized in the same way as in Example 1, using 4-(piperidin-4-yl)butanoic acid and (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 17. (2S,4R)-N-(2-(4-(4-(4-((3-((5-(4-(aminomethyl)-4-methylpiperidin-1-yl)pyrazin-2-yl)thio)-2-chlorophenyl)amino)-4-oxobutyl)piperidin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

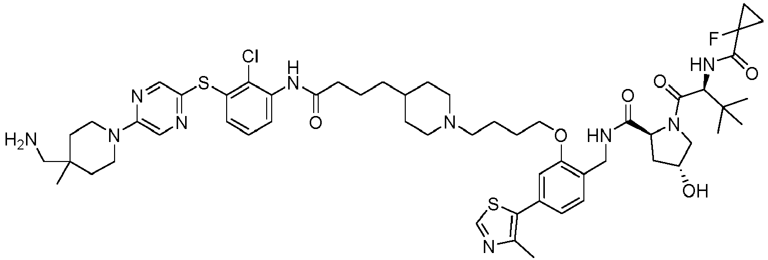

Example 17 was synthesized in the same way as in Example 1, using 4-(piperidin-4-yl)butanoic acid and (2S,4R)-N-(2-(4-chlorobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 12) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 18. (2S,4R)-N-(2-((6-(4-(4-((3-((5-(4-(aminomethyl)-4-methylpiperidin-1-yl)pyrazin-2-yl)thio)-2-chlorophenyl)amino)-4-oxobutyl)piperidin-1-yl)hexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

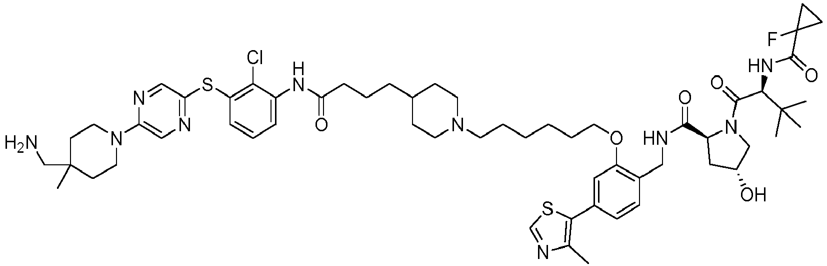

Example 18 was synthesized in the same way as in Example 1, using 4-(piperidin-4-yl)butanoic acid and (2S,4R)-N-(2-((6-chlorohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 13) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 19. (2S,4R)-N-(2-((6-(3-((3-((5-(4-(aminomethyl)-4-methylpiperidin-1-yl)pyrazin-2-yl)thio)-2-chlorophenyl)carbamoyl)azetidin-1-yl)hexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

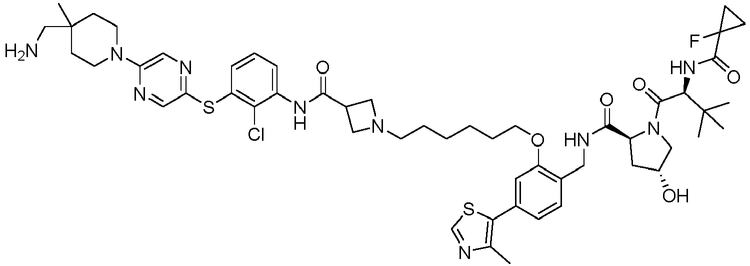

Example 19 was synthesized in the same way as in Example 1, using azetidine-3-carboxylic acid and (2S,4R)-N-(2-((6-chlorohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 13) instead of piperidine-4-carboxylic acid and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11), respectively.

### Example 20. ((2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-4-hydroxypiperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

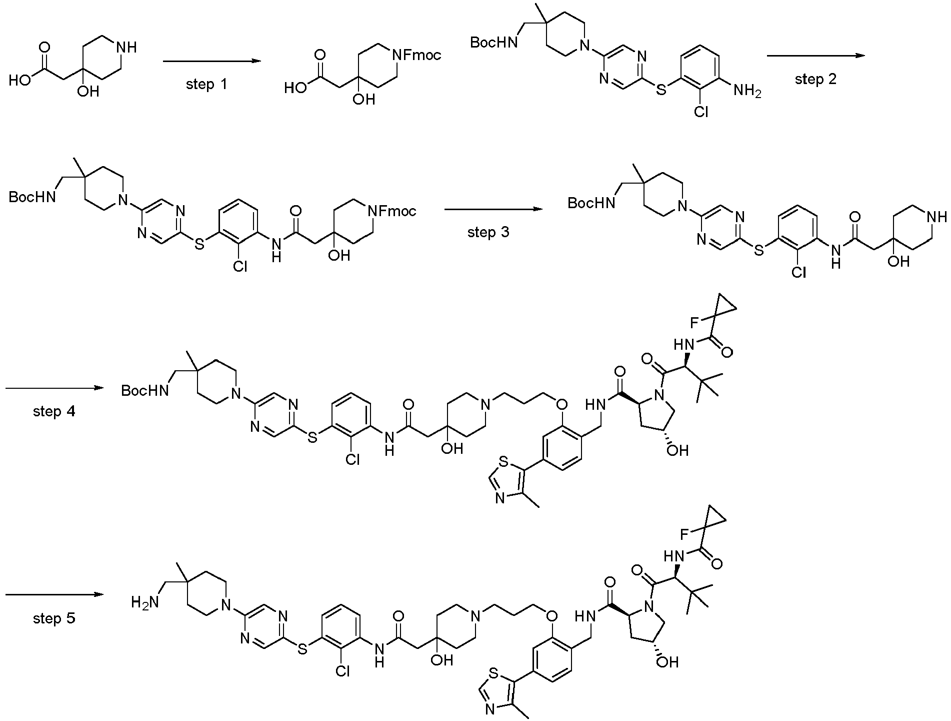

### Step 1: Synthesis of 2-(1-(((9H-fluoren-9-yl)methoxy)carbonyl)-4-hydroxypiperidin-4-yl)acetic acid

2-(4-hydroxypiperidin-4-yl)acetic acid (38 mg, 0.19 mmol), Na₂CO₃ (61 mg, 0.58 mmol) were suspended in water : 1,4-dioxane = 1 : 1 (1.6 ml) and then stirred at 0 °C for 10 minutes. After adding Fmoc-Cl (50 mg, 0.19 mmol), the mixture was stirred at room temperature for 16 hours. The reaction solution was adjusted to pH = 3 using 1N HCl aqueous solution, and then extracted with EtOAc (10 ml). The organic layer was dried over anhydrous sodium sulfate, filtered and concentrated under reduced pressure to obtain a residue. The obtained residue was subjected to MPLC (0-3% MeOH/DCM) to give 48.6 mg (66%) of a white solid.

### Step 2: Synthesis of (9H-fluoren-9-yl)methyl 4-(2-((3-((5-(4-(((tert-butoxycarbonyl)amino)methyl)-4-methylpiperidin-1-yl)pyrazin-2-yl)thio)-2-chlorophenyl)amino)-2-oxoethyl)-4-hydroxypiperidine-1-carboxylate

2-(1-(((9H-fluoren-9-yl)methoxy)carbonyl)-4-hydroxypiperidin-4-yl)acetic acid (25 mg, 0.06 mmol), tert-butyl ((1-(5-((3-amino-2-chlorophenyl)thio)pyrazin-2-yl)-4-methylpiperidin-4-yl)methyl)carbamate (Intermediate 3, 30 mg, 0.06 mmol), TCFH (108 mg, 0.38 mmol), and NMI (0.051 ml, 0.65 mmol) were suspended in ACN : THF = 1 : 1 (0.5 ml) and then stirred at room temperature for 5 hours. Distilled water (7 ml) was added to the reaction solution, followed by extraction with EtOAc (10 ml). The organic layer was washed with brine (7 ml), dried over anhydrous sodium sulfate, filtered, and concentrated under reduced pressure to obtain a residue. The obtained residue was subjected to MPLC (3-50% EtOAc/Hexane) to give 11.7 mg (23%) of an ivory solid.

### Step 3: Synthesis of tert-butyl ((1-(5-((2-chloro-3-(2-(4-hydroxypiperidin-4-yl)acetamido)phenyl)thio)pyrazin-2-yl)-4-methylpiperidin-4-yl)methyl)carbamate

(9H-fluoren-9-yl)methyl 4-(2-((3-((5-(4-(((tert-butoxycarbonyl)amino)methyl)-4-methylpiperidin-1-yl)pyrazin-2-yl)thio)-2-chlorophenyl)amino)-2-oxoethyl)-4-hydroxypiperidine-1-carboxylate (40 mg, 0.04 mmol) was dissolved in DMF (0.2 ml). Then, piperidine (0.01 ml, 0.97 mmol) was slowly added and stirred at room temperature for 2 hours. Distilled water (7 ml) was added to the reaction solution, followed by extraction with EtOAc (10 ml). The organic layer was washed with brine (7 ml), dried over anhydrous sodium sulfate, filtered, and concentrated under reduced pressure to obtain a residue. The obtained residue was subjected to MPLC (amine silica, 0-4% MeOH/DCM) to obtain 9.7 mg (37%) of an ivory solid.

### Step 4: Synthesis of tert-butyl ((1-(5-((2-chloro-3-(2-(1-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)-4-hydroxypiperidin-4-yl)acetamido)phenyl)thio)pyrazin-2-yl)-4-methylpiperidin-4-yl)methyl)carbamate

Tert-butyl ((1-(5-((2-chloro-3-(2-(4-hydroxypiperidin-4-yl)acetamido)phenyl)thio)pyrazin-2-yl)-4-methylpiperidin-4-yl)methyl)carbamate (9.7 mg, 0.02 mmol), and (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11, 12 mg, 0.02 mmol) were suspended in DMF (0.05 ml). Then, potassium carbonate (6.6 mg, 0.05 mmol) was added and then stirred at 70 °C for 16 hours. Distilled water (3 ml) was added to the reaction solution, followed by extraction with EtOAc (5 ml). The organic layer was washed with brine (3 ml), dried over anhydrous sodium sulfate, filtered, and concentrated under reduced pressure to obtain a residue. The obtained residue was subjected to MPLC (0-10% MeOH/DCM) to give 8.1 mg (43%) of an ivory solid.

### Step 5: Synthesis of ((2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-4-hydroxypiperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl ((1-(5-((2-chloro-3-(2-(1-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)-4-hydroxypiperidin-4-yl)acetamido)phenyl)thio)pyrazin-2-yl)-4-methylpiperidin-4-yl)methyl)carbamate (8.1 mg, 0.007 mmol) was suspended in DCM (0.7 ml). Then, 4 M HCl in dioxane (0.017 ml) was added and then stirred at room temperature for 1 hour. The reaction solution was neutralized by adding a saturated aqueous solution of NaHCOs (5 ml), followed by extraction with DCM (10 ml x 2). The organic layer was dried over anhydrous sodium sulfate, filtered and concentrated under reduced pressure to obtain a residue. The obtained residue was subjected to MPLC (amine-silica, 0-3% MeOH/DCM) to obtain 5.8 mg (78%) of an ivory solid.

### Example 21. N-(3-((5-(4-(aminomethyl)-4-methylpiperidin-1-yl)pyrazin-2-yl)thio)-2-chlorophenyl)-1-(4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoyl)piperidine-4-carboxamide

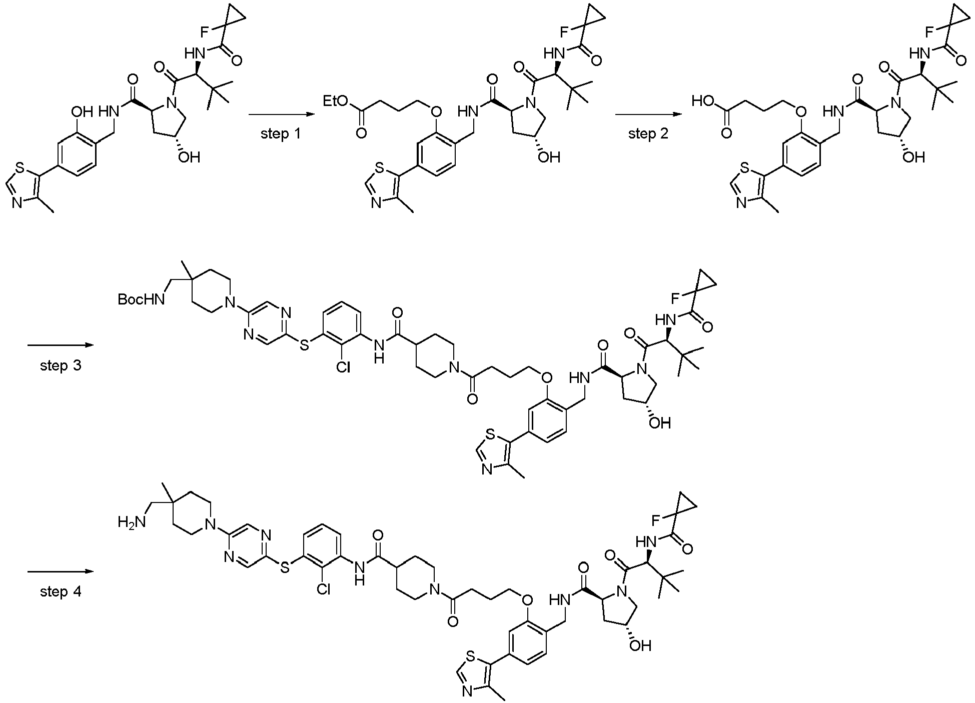

### Step 1: Synthesis of ethyl 4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoate

(2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 7, 97 mg, 0.18 mmol), and ethyl 4-bromobutyrate (0.05 ml, 0.36 mmol) were suspended in DMF (0.3 ml). Then, potassium carbonate (75 mg, 0.55 mmol) was added and then stirred at 70 °C for 16 hours. Distilled water (10 ml) was added to the reaction solution, followed by extraction with EtOAc (10 ml x 2). The organic layer was washed with brine (10 ml x 2), dried over anhydrous magnesium sulfate, filtered, and concentrated under reduced pressure to obtain a residue. The obtained residue was subjected to MPLC (0-10% MeOH/DCM) to give 104 mg (88%) of an ivory solid.

### Step 2: Synthesis of 4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoic acid

Ethyl 4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoate (102 mg, 0.16 mmol), and lithium hydroxide (16 mg, 0.37 mmol) were suspended in water : THF = 2 : 1 (1.8 ml) and then stirred at room temperature for 4 hours. The reaction solution was adjusted to pH = 2 using 1N HCl aqueous solution, and then extracted with EtOAc (10 ml x 2). The organic layer was dried over anhydrous sodium sulfate, filtered, and concentrated under reduced pressure to obtain 60 mg (61%) of an ivory solid.

### Step 3: Synthesis of tert-butyl ((1-(5-((2-chloro-3-(1-(4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoyl)piperidine-4-carboxamido)phenyl)thio)pyrazin-2-yl)-4-methylpiperidin-4-yl)methyl)carbamate

4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoic acid (19 mg 0.03 mmol), tert-butyl ((1-(5-((2-chloro-3-(piperidine-4-carboxamido)phenyl)thio)pyrazin-2-yl)-4-methylpiperidin-4-yl)methyl)carbamate (18 mg, 0.03 mmol), EDCI (7.2 mg, 0.04 mmol), HOBt (5 mg, 0.04 mmol), and DIPEA (0.02 mL, 0.13 mmol) were suspended in DMF (0.5 ml) and then stirred at room temperature for 16 hours. Distilled water (2 ml) was added to the reaction solution, followed by extraction with EtOAc (2 ml x 2). The organic layer was washed with brine (2 ml x 2), the organic layer was dried over anhydrous sodium sulfate, filtered, and concentrated under reduced pressure to obtain a residue. The obtained residue was subjected to MPLC (10% MeOH/DCM) to give 12.2 mg (33%) of an ivory solid.

### Step 4: Synthesis of N-(3-((5-(4-(aminomethyl)-4-methylpiperidin-1-yl)pyrazin-2-yl)thio)-2-chlorophenyl)-1-(4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoyl)piperidine-4-carboxamide

Tert-butyl ((1-(5-((2-chloro-3-(1-(4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoyl)piperidine-4-carboxamido)phenyl)thio)pyrazin-2-yl)-4-methylpiperidin-4-yl)methyl)carbamate (12.2 mg, 0.01 mmol) was suspended in DCM (0.5 ml). Then, 4 M HCl in dioxane (0.03 ml) was added and then stirred at room temperature for 1 hour. A saturated aqueous solution of NaHCOs (2 ml) was added to the reaction solution, followed by extraction with 10% MeOH/DCM (5 ml x 2). The organic layer was washed with brine (5 ml x 2), dried over anhydrous magnesium sulfate, filtered, and concentrated under reduced pressure to obtain a residue. The residue was subjected to MPLC (amine-silica, 0-5% MeOH/DCM) to give 7.8 mg (70%) of an ivory solid.

### Example 22. (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)piperidin-1-yl)-2-oxoethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

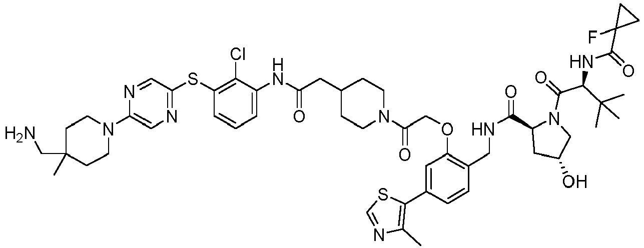

Example 22 was synthesized in the same way as in Example 21, using ethyl 2-bromoacetate and tert-butyl ((1-(5-((2-chloro-3-(2-(piperidin-4-yl)acetamido)phenyl)thio)pyrazin-2-yl)-4-methylpiperidin-4-yl)methyl)carbamate instead of ethyl 4-bromobutyrate and tert-butyl ((1-(5-((2-chloro-3-(piperidine-4-carboxamido)phenyl)thio)pyrazin-2-yl)-4-methylpiperidin-4-yl)methyl)carbamate, respectively.

### Example 23. (2S,4R)-N-(2-(4-(4-(2-((3-((5-(4-(aminomethyl)-4-methylpiperidin-1-yl)pyrazin-2-yl)thio)-2-chlorophenyl)amino)-2-oxoethyl)piperidin-1-yl)-4-oxobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

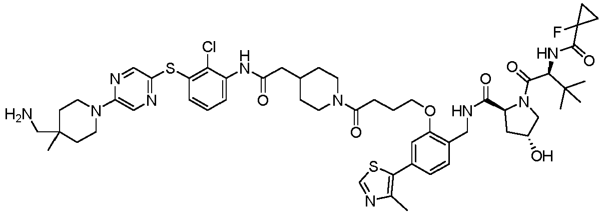

Example 23 was synthesized in the same way as in Example 21, using tert-butyl ((1-(5-((2-chloro-3-(2-(piperidin-4-yl)acetamido)phenyl)thio)pyrazin-2-yl)-4-methylpiperidin-4-yl)methyl)carbamate instead of tert-butyl ((1-(5-((2-chloro-3-(piperidine-4-carboxamido)phenyl)thio)pyrazin-2-yl)-4-methylpiperidin-4-yl)methyl)carbamate.

### Example 24. (2S,4R)-N-(2-(4-(4-(3-((3-((5-(4-(aminomethyl)-4-methylpiperidin-1-yl)pyrazin-2-yl)thio)-2-chlorophenyl)amino)-3-oxopropyl)piperidin-1-yl)-4-oxobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

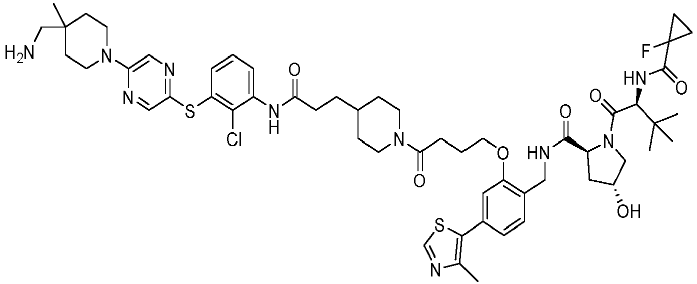

Example 24 was synthesized in the same way as in Example 21, using tert-butyl ((1-(5-((2-chloro-3-(3-(piperidin-4-yl)propanamido)phenyl)thio)pyrazin-2-yl)-4-methylpiperidin-4-yl)methyl)carbamate instead of tert-butyl ((1-(5-((2-chloro-3-(piperidine-4-carboxamido)phenyl)thio)pyrazin-2-yl)-4-methylpiperidin-4-yl)methyl)carbamate.

### Example 25. (2S,4R)-N-(2-(4-(4-(4-((3-((5-(4-(aminomethyl)-4-methylpiperidin-1-yl)pyrazin-2-yl)thio)-2-chlorophenyl)amino)-4-oxobutyl)piperidin-1-yl)-4-oxobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

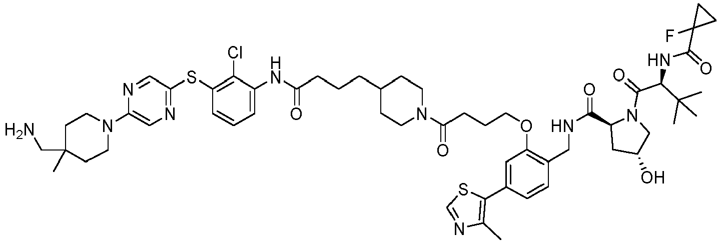

Example 25 was synthesized in the same way as in Example 21, using tert-butyl ((1-(5-((2-chloro-3-(4-(piperidin-4-yl)butanamido)phenyl)thio)pyrazin-2-yl)-4-methylpiperidin-4-yl)mthyl)carbamate instead of tert-butyl ((1-(5-((2-chloro-3-(piperidine-4-carboxamido)phenyl)thio)pyrazin-2-yl)-4-methylpiperidin-4-yl)methyl)carbamate.

### Example 26. (2S,4R)-N-(2-((6-(4-(4-((3-((5-(4-(aminomethyl)-4-methylpiperidin-1-yl)pyrazin-2-yl)thio)-2-chlorophenyl)amino)-4-oxobutyl)piperidin-1-yl)-6-oxohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

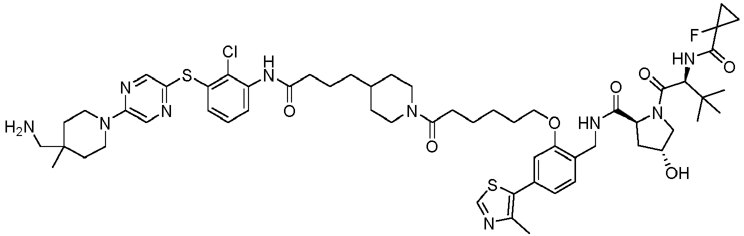

Example 26 was synthesized in the same way as in Example 21, using methyl 6-bromohexanoate and tert-butyl ((1-(5-((2-chloro-3-(4-(piperidin-4-yl)butanamido)phenyl)thio)pyrazin-2-yl)-4-methylpiperidin-4-yl)methyl)carbamate instead of ethyl 4-bromobutyrate and tert-butyl ((1-(5-((2-chloro-3-(piperidine-4-carboxamido)phenyl)thio)pyrazin-2-yl)-4-methylpiperidin-4-yl)methyl)carbamate, respectively.

### Example 27. (2S,4R)-N-(2-(2-(1-(2-((3-((5-(4-(aminomethyl)-4-methylpiperidin-1-yl)pyrazin-2-yl)thio)-2-chlorophenyl)amino)-2-oxoacetyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

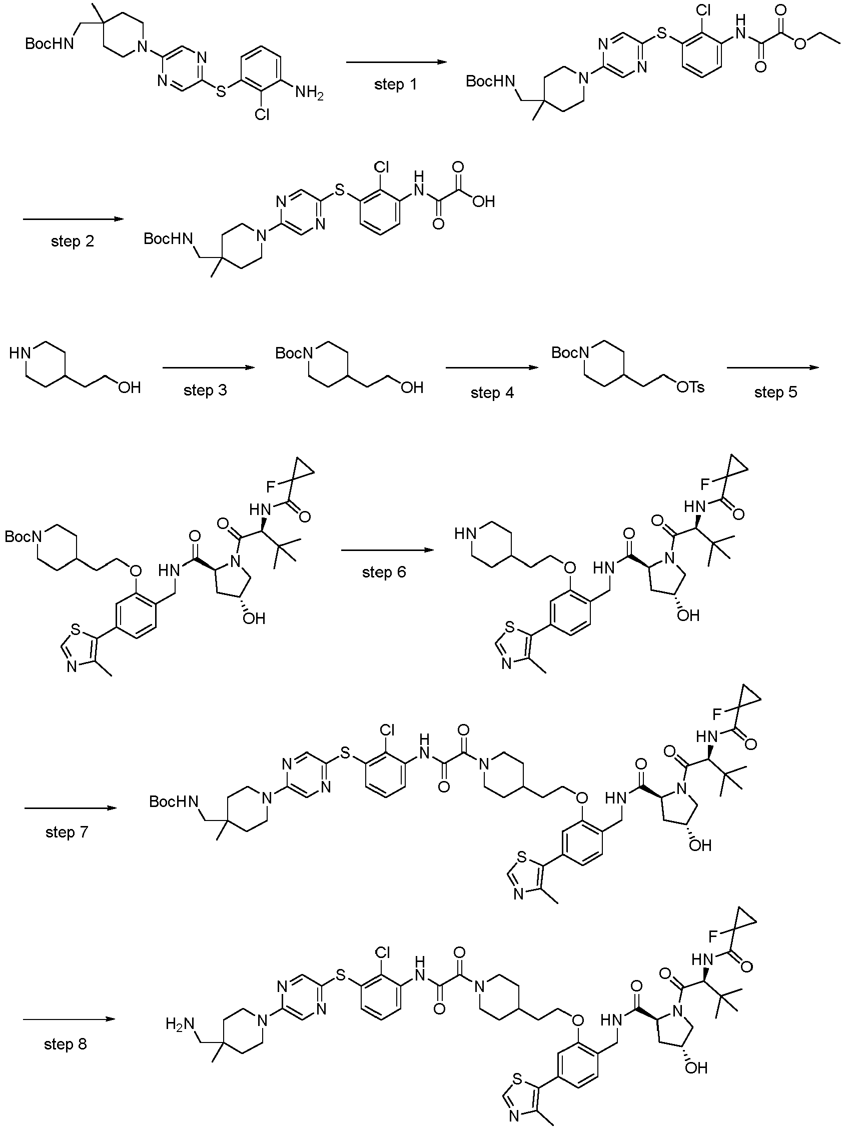

### Step 1: Synthesis of ethyl 2-((3-((5-(4-(((tert-butoxycarbonyl)amino)methyl)-4-methylpiperidin-1-yl)pyrazin-2-yl)thio)-2-chlorophenyl)amino)-2-oxoacetate

Tert-butyl ((1-(5-((3-amino-2-chlorophenyl)thio)pyrazin-2-yl)-4-methylpiperidin-4-yl)methyl)carbamate (Intermediate 3, 100 mg, 0.22 mmol) was suspended in DCM (2 ml). Then, ethyl chloroglyoxylate (27.9 mg, 0.20 mmol) dissolved in DCM (1 ml) was slowly added at 0 °C and stirred for 30 minutes. A saturated aqueous solution of NaHCOs (2 ml) was added to the reaction solution, followed by extraction with 10% MeOH/DCM (20 ml x 2). The organic layer was washed with brine (5 ml x 2), dried over anhydrous magnesium sulfate, filtered, and concentrated under reduced pressure to obtain a residue. The residue was subjected to MPLC (amine-silica, 30-40% EtOAc/Hexane) to give 116 mg (95%) of an ivory solid.

### Step 2: Synthesis of 2-((3-((5-(4-(((tert-butoxycarbonyl)amino)methyl)-4-methylpiperidin-1-yl)pyrazin-2-yl)thio)-2-chlorophenyl)amino)-2-oxoacetic acid

Ethyl 2-((3-((5-(4-(((tert-butoxycarbonyl)amino)methyl)-4-methylpiperidin-1-yl)pyrazin-2-yl)thio)-2-chlorophenyl)amino)-2-oxoacetate (116 mg, 0.21 mmol), lithium hydroxide (17 mg, 0.41 mmol) were suspended in THF : MeOH : H₂O = 3 : 2 : 1 (0.74 ml) and then stirred at room temperature for 2 hours. The reaction solution was adjusted to pH = 3 using 1N HCl aqueous solution, and then extracted with EtOAc (10 ml x 2). The organic layer was dried over anhydrous sodium sulfate, filtered, and concentrated under reduced pressure to obtain 100 mg (91%) of an ivory solid.

### Step 3: Synthesis of tert-butyl 4-(2-hydroxyethyl)piperidine-1-carboxylate

2-(piperidin-4-yl)ethan-1-ol (1 g, 7.74 mmol), and triethylamine (3.2 ml, 23.22 mmol) were suspended in chloroform (16 ml). Then, di-tert-butyl dicarbonate (1.9 ml, 8.05 mmol) was slowly added at 0 °C and stirred for 1 hour. A saturated NH₄Cl aqueous solution (45 ml) was added to the reaction solution, followed by extraction with DCM (20 ml x 2). The organic layer was washed with brine (20 ml x 2), dried over anhydrous magnesium sulfate, filtered, and concentrated under reduced pressure to obtain 2.12 g (quant.) of a colorless oil.

### Step 4: Synthesis of tert-butyl 4-(2-(tosyloxy)ethyl)piperidine-1-carboxylate

Tert-butyl 4-(2-hydroxyethyl)piperidine-1-carboxylate (2.1 g, 9.11 mmol), and triethylamine (1.4 ml, 10.02 mmol) were suspended in DCM (13 ml). Then, 4-toluenesulfonyl chloride (1.74 g, 9.11 mmol) was added and then stirred at room temperature for 16 hours. Distilled water (10 ml) was added to the reaction solution, followed by extraction with DCM (10 ml x 2). The organic layer was washed with brine (10 ml x 2), dried over anhydrous magnesium sulfate, filtered, and concentrated under reduced pressure to obtain a residue. The residue was subjected to MPLC (0-50% EtOAc/Hexane) to give 2.41 g (69%) of a colorless oil.

### Step 5: Synthesis of tert-butyl 4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-1-carboxylate

(2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 7, 261 mg, 0.49 mmol), and tert-butyl 4-(2-(tosyloxy)ethyl)piperidine-1-carboxylate (376 mg, 0.98 mmol) were suspended in DMF (3.2 ml). Then, potassium carbonate (203 mg, 1.47 mmol), and potassium iodide (163 mg, 0.98 mmol) was added and then stirred at 70 °C for 16 hours. Distilled water (10 ml) was added to the reaction solution, followed by extraction with EtOAc (10 ml x 2). The organic layer was washed with brine (10 ml x 2), dried over anhydrous magnesium sulfate, filtered, and concentrated under reduced pressure to obtain a residue. The obtained residue was subjected to MPLC (0-10% MeOH/DCM) to give 246 mg (68%) of a pale yellow solid.

### Step 6: Synthesis of (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperidin-4-yl)ethoxy)benzyl)pyrrolidine-2-carboxamide

Tert-butyl 4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-1-carboxylate (245 mg, 0.33 mmol) wase suspended in DCM (3.3 ml). Then, 4 M HCl in dioxane (1.65 ml) was added and then stirred at room temperature for 1 hour. A saturated aqueous solution of NaHCOs (10 ml) was added to the reaction solution, followed by extraction with DCM (5 ml x 2). The organic layer was washed with brine (5 ml x 2), dried over anhydrous magnesium sulfate, filtered and concentrated under reduced pressure to obtain 129 mg (61%) of a yellow solid.

### Step 7: Synthesis of tert-butyl ((1-(5-((2-chloro-3-(2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidin-1-yl)-2-oxoacetamido)phenyl)thio)pyrazin-2-yl)-4-methylpiperidin-4-yl)methyl)carbamate

2-((3-((5-(4-(((tert-butoxycarbonyl)amino)methyl)-4-methylpiperidin-1-yl)pyrazin-2-yl)thio)-2-chlorophenyl)amino)-2-oxoacetic acid (25 mg 0.05 mmol), (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperidin-4-yl)ethoxy)benzyl)pyrrolidine-2-carboxamide (30 mg, 0.05 mmol), EDCI (11 mg, 0.06 mmol), HOBt (8 mg, 0.06 mmol), and DIPEA (0.03 ml, 0.18 mmol) were suspended in DMF (0.75 ml) and then stirred at room temperature for 16 hours. Distilled water (3 ml) was added to the reaction solution, followed by extraction with EtOAc (3 ml x 2). The organic layer was washed with brine (3 ml x 2), dried over anhydrous sodium sulfate, filtered, and concentrated under reduced pressure. The resulting residue was subjected to MPLC (0-7% MeOH/DCM) to obtain 6.9 mg (13%) of an ivory solid.

### Step 8: Synthesis of (2S,4R)-N-(2-(2-(1-(2-((3-((5-(4-(aminomethyl)-4-methylpiperidin-1-yl)pyrazin-2-yl)thio)-2-chlorophenyl)amino)-2-oxoacetyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl ((1-(5-((2-chloro-3-(2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidin-1-yl)-2-oxoacetamido)phenyl)thio)pyrazin-2-yl)-4-methylpiperidin-4-yl)methyl)carbamate (6.9 mg, 0.005 mmol) were suspended in DCM (0.6 ml). Then, 4 M HCl in dioxane (0.014 ml) was added and then stirred at room temperature for 2 hours. A saturated aqueous solution of NaHCOs (1 ml) was added to the reaction solution, followed by extraction with 10% MeOH/DCM (5 ml x 2). The organic layer was washed with brine (1 ml x 2), dried over anhydrous magnesium sulfate, filtered and concentrated under reduced pressure to obtain a residue. The obtained residue was subjected to MPLC (amine-silica, 0-4% MeOH/DCM) to obtain 4.2 mg (67%) of an ivory solid.

### Example 28. (2S,4R)-N-(2-(3-(1-(2-((3-((5-(4-(aminomethyl)-4-methylpiperidin-1-yl)pyrazin-2-yl)thio)-2-chlorophenyl)amino)-2-oxoacetyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

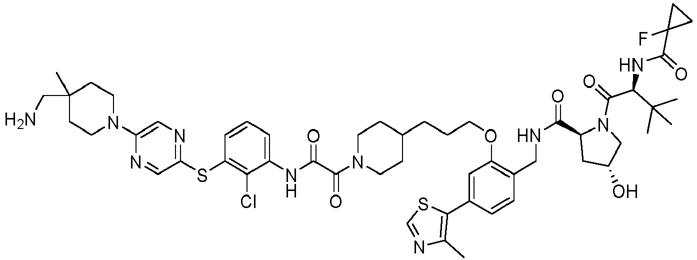

Example 28 was synthesized in the same way as in Example 27, using 3-(piperidin-4-yl)propan-1-ol instead of 2-(piperidin-4-yl)ethan-1-ol.

### Example 29. (2S,4R)-N-(2-((1-(3-((3-((5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

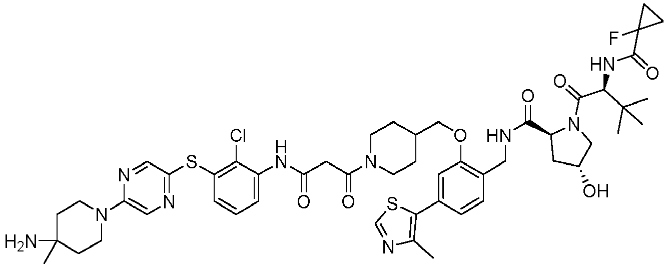

Example 29 was synthesized in the same way as in Example 27, using tert-butyl ((1-(5-((3-amino-2-chlorophenyl)thio)pyrazin-2-yl)-4-methylpiperidin-4-yl)methyl)carbamate (Intermediate 1) and piperidin-4-ylmethanol instead of tert-butyl ((1-(5-((3-amino-2-chlorophenyl)thio)pyrazin-2-yl)-4-methylpiperidin-4-yl)methyl)carbamate (Intermediate 3) and ethyl chloroglyoxylate, respectively.

### Example 30. (2S,4R)-N-(2-((1-(3-((3-((3-amino-5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

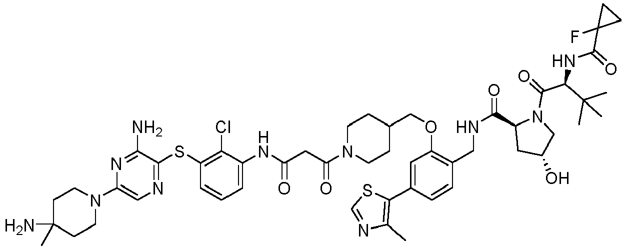

Example 30 was synthesized in the same way as in Example 27, using tert-butyl (1-(6-amino-5-((3-amino-2-chlorophenyl)thio)pyrazin-2-yl)-4-methylpiperidin-4-yl)carbamate (Intermediate 2), ethyl 3-chloro-3-oxopropanoate and piperidin-4-ylmethanol instead of tert-butyl ((1-(5-((3-amino-2-chlorophenyl)thio)pyrazin-2-yl)-4-methylpiperidin-4-yl)methyl)carbamate (Intermediate 3), ethyl chloroglyoxylate, and 2-(piperidin-4-yl)ethan-1-ol, respectively.

### Example 31. (2S,4R)-N-(2-((1-(3-((3-((5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

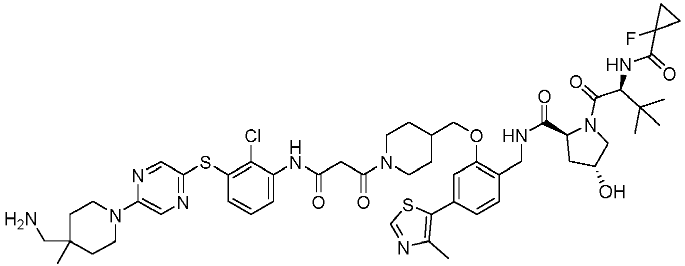

Example 31 was synthesized in the same way as in Example 27, using ethyl 3-chloro-3-oxopropanoate and piperidin-4-ylmethanol instead of ethyl chloroglyoxylate and 2-(piperidin-4-yl)ethan-1-ol, respectively.

### Example 32. (2S,4R)-N-(2-((1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

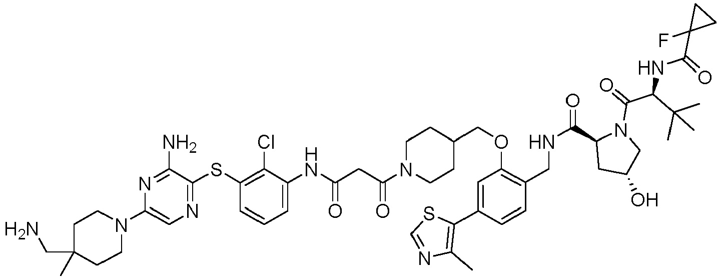

Example 32 was synthesized in the same way as in Example 27, using tert-butyl ((1-(6-amino-5-((3-amino-2-chlorophenyl)thio)pyrazin-2-yl)-4-methylpiperidin-4-yl)methyl)carbamate (Intermediate 4), ethyl 3-chloro-3-oxopropanoate and piperidin-4-ylmethanol instead of tert-butyl ((1-(5-((3-amino-2-chlorophenyl)thio)pyrazin-2-yl)-4-methylpiperidin-4-yl)methyl)carbamate (Intermediate 3), ethyl chloroglyoxylate, and 2-(piperidin-4-yl)ethan-1-ol, respectively.

### Example 33. (2S,4R)-1-((S)-2-acetamido-3,3-dimethylbutanoyl)-N-(2-((1-(3-((3-((5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-4-hydroxypyrrolidine-2-carboxamide

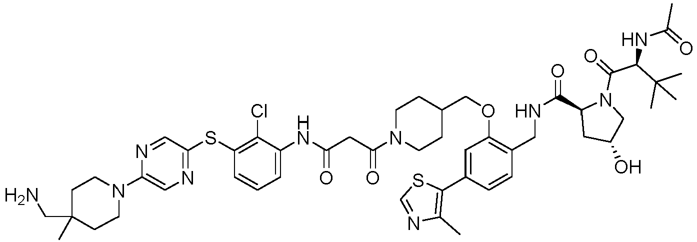

Example 33 was synthesized in the same way as in Example 27, using ethyl 3-chloro-3-oxopropanoate, piperidin-4-ylmethanol, and (2S,4R)-1-((S)-2-acetamido-3,3-dimethylbutanoyl)-4-hydroxy-N-(2-hydroxy-4-(4-methylthiazol-5-yl)benzyl)pyrrolidine-2-carboxamide (Intermediate 9) instead of ethyl chloroglyoxylate, 2-(piperidin-4-yl)ethan-1-ol, and (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 7), respectively.

### Example 34. (2S,4R)-N-(2-(2-(1-(3-((3-((5-(4-amino-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

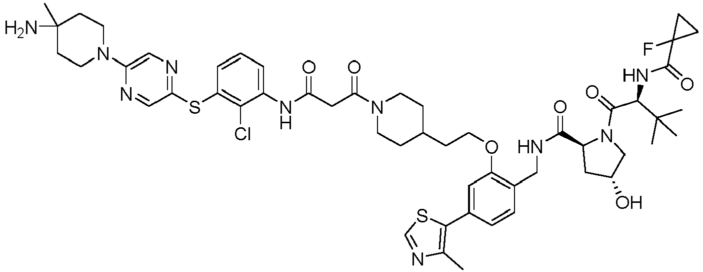

Example 34 was synthesized in the same way as in Example 27, using tert-butyl ((1-(5-((3-amino-2-chlorophenyl)thio)pyrazin-2-yl)-4-methylpiperidin-4-yl)methyl)carbamate (Intermediate 1) and ethyl 3-chloro-3-oxopropanoate instead of tert-butyl ((1-(5-((3-amino-2-chlorophenyl)thio)pyrazin-2-yl)-4-methylpiperidin-4-yl)methyl)carbamate (Intermediate 3) and ethyl chloroglyoxylate, respectively.

### Example 35. (2S,4R)-N-(2-(2-(1-(3-((3-((3-amino-5-(4-amino-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

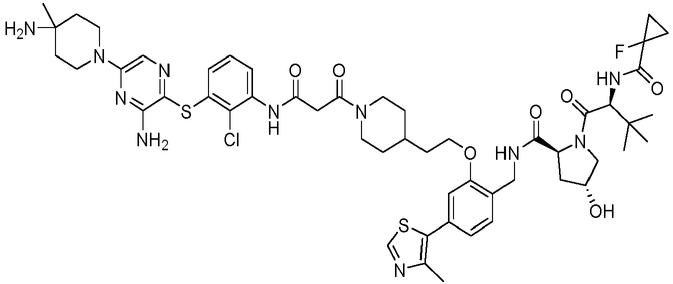

Example 35 was synthesized in the same way as in Example 27, using tert-butyl (1-(6-amino-5-((3-amino-2-chlorophenyl)thio)pyrazin-2-yl)-4-methylpiperidin-4-yl)carbamate (Intermediate 2) and ethyl 3-chloro-3-oxopropanoate instead of tert-butyl ((1-(5-((3-amino-2-chlorophenyl)thio)pyrazin-2-yl)-4-methylpiperidin-4-yl)methyl)carbamate (Intermediate 3) and ethyl chloroglyoxylate, respectively.

### Example 36. (2S,4R)-N-(2-(2-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

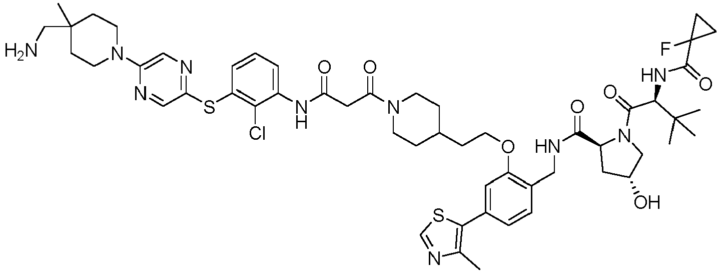

Example 36 was synthesized in the same way as in Example 27, using ethyl 3-chloro-3-oxopropanoate instead of ethyl chloroglyoxylate.

### Example 37. (2S,4R)-N-(2-(2-(1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

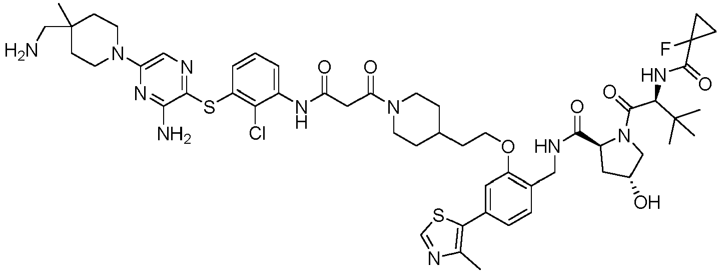

Example 37 was synthesized in the same way as in Example 27, using tert-butyl ((1-(6-amino-5-((3-amino-2-chlorophenyl)thio)pyrazin-2-yl)-4-methylpiperidin-4-yl)methyl)carbamate (Intermediate 4) and ethyl 3-chloro-3-oxopropanoate instead of tert-butyl ((1-(5-((3-amino-2-chlorophenyl)thio)pyrazin-2-yl)-4-methylpiperidin-4-yl)methyl)carbamate (Intermediate 3) and ethyl chloroglyoxylate, respectively.

### Example 38. (2S,4R)-1-((S)-2-acetamido-3,3-dimethylbutanoyl)-N-(2-(2-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-4-hydroxypyrrolidine-2-carboxamide

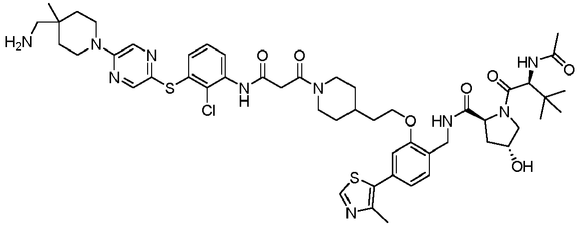

Example 38 was synthesized in the same way as in Example 27, using ethyl 3-chloro-3-oxopropanoate and (2S,4R)-1-((S)-2-acetamido-3,3-dimethylbutanoyl)-4-hydroxy-N-(2-hydroxy-4-(4-methylthiazol-5-yl)benzyl)pyrrolidine-2-carboxamide (Intermediate 9) instead of ethyl chloroglyoxylate and (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 7), respectively.

### Example 39. (2S,4R)-N-(2-((1-(3-((3-((5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

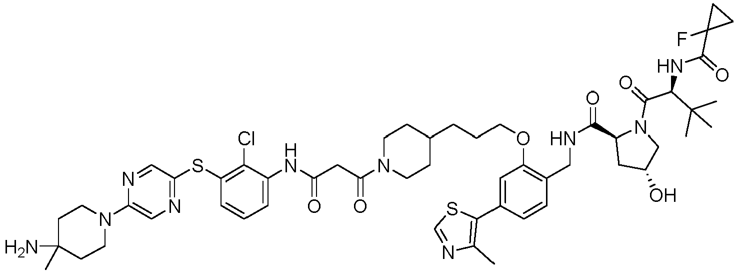

Example 39 was synthesized in the same way as in Example 27, using tert-butyl ((1-(5-((3-amino-2-chlorophenyl)thio)pyrazin-2-yl)-4-methylpiperidin-4-yl)methyl)carbamate (Intermediate 1), ethyl 3-chloro-3-oxopropanoate and 3-(piperidin-4-yl)propan-1-ol instead of tert-butyl ((1-(5-((3-amino-2-chlorophenyl)thio)pyrazin-2-yl)-4-methylpiperidin-4-yl)methyl)carbamate (Intermediate 3), ethyl chloroglyoxylate and 2-(piperidin-4-yl)ethan-1-ol, respectively.

### Example 40. (2S,4R)-N-(2-(3-(1-(3-((3-((3-amino-5-(4-amino-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

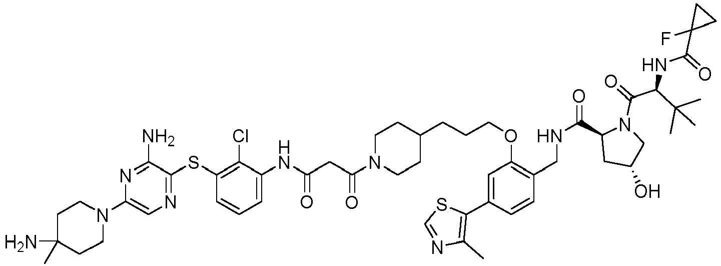

Example 40 was synthesized in the same way as in Example 27, using tert-butyl (1-(6-amino-5-((3-amino-2-chlorophenyl)thio)pyrazin-2-yl)-4-methylpiperidin-4-yl)carbamate (Intermediate 2), ethyl 3-chloro-3-oxopropanoate and 3-(piperidin-4-yl)propan-1-ol instead of tert-butyl ((1-(5-((3-amino-2-chlorophenyl)thio)pyrazin-2-yl)-4-methylpiperidin-4-yl)methyl)carbamate (Intermediate 3), ethyl chloroglyoxylate and 2-(piperidin-4-yl)ethan-1-ol, respectively.

### Example 41. (2S,4R)-N-(2-(3-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

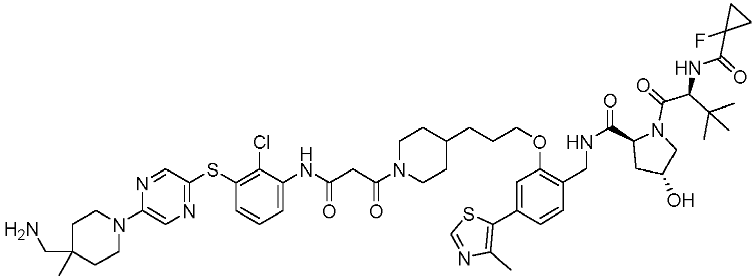

Example 41 was synthesized in the same way as in Example 27, using ethyl 3-chloro-3-oxopropanoate and 3-(piperidin-4-yl)propan-1-ol instead of ethyl chloroglyoxylate and 2-(piperidin-4-yl)ethan-1-ol, respectively.

### Example 42. (2S,4R)-N-(2-(3-(1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

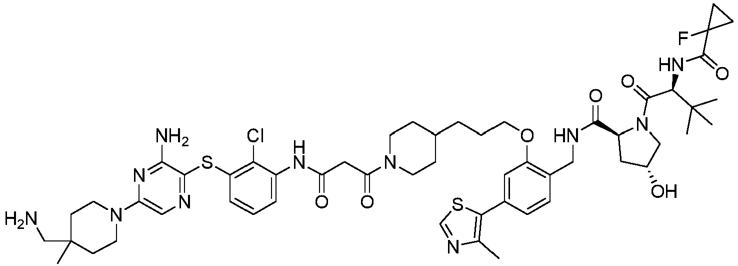

Example 42 was synthesized in the same way as in Example 27, using tert-butyl ((1-(6-amino-5-((3-amino-2-chlorophenyl)thio)pyrazin-2-yl)-4-methylpiperidin-4-yl)methyl)carbamate (Intermediate 4), ethyl 3-chloro-3-oxopropanoate and 3-(piperidin-4-yl)propan-1-ol instead of tert-butyl ((1-(5-((3-amino-2-chlorophenyl)thio)pyrazin-2-yl)-4-methylpiperidin-4-yl)methyl)carbamate (Intermediate 3), ethyl chloroglyoxylate and 2-(piperidin-4-yl)ethan-1-ol, respectively.

### Example 43. (2S,4R)-1-((S)-2-acetamido-3,3-dimethylbutanoyl)-N-(2-(3-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-4-hydroxypyrrolidine-2-carboxamide

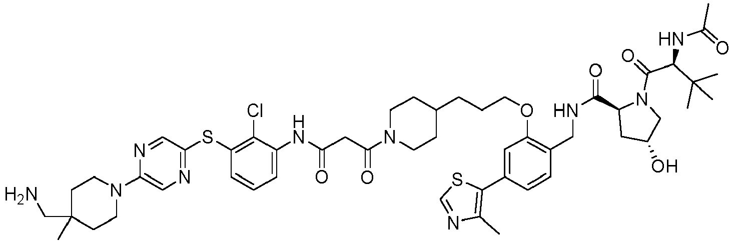

Example 43 was synthesized in the same way as in Example 27, using ethyl 3-chloro-3-oxopropanoate, 3-(piperidin-4-yl)propan-1-ol and (2S,4R)-1-((S)-2-acetamido-3,3-dimethylbutanoyl)-4-hydroxy-N-(2-hydroxy-4-(4-methylthiazol-5-yl)benzyl)pyrrolidine-2-carboxamide (Intermediate 9) instead of ethyl chloroglyoxylate, 2-(piperidin-4-yl)ethan-1-ol and (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 7), respectively.

### Example 44. (2S,4R)-N-(2-((1-(4-((3-((5-(4-(aminomethyl)-4-methylpiperidin-1-yl)pyrazin-2-yl)thio)-2-chlorophenyl)amino)-4-oxobutanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

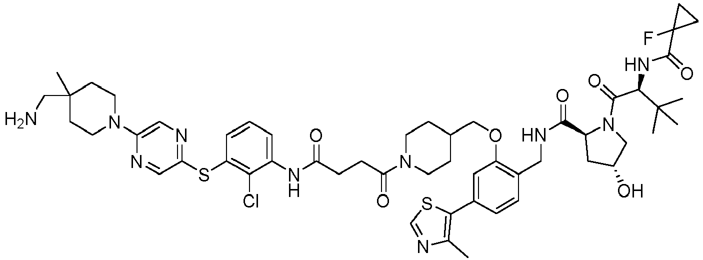

Example 44 was synthesized in the same way as in Example 27, using methyl 4-chloro-4-oxobutanoate and piperidin-4-ylmethanol instead of ethyl chloroglyoxylate and 2-(piperidin-4-yl)ethan-1-ol, respectively.

### Example 45. (2S,4R)-N-(2-(2-(1-(4-((3-((5-(4-(aminomethyl)-4-methylpiperidin-1-yl)pyrazin-2-yl)thio)-2-chlorophenyl)amino)-4-oxobutanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

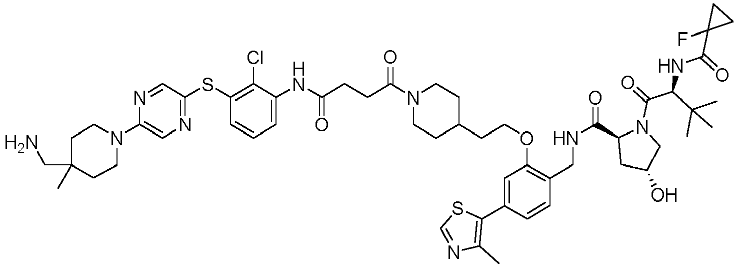

Example 45 was synthesized in the same way as in Example 27, using methyl 4-chloro-4-oxobutanoate instead of ethyl chloroglyoxylate.

### Example 46. (2S,4R)-N-(2-((1-(1-((3-((5-(4-(aminomethyl)-4-methylpiperidin-1-yl)pyrazin-2-yl)thio)-2-chlorophenyl)carbamoyl)cyclopropane-1-carbonyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

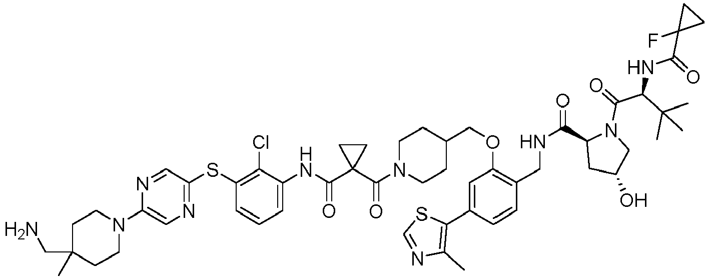

Example 46 was synthesized in the same way as in Example 27, using methyl 1-(chlorocarbonyl)cyclopropane-1-carboxylate and piperidin-4-ylmethanol instead of ethyl chloroglyoxylate and 2-(piperidin-4-yl)ethan-1-ol, respectively.

### Example 47. (2S,4R)-N-(2-(2-(1-(1-((3-((5-(4-(aminomethyl)-4-methylpiperidin-1-yl)pyrazin-2-yl)thio)-2-chlorophenyl)carbamoyl)cyclopropane-1-carbonyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

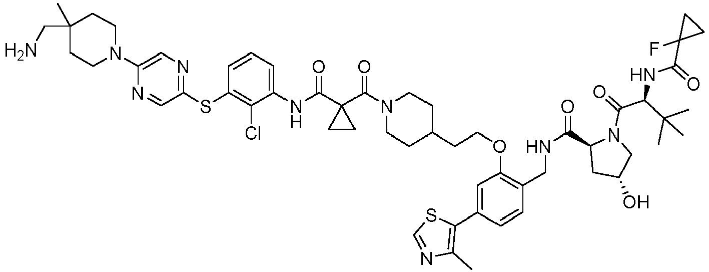

Example 47 was synthesized in the same way as in Example 27, using methyl 1-(chlorocarbonyl)cyclopropane-1-carboxylate instead of ethyl chloroglyoxylate.

### Example 48. (2S,4R)-N-(2-(3-(1-(1-((3-((5-(4-(aminomethyl)-4-methylpiperidin-1-yl)pyrazin-2-yl)thio)-2-chlorophenyl)carbamoyl)cyclopropane-1-carbonyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

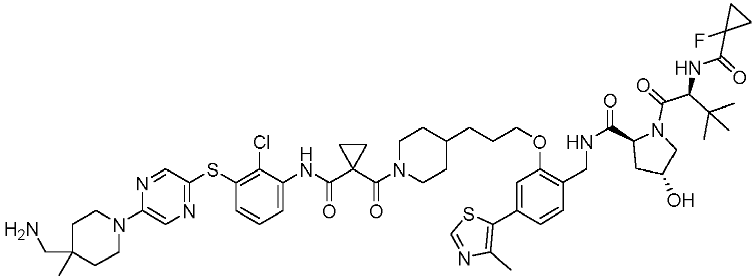

Example 48 was synthesized in the same way as in Example 27, using methyl 1-(chlorocarbonyl)cyclopropane-1-carboxylate and 3-(piperidin-4-yl)propan-1-ol instead of ethyl chloroglyoxylate and 2-(piperidin-4-yl)ethan-1-ol, respectively.

### Example 49. (2S,4R)-N-(2-(2-(1-(3-((3-((5-(4-(aminomethyl)-4-methylpiperidin-1-yl)pyrazin-2-yl)thio)-2-chlorophenyl)amino)-2,2-dimethyl-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

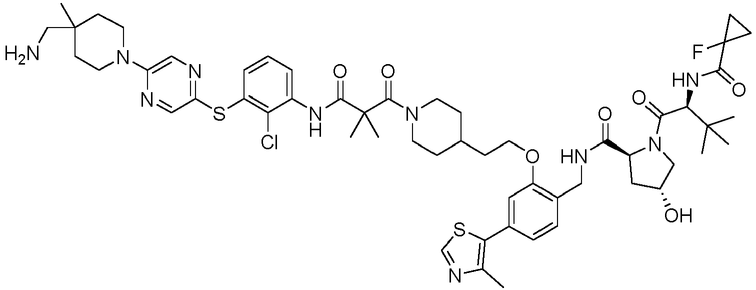

Example 49 was synthesized in the same way as in Example 27, using methyl 3-chloro-2,2-dimethyl-3-oxopropanoate.

### Example 50. (2S,4R)-N-(2-((7-(3-((3-((5-(4-(aminomethyl)-4-methylpiperidin-1-yl)pyrazin-2-yl)thio)-2-chlorophenyl)amino)-3-oxopropanoyl)-7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

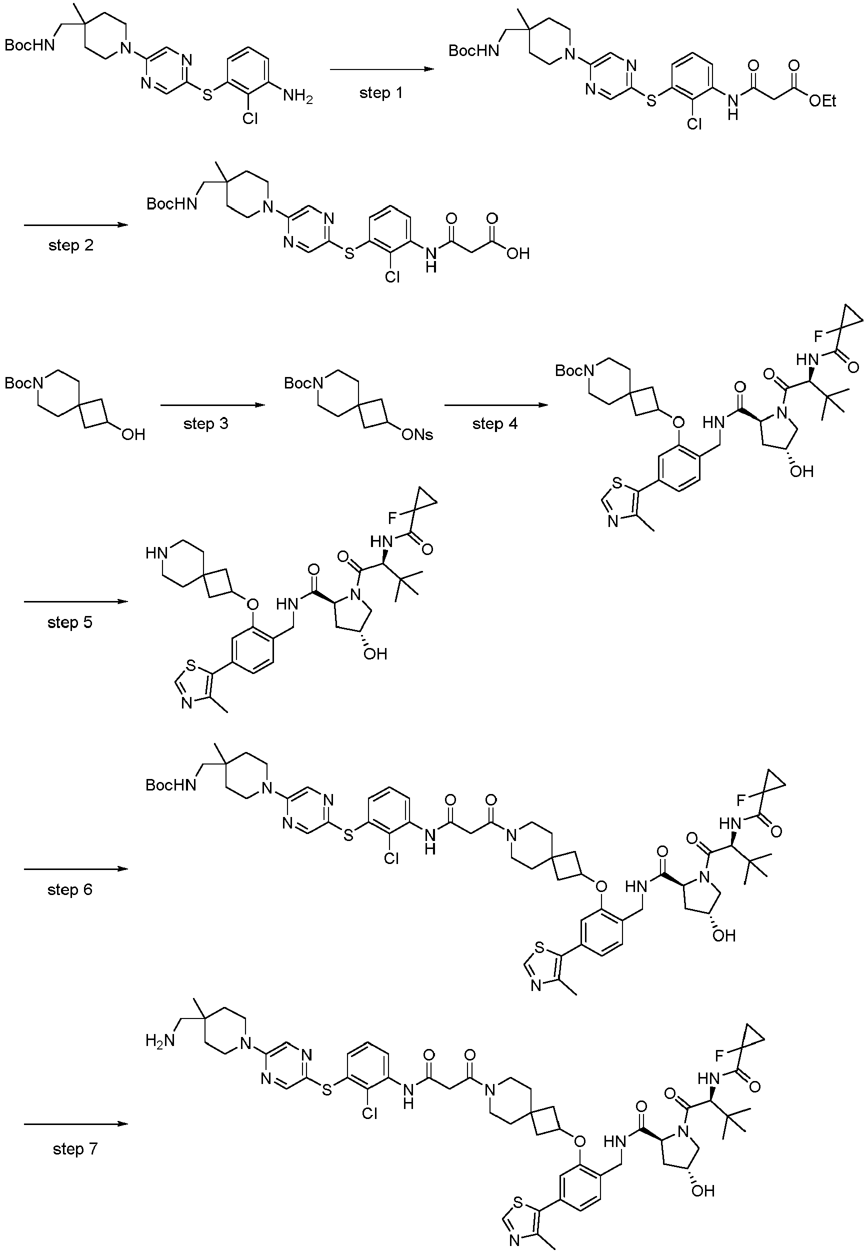

### Step 1: Synthesis of ethyl 3-((3-((5-(4-(((tert-butoxycarbonyl)amino)methyl)-4-methylpiperidin-1-yl)pyrazin-2-yl)thio)-2-chlorophenyl)amino)-3-oxopropanoate

Tert-butyl ((1-(5-((3-amino-2-chlorophenyl)thio)pyrazin-2-yl)-4-methylpiperidin-4-yl)methyl)carbamate (Intermediate 3, 240 mg, 0.52 mmol) was suspended in DCM (3.2 ml). Then, ethyl malonyl chloride (0.08 ml, 0.62 mmol) was slowly added at 0 °C and stirred for 30 minutes. A saturated aqueous solution of NaHCOs (10 ml) was added to the reaction solution, followed by extraction with DCM (10 ml x 2). The organic layer was dried over anhydrous sodium sulfate, filtered, and concentrated under reduced pressure. The resulting residue was subjected to MPLC (3-35% EtOAc/Hexane) to obtain 206 mg (69%) of an ivory solid.

### Step 2: Synthesis of 3-((3-((5-(4-(((tert-butoxycarbonyl)amino)methyl)-4-methylpiperidin-1-yl)pyrazin-2-yl)thio)-2-chlorophenyl)amino)-3-oxopropanoic acid

Ethyl 3-((3-((5-(4-(((tert-butoxycarbonyl)amino)methyl)-4-methylpiperidin-1-yl)pyrazin-2-yl)thio)-2-chlorophenyl)amino)-3-oxopropanoate (50 mg, 0.09 mmol), and lithium hydroxide (11 mg, 0.26 mmol) were suspended in THF : MeOH : H₂O = 3 : 2 : 1 (0.34 ml) and then stirred at room temperature for 2 hours. The reaction solution was adjusted to pH = 2 using 1N HCl aqueous solution, and then extracted with EtOAc (10 ml x 2). The organic layer was dried over anhydrous sodium sulfate, filtered, and concentrated under reduced pressure to obtain 46 mg (97%) of an ivory solid.

### Step 3: Synthesis of tert-butyl 2-(((2-nitrophenyl)sulfonyl)oxy)-7-azaspiro[3.5]nonane-7-carboxylate

Tert-butyl 2-hydroxy-7-azaspiro[3.5]nonane-7-carboxylate (2 g, 8.29 mmol), and triethylamine (2.3 ml, 16.58 mmol) were suspended in DCM (16 ml). Then, 2-nitrobenzenesulfonyl chloride (1.84 g, 8.29 mmol) was added and then stirred at room temperature for 2 hours. Distilled water (30 ml) was added to the reaction solution, followed by extraction with DCM (50 ml). The organic layer was washed with brine (30 ml), dried over anhydrous sodium sulfate, filtered, and concentrated under reduced pressure. The resulting residue was subjected to MPLC (1-40% EtOAc/Hexane) to obtain 3.3 g (93%) of a colorless oil.

### Step 4: Synthesis of tert-butyl 2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)-7-azaspiro[3.5]nonane-7-carboxylate

(2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 7, 500 mg, 0.494 mmol), and tert-butyl 2-(((2-nitrophenyl)sulfonyl)oxy)-7-azaspiro[3.5]nonane-7-carboxylate (1.2 g, 2.82 mmol) were suspended in DMF (1.5 ml). Then, cesium carbonate (612 mg, 1.88 mmol) was added and then stirred at 50 °C for 16 hours. Distilled water (20 ml) was added to the reaction solution, followed by extraction with EtOAc (30 ml). The organic layer was washed with brine (20 ml), dried over anhydrous sodium sulfate, filtered, and concentrated under reduced pressure. The resulting residue was subjected to MPLC (0-4% MeOH/DCM) to obtain 675 mg (95%) of an ivory solid.

### Step 5: Synthesis of (2S,4R)-N-(2-((7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl 2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)-7-azaspiro[3.5]nonane-7-carboxylate (675 mg, 0.89 mmol) wase suspended in DCM (18 ml). Then, 4 M HCl in dioxane (4.4 ml) was added and then stirred at room temperature for 1 hour. A saturated aqueous solution of NaHCOs (10 ml) was added to the reaction solution, followed by extraction with DCM (15 ml x 2). The organic layer was dried over anhydrous sodium sulfate, filtered, and concentrated under reduced pressure to obtain 483 mg (82%) of an ivory solid.

### Step 6: Synthesis of tert-butyl ((1-(5-((2-chloro-3-(3-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)-7-azaspiro[3.5]nonan-7-yl)-3-oxopropanamido)phenyl)thio)pyrazin-2-yl)-4-methylpiperidin-4-yl)methyl)carbamate

3-((3-((5-(4-(((tert-butoxycarbonyl)amino)methyl)-4-methylpiperidin-1-yl)pyrazin-2-yl)thio)-2-chlorophenyl)amino)-3-oxopropanoic acid (16.5 mg 0.03 mmol), (2S,4R)-N-(2-((7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (19.4 mg, 0.03 mmol), EDCI (6.8 mg, 0.04 mmol), HOBt (4.8 mg, 0.04 mmol), and DIPEA (0.02 ml, 0.12 mmol) were suspended in DMF (0.1 ml) and then stirred at room temperature for 16 hours. Distilled water (7 ml) was added to the reaction solution, followed by extraction with EtOAc (10 ml). The organic layer was washed with brine (7 ml), dried over anhydrous sodium sulfate, filtered, and concentrated under reduced pressure. The resulting residue was subjected to MPLC (0-5% MeOH/DCM) to obtain 20 mg (42%) of an ivory solid.

### Step 7: Synthesis of (2S,4R)-N-(2-((7-(3-((3-((5-(4-(aminomethyl)-4-methylpiperidin-1-yl)pyrazin-2-yl)thio)-2-chlorophenyl)amino)-3-oxopropanoyl)-7-azaspiro [3.5] nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl ((1-(5-((2-chloro-3-(3-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)-7-azaspiro[3.5]nonan-7-yl)-3-oxopropanamido)phenyl)thio)pyrazin-2-yl)-4-methylpiperidin-4-yl)methyl)carbamate (20 mg, 0.017 mmol) wase suspended in DCM (1.7 ml). Then, 4 M HCl in dioxane (0.04 ml) was added and then stirred at room temperature for 2 hours. A saturated aqueous solution of NaHCOs (7 ml) was added to the reaction solution, followed by extraction with DCM (10 ml x 3). The organic layer was dried over anhydrous sodium sulfate, filtered, and concentrated under reduced pressure. The resulting residue was subjected to MPLC (amine-silica, 0-3% MeOH/DCM) to obtain 16 mg (87%) of an ivory solid.

### Example 51. (2S,4R)-N-(2-((7-(3-((3-((5-(4-(aminomethyl)-4-methylpiperidin-1-yl)pyrazin-2-yl)thio)-2-chlorophenyl)(methyl)amino)-3-oxopropanoyl)-7-azaspiro [3.5] nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

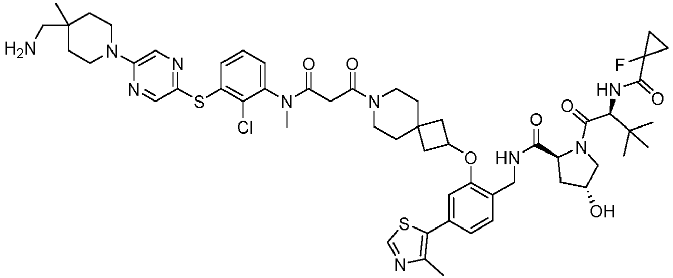

Example 51 was synthesized in the same way as in Example 50, using tert-butyl ((1-(5-((2-chloro-3-(methylamino)phenyl)thio)pyrazin-2-yl)-4-methylpiperidin-4-yl)methyl)carbamate (Intermediate 5) instead of tert-butyl ((1-(5-((3-amino-2-chlorophenyl)thio)pyrazin-2-yl)-4-methylpiperidin-4-yl)methyl)carbamate (Intermediate 3).

### Example 52. (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

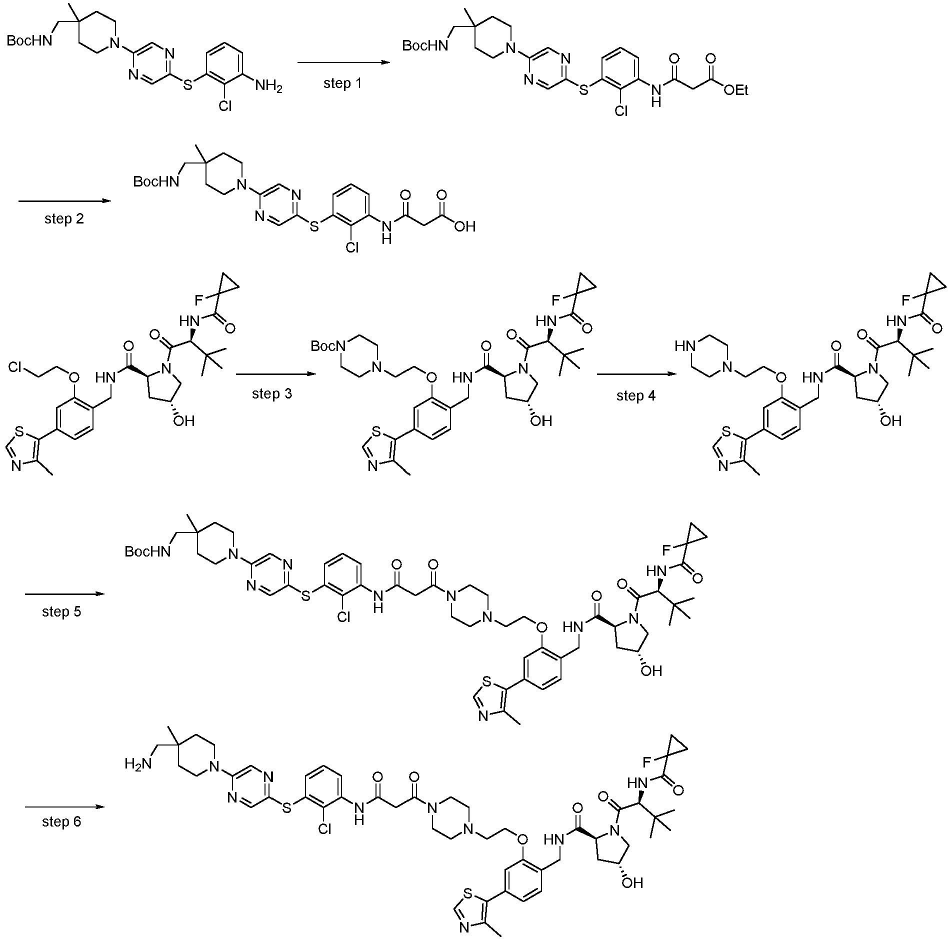

### Step 1: Synthesis of ethyl 3-((3-((5-(4-(((tert-butoxycarbonyl)amino)methyl)-4-methylpiperidin-1-yl)pyrazin-2-yl)thio)-2-chlorophenyl)amino)-3-oxopropanoate

Tert-butyl ((1-(5-((3-amino-2-chlorophenyl)thio)pyrazin-2-yl)-4-methylpiperidin-4-yl)methyl)carbamate (Intermediate 3, 240 mg, 0.52 mmol) wase suspended in DCM (3.2 ml). Then, ethyl malonyl chloride (0.08 ml, 0.62 mmol) was slowly added at 0 °C and stirred for 30 minutes. A saturated aqueous solution of NaHCOs (10 ml) was added to the reaction solution, followed by extraction with DCM (10 ml x 2). The organic layer was dried over anhydrous sodium sulfate, filtered and concentrated under reduced pressure to obtain a residue. The obtained residue was subjected to MPLC (3-35% EtOAc/Hexane) to give 206 mg (69%) of an ivory solid.

### Step 2: Synthesis of 3-((3-((5-(4-(((tert-butoxycarbonyl)amino)methyl)-4-methylpiperidin-1-yl)pyrazin-2-yl)thio)-2-chlorophenyl)amino)-3-oxopropanoic acid

Ethyl 3-((3-((5-(4-(((tert-butoxycarbonyl)amino)methyl)-4-methylpiperidin-1-yl)pyrazin-2-yl)thio)-2-chlorophenyl)amino)-3-oxopropanoate (50 mg, 0.09 mmol), and lithium hydroxide (11 mg, 0.26 mmol) were suspended in THF : MeOH : H₂O = 3 : 2 : 1 (0.34 ml) and then stirred at room temperature for 2 hours. The reaction solution was adjusted to pH = 2 using 1N HCl aqueous solution, and then extracted with EtOAc (10 ml x 2). The organic layer was dried over anhydrous sodium sulfate, filtered, and concentrated under reduced pressure to obtain 46 mg (97%) of an ivory solid.

### Step 3. Synthesis of tert-butyl 4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-1-carboxylate

(2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10, 100 mg, 0.16 mmol), tert-butyl piperazine-1-carboxylate (38 mg, 0.2 mmol), potassium carbonate (70 mg, 0.5 mmol), and sodium iodide (26 mg, 0.16 mmol) were suspended in acetonitrile (1 ml) and then stirred at 60 °C for 16 hours. Distilled water (10 ml) was added to the reaction solution, followed by extraction with EtOAc (10 ml x 2). The organic layer was washed with brine (10 ml x 2), dried over anhydrous magnesium sulfate, filtered, and concentrated under reduced pressure to obtain a residue. The obtained residue was subjected to MPLC (0-10% MeOH/DCM) to give 89 mg (72%) of a white solid.

### Step 4: Synthesis of (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide

Tert-butyl 4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-1-carboxylate (86 mg, 0.12 mmol) was suspended in DCM (1.15 ml). Then, 4 M HCl in dioxane (0.58 ml) was added and then stirred at room temperature for 1 hour. A saturated aqueous solution of NaHCOs (15 ml) was added to the reaction solution, followed by extraction with 10% MeOH/DCM (15 ml x 2). The organic layer was washed with brine (15 ml x 2), dried over anhydrous magnesium sulfate, filtered and concentrated under reduced pressure to obtain 69 mg (93%) of an ivory solid.

### Step 5: Synthesis of tert-butyl ((1-(5-((2-chloro-3-(3-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)-3-oxopropanamido)phenyl)thio)pyrazin-2-yl)-4-methylpiperidin-4-yl)methyl)carbamate

3-((3-((5-(4-(((tert-butoxycarbonyl)amino)methyl)-4-methylpiperidin-1-yl)pyrazin-2-yl)thio)-2-chlorophenyl)amino)-3-oxopropanoic acid (25 mg 0.05 mmol), (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide (29 mg, 0.05 mmol), EDCI (10 mg, 0.05 mmol), HOBt (7 mg, 0.05 mmol), and DIPEA (0.02 ml, 0.02 mmol) were suspended in DMF (0.15 ml) and then stirred at room temperature for 16 hours. Distilled water (10 ml) was added to the reaction solution, followed by extraction with EtOAc (5 ml x 2). The organic layer was washed with brine (5 ml x 2), dried over anhydrous sodium sulfate, filtered, and concentrated under reduced pressure to obtain a residue. The obtained residue was subjected to MPLC (10% MeOH/DCM) to give 19.3 mg (36%) of an ivory solid.

### Step 6: Synthesis of (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl ((1-(5-((2-chloro-3-(3-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)-3-oxopropanamido)phenyl)thio)pyrazin-2-yl)-4-methylpiperidin-4-yl)methyl)carbamate (17.1 mg, 0.01 mmol) was suspended in DCM (0.3 ml). Then, 4 M HCl in dioxane (0.07 ml) was added and stirred at room temperature for 1 hour. To the reaction solution, a saturated aqueous solution of NaHCOs (10 ml) was added, followed by extraction with 10% MeOH/DCM (5 ml x 2). The organic layer was washed with brine (5 ml x 2), dried over anhydrous magnesium sulfate, filtered, and concentrated under reduced pressure to obtain a residue. The obtained residue was subjected to MPLC (amine-silica, 0-5% MeOH/DCM) to obtain 13.5 mg (86%) of a white solid.

### Example 53. (2S,4R)-N-(2-(2-(4-(3-((3-((5-(4-amino-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

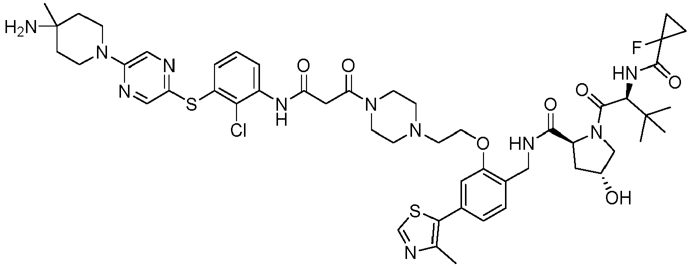

Example 53 was synthesized in the same way as in Example 52, using tert-butyl ((1-(5-((3-amino-2-chlorophenyl)thio)pyrazin-2-yl)-4-methylpiperidin-4-yl)methyl)carbamate (Intermediate 1) instead of tert-butyl ((1-(5-((3-amino-2-chlorophenyl)thio)pyrazin-2-yl)-4-methylpiperidin-4-yl)methyl)carbamate (Intermediate 3).

### Example 54. (2S,4R)-N-(2-(2-(4-(3-((3-((3-amino-5-(4-amino-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

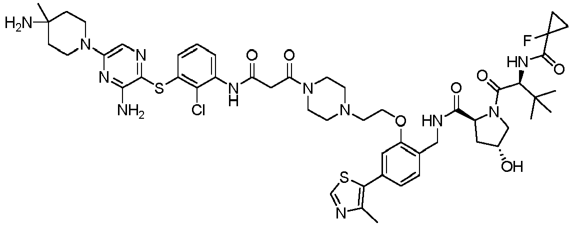

Example 54 was synthesized in the same way as in Example 52, using tert-butyl (1-(6-amino-5-((3-amino-2-chlorophenyl)thio)pyrazin-2-yl)-4-methylpiperidin-4-yl)carbamate (Intermediate 2) instead of tert-butyl ((1-(5-((3-amino-2-chlorophenyl)thio)pyrazin-2-yl)-4-methylpiperidin-4-yl)methyl)carbamate (Intermediate 3).

### Example 55. (2S,4R)-N-(2-(2-(4-(3-((3-((3-amino-5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

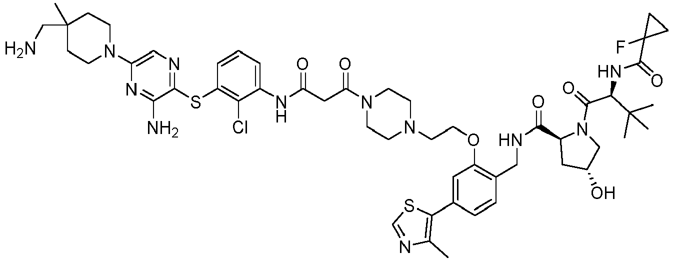

Example 55 was synthesized in the same way as in Example 52, using tert-butyl ((1-(6-amino-5-((3-amino-2-chlorophenyl)thio)pyrazin-2-yl)-4-methylpiperidin-4-yl)methyl)carbamate (Intermediate 4) instead of tert-butyl ((1-(5-((3-amino-2-chlorophenyl)thio)pyrazin-2-yl)-4-methylpiperidin-4-yl)methyl)carbamate (Intermediate 3).

### Example 56. (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(cyclopropanecarboxamido)-3,3-dimethylbutanoyl)-4-hydroxypyrrolidine-2-carboxamide

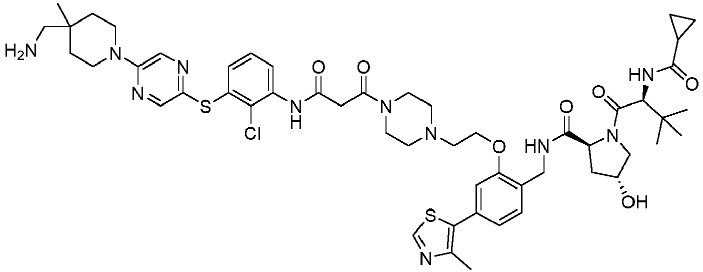

Example 56 was synthesized in the same way as in Example 52, using (2S,4R)-N-(2-(2-chloroethoxy)-4-(4-methylthiazol-5-yl)benzyl)-1-((S)-2-(cyclopropanecarboxamido)-3,3-dimethylbutanoyl)-4-hydroxypyrrolidine-2-carboxamide (Intermediate 15) instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10).

### Example 57. (2S,4R)-1-((S)-2-acetamido-3,3-dimethylbutanoyl)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-4-hydroxypyrrolidine-2-carboxamide

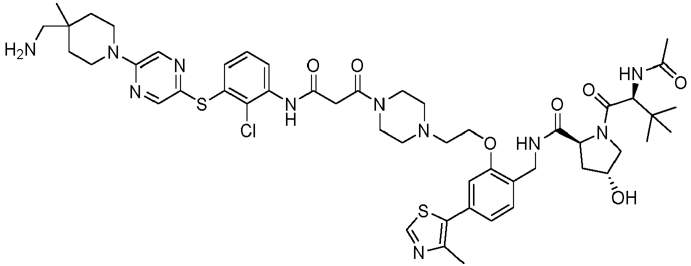

Example 57 was synthesized in the same way as in Example 52, using ((2S,4R)-1-((S)-2-acetamido-3,3-dimethylbutanoyl)-N-(2-(2-chloroethoxy)-4-(4-methylthiazol-5-yl)benzyl)-4-hydroxypyrrolidine-2-carboxamide (Intermediate 17) instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10).

### Example 58. (2S,4R)-N-(2-(2-(4-(3-((3-((5-(4-(aminomethyl)-4-methylpiperidin-1-yl)pyrazin-2-yl)thio)-2-chlorophenyl)amino)-2-methyl-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

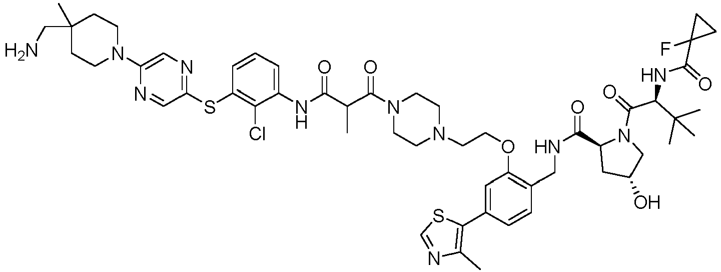

Example 58 was synthesized in the same way as in Example 52, using ethyl 3-chloro-2-methyl-3-oxopropanoate instead of ethyl malonyl chloride.

### Example 59. (2S,4R)-N-(2-(2-(4-(3-((3-((5-(4-(aminomethyl)-4-methylpiperidin-1-yl)pyrazin-2-yl)thio)-2-chlorophenyl)amino)-2,2-dimethyl-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

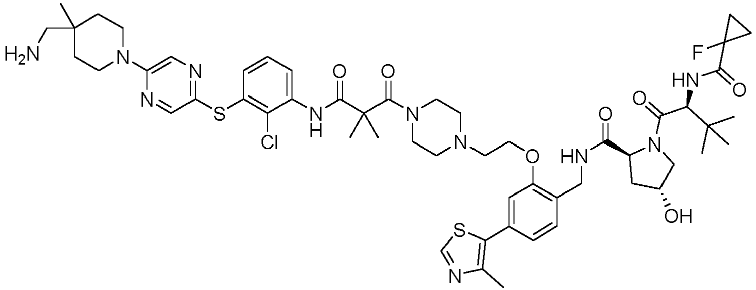

Example 59 was synthesized in the same way as in Example 52, using methyl 3-chloro-2,2-dimethyl-3-oxopropanoate instead of ethyl malonyl chloride.

### Example 60. (2S,4R)-N-(2-(3-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

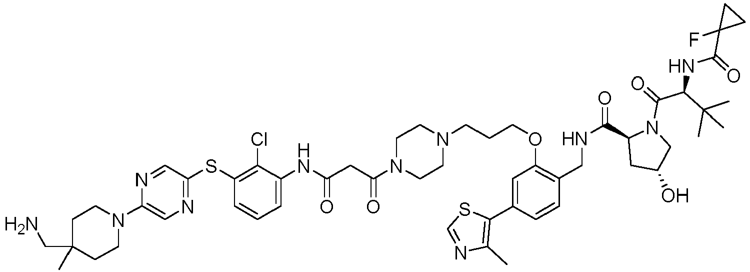

Example 60 was synthesized in the same way as in Example 52, using (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11) instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10).

### Example 61. (2S,4R)-1-((S)-2-acetamido-3,3-dimethylbutanoyl)-N-(2-(3-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)propoxy)-4-(4-methylthiazol-5-yl)benzyl)-4-hydroxypyrrolidine-2-carboxamide

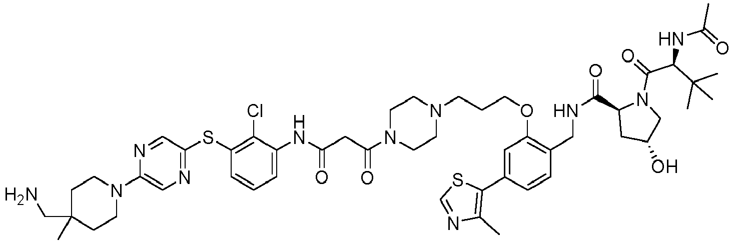

Example 61 was synthesized in the same way as in Example 52, using (2S,4R)-1-((S)-2-acetamido-3,3-dimethylbutanoyl)-N-(2-(3-chloropropoxy)-4-(4-methylthiazol-5-yl)benzyl)-4-hydroxypyrrolidine-2-carboxamide (Intermediate 18) instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10).

### Example 62. (2S,4R)-N-(2-(2-((R)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

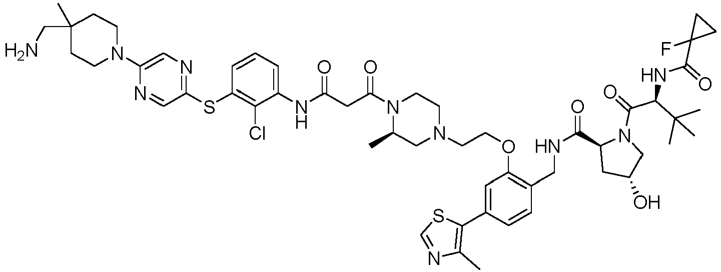

Example 62 was synthesized in the same way as in Example 52, using tert-butyl (R)-2-methylpiperazine-1-carboxylate instead of tert-butyl piperazine-1-carboxylate.

### Example 63. (2S,4R)-N-(2-(2-((S)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

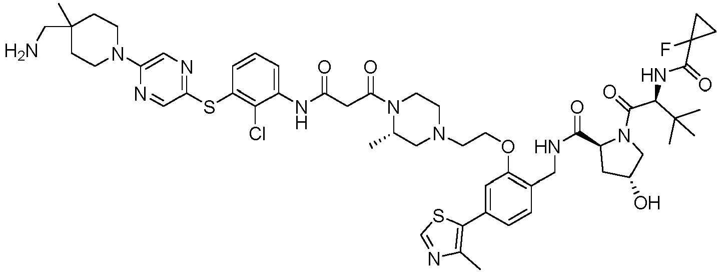

Example 63 was synthesized in the same way as in Example 52, using tert-butyl (S)-2-methylpiperazine-1-carboxylate instead of tert-butyl piperazine-1-carboxylate.

### Example 64. (2S,4R)-N-(2-(3-((S)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

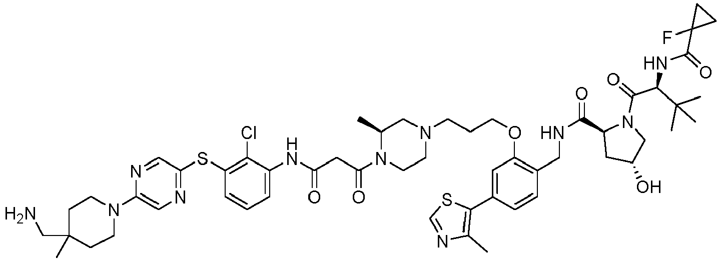

Example 64 was synthesized in the same way as in Example 52, using (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11) and tert-butyl (S)-2-methylpiperazine-1-carboxylate instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10) and tert-butyl piperazine-1-carboxylate, respectively.

### Example 65. ((2S,4R)-N-(2-(3-((R)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

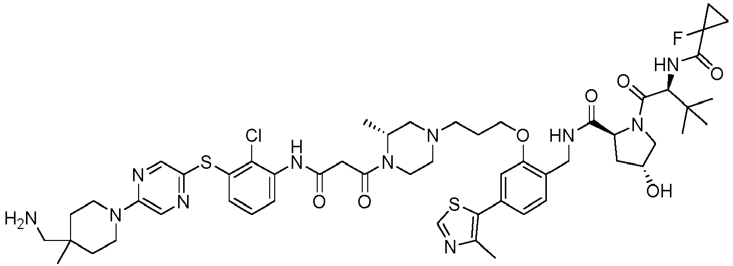

Example 65 was synthesized in the same way as in Example 52, using (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11) and tert-butyl (R)-2-methylpiperazine-1-carboxylate instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10) and tert-butyl piperazine-1-carboxylate, respectively.

### Example 66. (S)-methyl 4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate

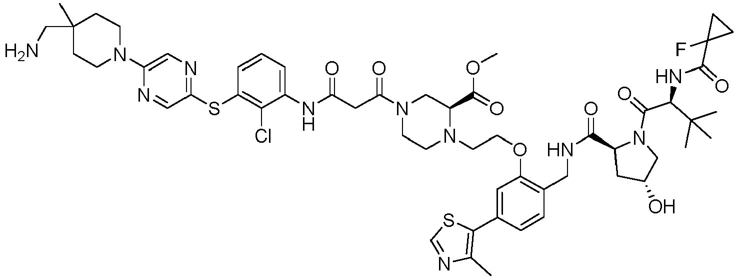

Example 66 was synthesized in the same way as in Example 52, using (S)-1-tert-butyl 3-methyl piperazine-1,3-dicarboxylate instead of tert-butyl piperazine-1-carboxylate.

### Example 67. methyl (R)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate

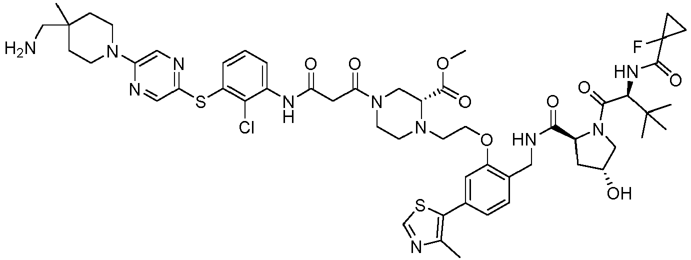

Example 67 was synthesized in the same way as in Example 52, using (R)-1-(tert-butyl) 3-methyl piperazine-1,3-dicarboxylate instead of tert-butyl piperazine-1-carboxylate.

### Example 68. (2S,4R)-N-(2-(2-(8-(3-((3-((5-(4-(aminomethyl)-4-methylpiperidin-1-yl)pyrazin-2-yl)thio)-2-chlorophenyl)amino)-3-oxopropanoyl)-3,8-diazabicyclo [3.2.1] octan-3-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

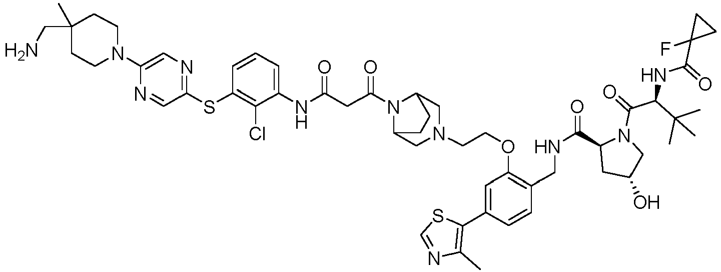

Example 68 was synthesized in the same way as in Example 52, using tert-butyl 3,8-diazabicyclo[3.2.1]octane-8-carboxylate instead of tert-butyl piperazine-1-carboxylate.

### Example 69. (S)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid

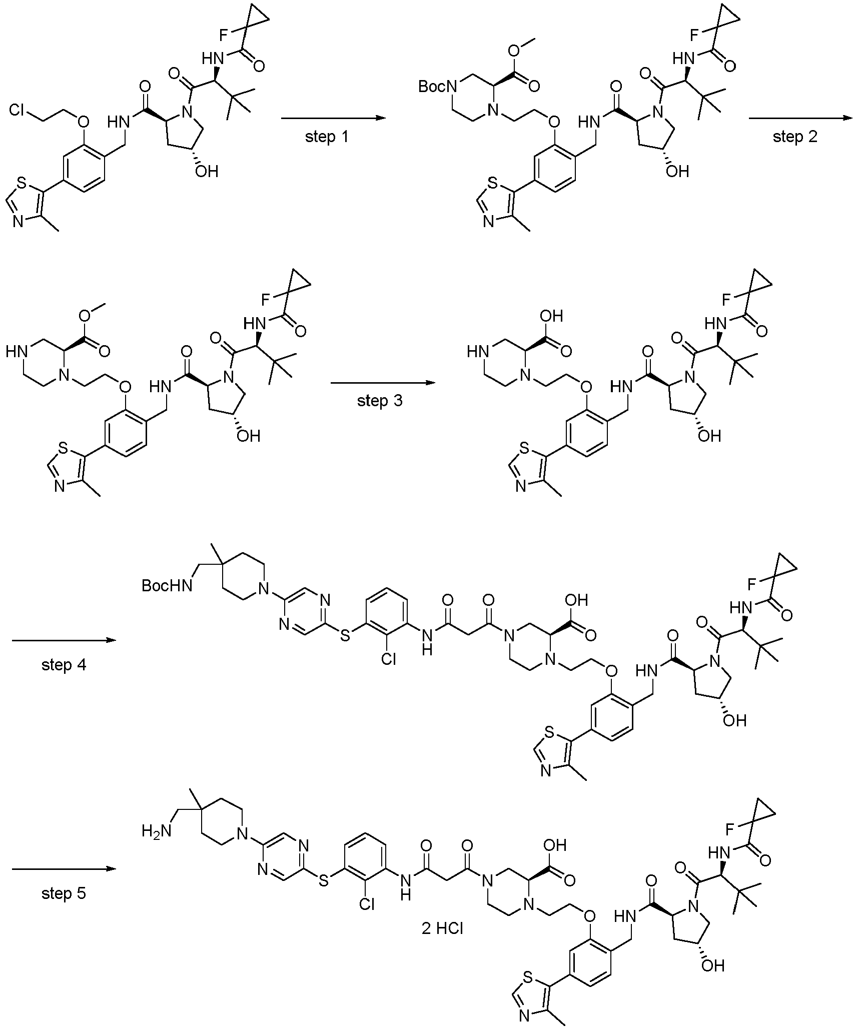

### Step 1: Synthesis of 1-(tert-butyl) 3-methyl (S)-4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-1,3-dicarboxylate\

(2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10, 100 mg, 0.16 mmol), 1-(tert-butyl) 3-methyl (S)-piperazine-1,3-dicarboxylate (82 mg, 0.34 mmol), potassium carbonate (70 mg, 0.50 mmol), and sodium iodide (56 mg, 0.34 mmol) were suspended in DMF (0.6 ml) and then stirred at 70 °C for 16 hours. Distilled water (10 ml) was added to the reaction solution, followed by extraction with EtOAc (15 ml). The organic layer was washed with brine (10 ml), dried over anhydrous sodium sulfate, filtered, and concentrated under reduced pressure to obtain a residue. The obtained residue was subjected to MPLC (0-4% MeOH/DCM) to give 91 mg (67%) of a white solid.

### Step 2: Synthesis of methyl (S)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate

1-(tert-butyl) 3-methyl (S)-4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-1,3-dicarboxylate (90.7 mg, 0.11 mmol) was suspended in DCM (1.1 ml). Then, 4 M HCl in dioxane (0.28 ml) was added and stirred at room temperature for 1 hour. A saturated aqueous solution of NaHCOs (10 ml) was added to the reaction solution, followed by extraction with DCM (20 ml × 2). The organic layer was dried over anhydrous sodium sulfate, filtered and concentrated under reduced pressure to obtain a residue. The obtained residue was subjected to MPLC (amine silica, 0-3% MeOH/DCM) to obtain 64 mg (80%) of an ivory solid.

### Step 3: Synthesis of (S)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid

Methyl (S)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate (64 mg 0.05 mmol), and lithium hydroxide (8 mg, 0.18 mmol) were suspended in THF : MeOH : H₂O = 3 : 2 : 1 (0.65 ml) and then stirred at room temperature for 16 hours. The reaction solution was adjusted to pH = 2 using 1N HCl aqueous solution and then concentrated under reduced pressure to obtain 82 mg (quant.) of an ivory solid.

### Step 4: Synthesis of (S)-4-(3-((3-((5-(4-(((tert-butoxycarbonyl)amino)methyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid

3-((3-((5-(4-(((tert-butoxycarbonyl)amino)methyl)-4-methylpiperidin-1-yl)pyrazin-2-yl)thio)-2-chlorophenyl)amino)-3-oxopropanoic acid (20 mg, 0.04 mmol), NHS (4.3 mg, 0.04 mmol), and DCC (8 mg, 0.04 mmol) were suspended in THF (0.4 ml) and then stirred at room temperature for 30 minutes. (S)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid (33.6 mg, 0.04 mmol), DIPEA (0.03 ml, 0.15 mmol), and DMF (0.4 ml) were added to the reaction solution and then stirred at room temperature for 16 hours. Distilled water (5 ml) was added to the reaction solution, followed by extraction with DCM (10 ml × 2). The organic layer was washed with brine (5 ml), dried over anhydrous sodium sulfate, filtered, and concentrated under reduced pressure to obtain a residue. The obtained residue was subjected to MPLC (0-15% MeOH/DCM -> C18 reversed phase silica, 10-50% ACN/water) to obtain 16.2 mg (36%) of a white solid.

### Step 5: Synthesis of (S)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid

(S)-4-(3-((3-((5-(4-(((tert-butoxycarbonyl)amino)methyl)-4-methylpiperidin-1-yl)pyrazin-2-yl)thio)-2-chlorophenyl)amino)-3-oxopropanoyl)-1-(2-(2-(((2S,4R)-1-((S)-2-(1 - fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid (14 mg, 0.01 mmol) was suspended in DCM (1.1 ml). 4 M HCl in dioxane (0.03 ml) was added and then stirred at room temperature for 2 hours. The reaction solution was concentrated under reduced pressure and dried *in vacuo* to obtain 7.5 mg (55%) of an ivory solid.

### Example 70. (R)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid

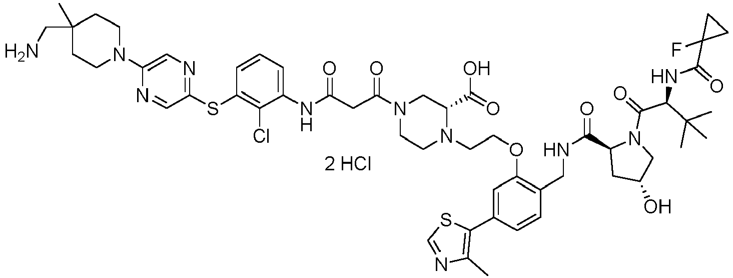

Example 70 was synthesized in the same way as in Example 69, using 1-(tert-butyl) 3-methyl (R)-piperazine-1,3-dicarboxylate instead of 1-(tert-butyl) 3-methyl (S)-piperazine-1,3-dicarboxylate.

### Example 71. (2S,4R)-N-(2-(3-(1-(2-((3-((5-(4-(aminomethyl)-4-methylpiperidin-1-yl)pyrazin-2-yl)thio)-2-chlorophenyl)amino)-2-oxoethyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

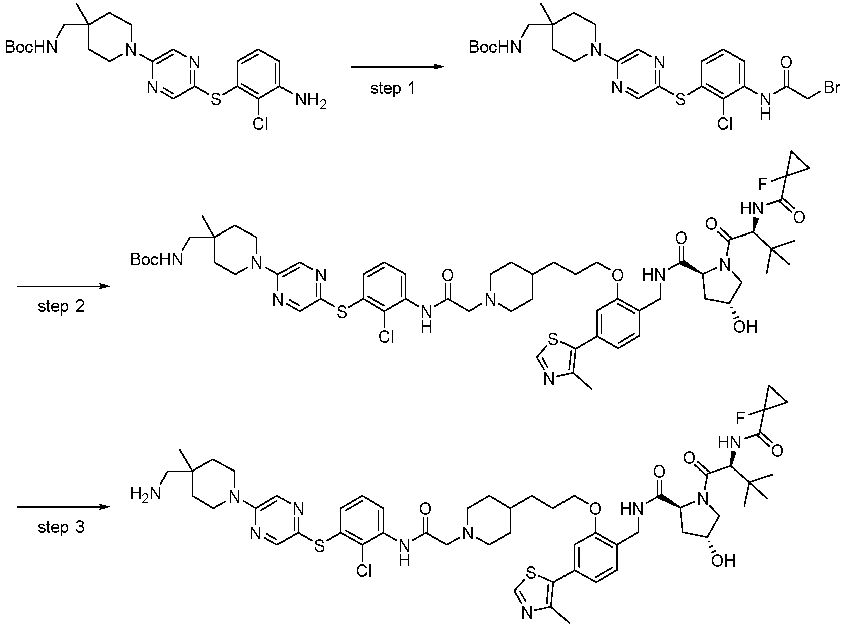

### Step 1: Synthesis of tert-butyl ((1-(5-((3-(2-bromoacetamido)-2-chlorophenyl)thio)pyrazin-2-yl)-4-methylpiperidin-4-yl)methyl)carbamate

Tert-butyl ((1-(5-((3-amino-2-chlorophenyl)thio)pyrazin-2-yl)-4-methylpiperidin-4-yl)methyl)carbamate (Intermediate 3, 20 mg, 0.04 mmol), and triethylamine (0.018 ml, 0.13 mmol) were suspended in DCM (0.2 ml). Then, bromoacetyl chloride (7.5 mg, 0.05 mmol) was slowly added at 0 °C and stirred for 30 minutes. A saturated aqueous solution of NaHCOs (2 ml) was added to the reaction solution, followed by extraction with DCM (5 ml × 2). The organic layer was washed with brine (5 ml × 2), dried over anhydrous magnesium sulfate, filtered, and concentrated under reduced pressure to obtain a residue. The obtained residue was subjected to MPLC (5-40% EtOAc/Hexane) to give 10 mg (40%) of an ivory solid.

### Step 2: Synthesis of tert-butyl ((1-(5-((2-chloro-3-(2-(4-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)piperidin-1-yl)acetamido)phenyl)thio)pyrazin-2-yl)-4-methylpiperidin-4-yl)methyl)carbamate

Tert-butyl ((1-(5-((3-(2-bromoacetamido)-2-chlorophenyl)thio)pyrazin-2-yl)-4-methylpiperidin-4-yl)methyl)carbamate (35 mg, 0.06 mmol), (2S,4R)-1-((S)-2-(1-fluorocyclopropane-1-carboxamido)-3,3-dimethylbutanoyl)-4-hydroxy-N-(4-(4-methylthiazol-5-yl)-2-(3-(piperidin-4-yl)propoxy)benzyl)pyrrolidine-2-carboxamide (20 mg, 0.03 mmol), potassium carbonate (13 mg, 0.09 mmol), and potassium iodide (10 mg, 0.06 mmol) were suspended in DMF (0.2 ml) and then stirred at 70 °C for 16 hours. Distilled water (10 ml) was added to the reaction solution, followed by extraction with EtOAc (5 ml × 2). The organic layer was washed with brine (5 ml × 2), dried over anhydrous magnesium sulfate, filtered, and concentrated under reduced pressure to obtain a residue. The obtained residue was subjected to MPLC (0-10% MeOH/DCM) to give 22.3 mg (63%) of a white solid.

### Step 3: Synthesis of (2S,4R)-N-(2-(3-(1-(2-((3-((5-(4-(aminomethyl)-4-methylpiperidin-1-yl)pyrazin-2-yl)thio)-2-chlorophenyl)amino)-2-oxoethyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl ((1-(5-((2-chloro-3-(2-(4-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)piperidin-1-yl)acetamido)phenyl)thio)pyrazin-2-yl)-4-methylpiperidin-4-yl)methyl)carbamate (22.3 mg, 0.02 mmol) was suspended in DCM (1.9 ml). 4 M HCl in dioxane (0.05 ml) was added and then stirred at room temperature for 1 hour. A saturated aqueous solution of NaHCOs (1 ml) was added, followed by extraction with 10% MeOH/DCM (2 ml × 2). The organic layer was washed with brine (1 ml × 2), dried over anhydrous magnesium sulfate, filtered, and concentrated under reduced pressure to obtain a residue. The obtained residue was subjected to MPLC (amine-silica, 0-5% MeOH/DCM) to obtain 16.2 mg (79%) of a white solid.

### Example 72. (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

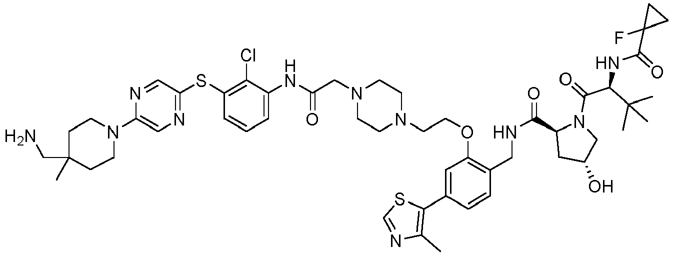

Example 72 was synthesized in the same way as in Example 71, using (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide instead of (2S,4R)-1-((S)-2-(1-fluorocyclopropane-1-carboxamido)-3,3-dimethylbutanoyl)-4-hydroxy-N-(4-(4-methylthiazol-5-yl)-2-(3-(piperidin-4-yl)propoxy)benzyl)pyrrolidine-2-carboxamide.

### Example 73. (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)piperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

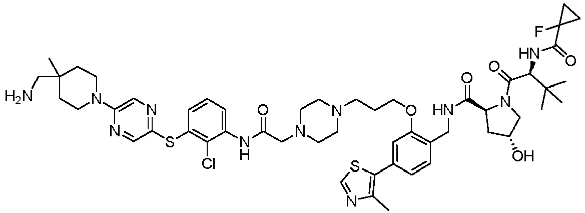

Example 73 was synthesized in the same way as in Example 71, using (2S,4R)-1-((S)-2-(1-fluorocyclopropane-1-carboxamido)-3,3-dimethylbutanoyl)-4-hydroxy-N-(4-(4-methylthiazol-5-yl)-2-(3-(piperazin-1-yl)propoxy)benzyl)pyrrolidine-2-carboxamide instead of (2S,4R)-1-((S)-2-(1-fluorocyclopropane-1-carboxamido)-3,3-dimethylbutanoyl)-4-hydroxy-N-(4-(4-methylthiazol-5-yl)-2-(3-(piperidin-4-yl)propoxy)benzyl)pyrrolidine-2-carboxamide.

### Example 74. (2S,4R)-N-(2-(2-(1-(3-((3-((5-(4-(aminomethyl)-4-methylpiperidin-1-yl)pyrazin-2-yl)thio)-2-chlorophenyl)amino)-3-oxopropyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

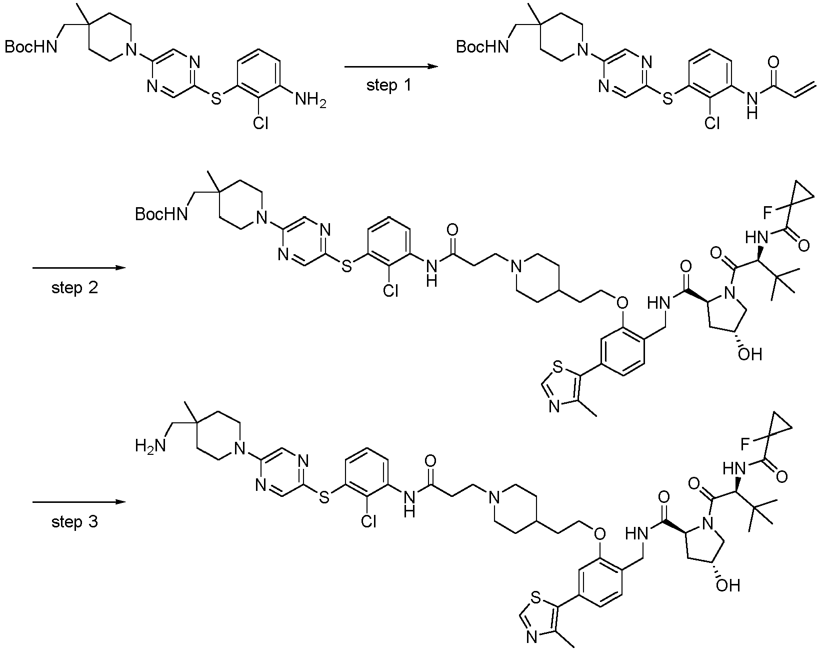

### Step 1: Synthesis of tert-butyl ((1-(5-((3-acrylamido-2-chlorophenyl)thio)pyrazin-2-yl)-4-methylpiperidin-4-yl)methyl)carbamate

Tert-butyl ((1-(5-((3-amino-2-chlorophenyl)thio)pyrazin-2-yl)-4-methylpiperidin-4-yl)methyl)carbamate (Intermediate 3, 50 mg, 0.11 mmol), and triethylamine (0.090 ml, 0.65 mmol) were suspended in DCM (2 ml). Then, 3-bromopropanoyl chloride (65 mg, 0.38 mmol) was slowly added at 0 °C and stirred for 1 hour. A saturated aqueous solution of NaHCOs (10 ml) was added to the reaction solution, followed by extraction with DCM (20 ml). The organic layer was washed with brine (10 ml), dried over anhydrous magnesium sulfate, filtered, and concentrated under reduced pressure to obtain a residue. The obtained residue was subjected to MPLC (5-40% EtOAc/Hexane) to give 36 mg (64%) of an ivory solid.

### Step 2: Synthesis of tert-butyl ((1-(5-((2-chloro-3-(3-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidin-1-yl)propanamido)phenyl)thio)pyrazin-2-yl)-4-methylpiperidin-4-yl)methyl)carbamate

Tert-butyl ((1-(5-((3-acrylamido-2-chlorophenyl)thio)pyrazin-2-yl)-4-methylpiperidin-4-yl)methyl)carbamate (18 mg, 0.03 mmol), (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperidin-4-yl)ethoxy)benzyl)pyrrolidine-2-carboxamide (25 mg, 0.04 mmol), and triethylamine (0.01 ml, 0.07 mmol) were suspended in DCM : MeOH = 1 : 1 (0.2 ml) and then stirred at 60 °C for 16 hours. Distilled water (3 ml) was added to the reaction solution, followed by extraction with DCM (5 ml), and the organic layer was dried over anhydrous magnesium sulfate, filtered, and concentrated under reduced pressure to obtain a residue. The obtained residue was subjected to MPLC (0-10% MeOH/DCM) to give 21.3 mg (53%) of an ivory solid.

### Step 3: Synthesis of (2S,4R)-N-(2-(2-(1-(3-((3-((5-(4-(aminomethyl)-4-methylpiperidin-1-yl)pyrazin-2-yl)thio)-2-chlorophenyl)amino)-3-oxopropyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl ((1-(5-((2-chloro-3-(3-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidin-1-yl)propanamido)phenyl)thio)pyrazin-2-yl)-4-methylpiperidin-4-yl)methyl)carbamate (21.3 mg, 0.02 mmol) was suspended in DCM (1.8 ml). Then, 4 M HCl in dioxane (0.05 ml) was added and then stirred at room temperature for 1 hour. A saturated aqueous solution of NaHCOs (1 ml) was added to the reaction solution, followed by extraction with DCM (5 ml × 3). The organic layer was washed with brine (1 ml × 2), dried over anhydrous magnesium sulfate, filtered and concentrated under reduced pressure to obtain a residue. The obtained residue was subjected to MPLC (amine-silica, 0-4% MeOH/DCM) to obtain 16.3 mg (84%) of a white solid.

### Example 75. (2S,4R)-N-(2-(3-(1-(3-((3-((5-(4-(aminomethyl)-4-methylpiperidin-1-yl)pyrazin-2-yl)thio)-2-chlorophenyl)amino)-3-oxopropyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

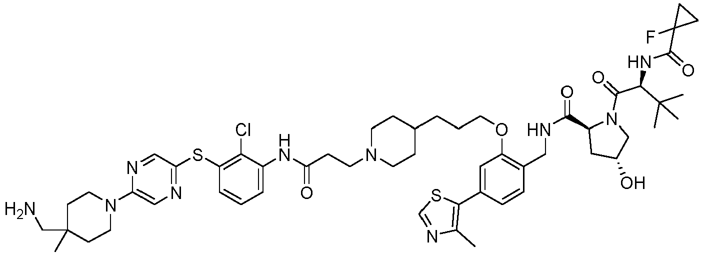

Example 75 was synthesized in the same way as in Example 74, using (2S,4R)-1-((S)-2-(1-fluorocyclopropane-1-carboxamido)-3,3-dimethylbutanoyl)-4-hydroxy-N-(4-(4-methylthiazol-5-yl)-2-(3-(piperidin-4-yl)propoxy)benzyl)pyrrolidine-2-carboxamide instead of (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperidin-4-yl)ethoxy)benzyl)pyrrolidine-2-carboxamide.

### Example 76. (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

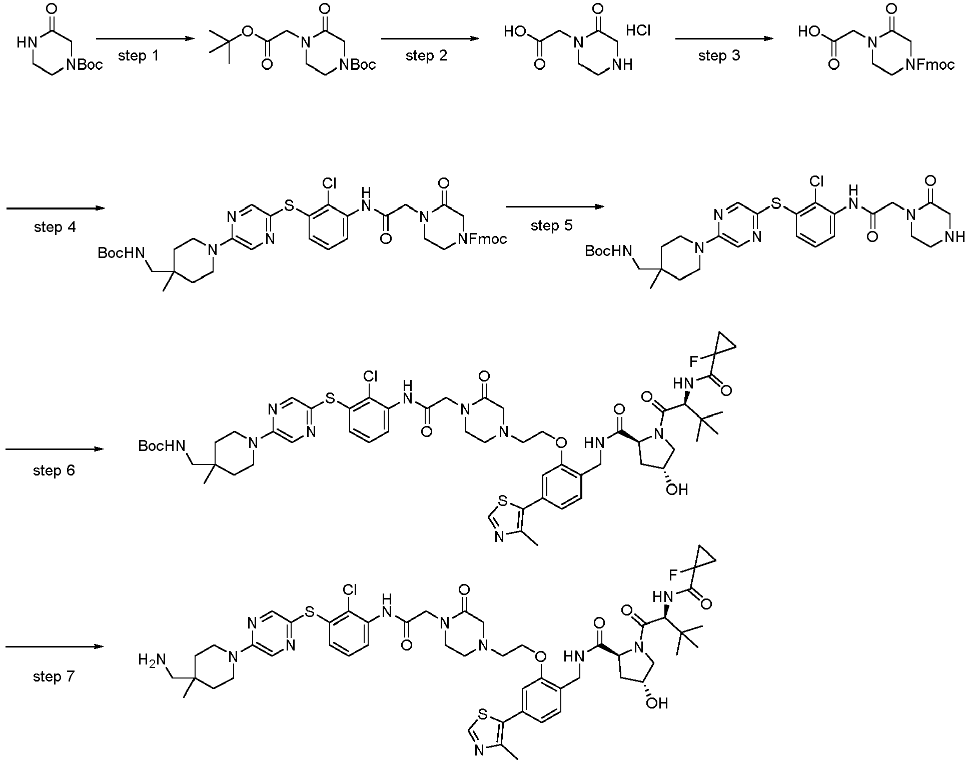

### Step 1: Synthesis of tert-butyl 4-(2-(tert-butoxy)-2-oxoethyl)-3-oxopiperazine-1-carboxylate

Tert-butyl 3-oxopiperazine-1-carboxylate (200 mg, 1.00 mmol) was suspended in DMF (1 ml). Then, sodium hydride (48 mg, 1.20 mmol) was slowly added at 0 °C and stirred for 10 minutes. Tert-butyl bromoacetate (0.16 ml, 1.10 mmol) was added to the reaction solution and stirred for 1 hour. Distilled water (10 ml) was added to the reaction solution, followed by extraction with EtOAc (15 ml). The organic layer was washed with brine (10 ml), dried over anhydrous sodium sulfate, filtered, and concentrated under reduced pressure to obtain a residue. The obtained residue was subjected to MPLC (3-50% EtOAc/Hexane) to give 286 mg (91%) of a colorless oil.

### Step 2: Synthesis of 2-(2-oxopiperazin-1-yl)acetic acid

Tert-butyl 4-(2-(tert-butoxy)-2-oxoethyl)-3-oxopiperazine-1-carboxylate (286 mg, 0.91 mmol) was suspended in DCM (3 ml). 4 M HCl in dioxane (4.5 ml) was added and then stirred at room temperature for 4 hours. The reaction solution was concentrated under reduced pressure to obtain 173 mg (98%) of a white solid.

### Step 3: Synthesis of 2-(4-(((9H-fluoren-9-yl)methoxy)carbonyl)-2-oxopiperazin-1-yl)acetic acid

2-(2-oxopiperazin-1-yl)acetic acid (173 mg, 0.89 mmol), and Na₂CO₃ (282 mg, 2.67 mmol) were suspended in water : 1,4-dioxane = 1 : 1 (7.4 ml) and then stirred at 0 °C for 10 minutes. After adding Fmoc-Cl (230 mg, 0.89 mmol), the mixture was stirred at room temperature for 16 hours. The reaction solution was adjusted to pH = 3 using 1N HCl aqueous solution, and then extracted with EtOAc (15 ml). The organic layer was dried over anhydrous sodium sulfate, filtered and concentrated under reduced pressure to obtain a residue. The obtained residue was subjected to MPLC (0-5% MeOH/DCM) to give 278 mg (82%) of a white solid.

### Step 4: Synthesis of (9H-fluoren-9-yl)methyl 4-((3-((5-(4-(((tert-butoxycarbonyl)amino)methyl)-4-methylpiperidin-1-yl)pyrazin-2-yl)thio)-2-chlorophenyl)carbamoyl)piperidine-1-carboxylate

2-(4-(((9H-fluoren-9-yl)methoxy)carbonyl)-2-oxopiperazin-1-yl)acetic acid (80 mg, 0.22 mmol), tert-butyl ((1-(5-((3-amino-2-chlorophenyl)thio)pyrazin-2-yl)-4-methylpiperidin-4-yl)methyl)carbamate (Intermediate 3, 85 mg, 0.17 mmol), TCFH (145 mg, 0.52 mmol), and NMI (0.068 ml, 0.86 mmol) were suspended in ACN : THF = 1 : 1 (1.2 ml) and then stirred at room temperature for 16 hours. Distilled water (10 ml) was added to the reaction solution, followed by extraction with EtOAc (15 ml). The organic layer was washed with brine (10 ml), dried over anhydrous sodium sulfate, filtered, and concentrated under reduced pressure to obtain a residue. The obtained residue was subjected to MPLC (3-70% EtOAc/Hexane) to give 156 mg (quant.) of an ivory solid.

### Step 5: Synthesis of tert-butyl ((1-(5-((2-chloro-3-(2-(2-oxopiperazin-1-yl)acetamido)phenyl)thio)pyrazin-2-yl)-4-methylpiperidin-4-yl)methyl)carbamate

((9H-fluoren-9-yl)methyl 4-((3-((5-(4-(((tert-butoxycarbonyl)amino)methyl)-4-methylpiperidin-1-yl)pyrazin-2-yl)thio)-2-chlorophenyl)carbamoyl)piperidine-1-carboxylate (156 mg, 0.17 mmol) was dissolved in DMF (0.8 ml). Then, piperidine (0.34 ml, 3.45 mmol) was slowly added and then stirred at room temperature for 2 hours. Distilled water (10 ml) was added to the reaction solution, followed by extraction with EtOAc (20 ml). The organic layer was washed with brine (10 ml × 2), dried over anhydrous sodium sulfate, filtered, and concentrated under reduced pressure to obtain a residue. The obtained residue was subjected to MPLC (amine-silica, 0-4 % MeOH/DCM) to obtain 64.5 mg (57%) of an ivory solid.

### Step 6: Synthesis of tert-butyl ((1-(5-((2-chloro-3-(2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)-2-oxopiperazin-1-yl)acetamido)phenyl)thio)pyrazin-2-yl)-4-methylpiperidin-4-yl)methyl)carbamate

Tert-butyl ((1-(5-((2-chloro-3-(2-(2-oxopiperazin-1-yl)acetamido)phenyl)thio)pyrazin-2-yl)-4-methylpiperidin-4-yl)methyl)carbamate (20 mg, 0.03 mmol), and (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10, 25 mg, 0.04 mmol) were suspended in DMF (0.01 ml). Then, potassium carbonate (14 mg, 0.10 mmol) and potassium iodide (11 mg, 0.07 mmol) were added and stirred at 70 °C for 16 hours. Distilled water (7 ml) was added to the reaction solution, followed by extraction with EtOAc (10 ml). The organic layer was washed with brine (7 ml × 2), dried over anhydrous sodium sulfate, filtered, and concentrated under reduced pressure to obtain a residue. The obtained residue was subjected to MPLC (0-5% MeOH/DCM) to give 27 mg (70%) of an ivory solid.

### Step 7: Synthesis of (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl ((1-(5-((2-chloro-3-(2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)-2-oxopiperazin-1-yl)acetamido)phenyl)thio)pyrazin-2-yl)-4-methylpiperidin-4-yl)methyl)carbamate (27 mg, 0.02 mmol) was suspended in DCM (2.3 ml). 4 M HCl in dioxane (0.06 ml) was added and stirred at room temperature for 1 hour. The reaction solution was neutralized by adding a saturated aqueous solution of NaHCOs (5 ml), followed by extraction with DCM (10 ml × 2). The organic layer was dried over anhydrous sodium sulfate, filtered and concentrated under reduced pressure to obtain a residue. The obtained residue was subjected to MPLC (amine-silica, 0-3% MeOH/DCM) to obtain 15 mg (61%) of an ivory solid.

### Example 77. (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

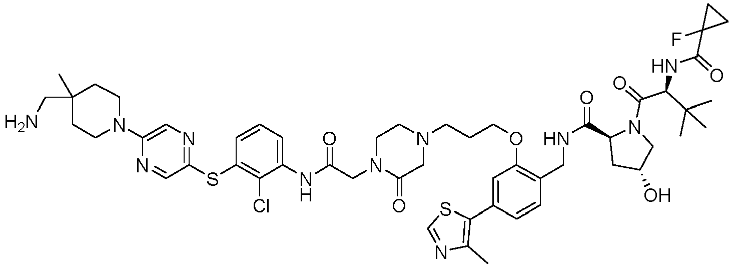

Example 77 was synthesized in the same way as in Example 76, using (2S,4R)-N-(2-(3-chloropropoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 11) instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10).

### Example 78. (2S,4R)-N-(2-(4-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

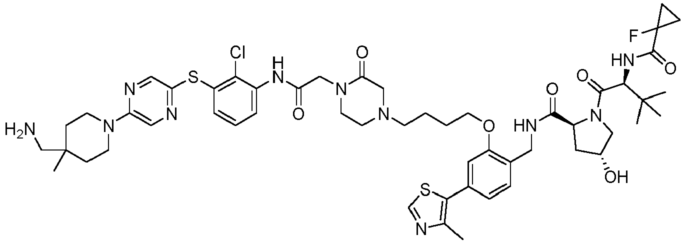

Example 78 was synthesized in the same way as in Example 76, using (2S,4R)-N-(2-(4-chlorobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 12) instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10).

### Example 79. (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(cyclopropanecarboxamido)-3,3-dimethylbutanoyl)-4-hydroxypyrrolidine-2-carboxamide

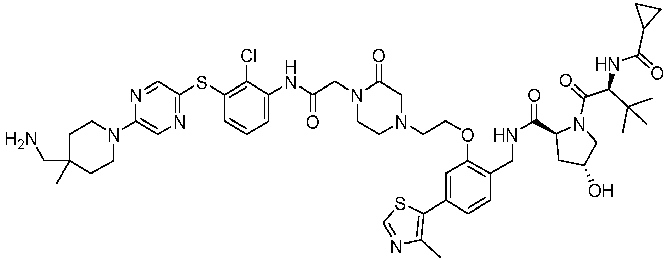

Example 79 was synthesized in the same way as in Example 76, using (2S,4R)-N-(2-(2-chloroethoxy)-4-(4-methylthiazol-5-yl)benzyl)-1-((S)-2-(cyclopropanecarboxamido)-3,3-dimethylbutanoyl)-4-hydroxypyrrolidine-2-carboxamide (Intermediate 15) instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10).

### Example 80. (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)propoxy)-4-(4-methylthiazol-5-yl)benzyl)-1-((S)-2-(cyclopropanecarboxamido)-3,3-dimethylbutanoyl)-4-hydroxypyrrolidine-2-carboxamide

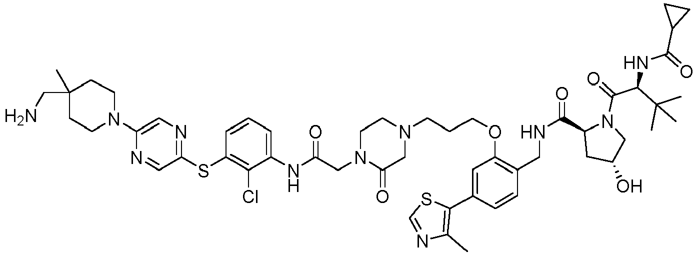

Example 80 was synthesized in the same way as in Example 76, using (2S,4R)-N-(2-(3-chloropropoxy)-4-(4-methylthiazol-5-yl)benzyl)-1-((S)-2-(cyclopropanecarboxamido)-3,3-dimethylbutanoyl)-4-hydroxypyrrolidine-2-carboxamide (Intermediate 16) instead of (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10).

### Example 81. N-(3-((5-(4-(aminomethyl)-4-methylpiperidin-1-yl)pyrazin-2-yl)thio)-2-chlorophenyl)-5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)picolinamide

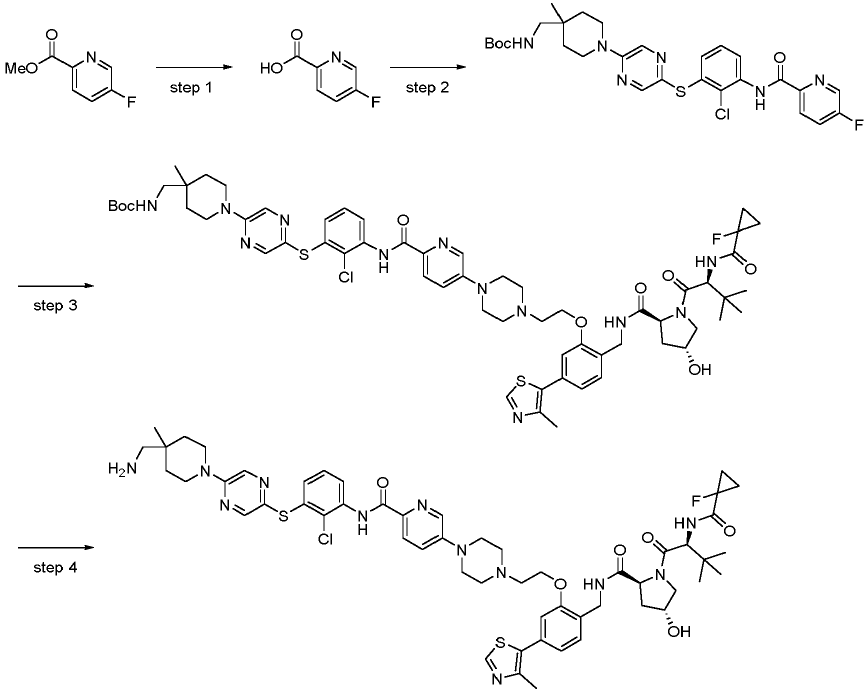

### Step 1: Synthesis of 5-fluoropicolinic acid

Methyl 5-fluoropicolinate (500 mg, 3.22 mmol) was suspended in THF (5 ml). Then, 2N NaOH aqueous solution (4.8 ml, 9.67 mmol) was added and stirred at room temperature for 1 hour. The reaction solution was adjusted to pH = 2 using 1N HCl aqueous solution, and then extracted with EtOAc (20 ml × 2). The organic layer was dried over anhydrous sodium sulfate, filtered, and concentrated under reduced pressure to obtain 320 mg (70%) of a white solid.

### Step2: Synthesis of tert-butyl ((1-(5-((2-chloro-3-(5-fluoropicolinamido)phenyl)thio)pyrazin-2-yl)-4-methylpiperidin-4-yl)methyl)carbamate

5-fluoropicolinic acid (30 mg, 0.214 mmol) was suspended in DCM (3 ml). Oxalyl chloride (0.05 ml, 0.56 mmol), and DMF (3 drops) were added thereto, followed by stirring at room temperature for 1 hour. The yellow oil obtained by concentrating the reaction solution was dissolved in DCM (1 ml) and then tert-butyl ((1-(5-((3-amino-2-chlorophenyl)thio)pyrazin-2-yl)-4-methylpiperidin- 4-yl)methyl)carbamate (Intermediate 3, 50 mg, 0.11 mmol), and DIPEA (0.9 ml, 0.53 mmol) were added and stirred at room temperature for 1 hour. Distilled water (10 ml) was added to the reaction solution, extracted with DCM (10 ml × 2), the organic layer was dried over anhydrous sodium sulfate, filtered, and concentrated under reduced pressure. The residue obtained by MPLC (0-100% EtOAc/Hexane) was performed as a yellow solid 33 mg (53%) was obtained.

### Step 3: Synthesis of tert-butyl ((1-(5-((2-chloro-3-(5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)picolinamido)phenyl)thio)pyrazin-2-yl)-4-methylpiperidin-4-yl)methyl)carbamate

Tert-butyl ((1-(5-((2-chloro-3-(5-fluoropicolinamido)phenyl)thio)pyrazin-2-yl)-4-methylpiperidin-4-yl)methyl)carbamate (32 mg, 0.06 mmol), and (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide (39 mg, 0.06 mmol) were suspended in DMSO (0.2 ml). DIPEA (0.03 ml, 0.17 mmol) was added and stirred at 75 °C for 16 hours. Distilled water (10 ml) was added to the reaction solution, followed by extraction with EtOAc (10 ml × 2). The organic layer was washed with brine (10 ml × 2), dried over anhydrous magnesium sulfate, filtered, and concentrated under reduced pressure to obtain a residue. The obtained residue was subjected to MPLC (0-5% MeOH/DCM) to give 36 mg (54%) of an ivory solid.

### Step 4: Synthesis of N-(3-((5-(4-(aminomethyl)-4-methylpiperidin-1-yl)pyrazin-2-yl)thio)-2-chlorophenyl)-5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)picolinamide

Tert-butyl ((1-(5-((2-chloro-3-(5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)picolinamido)phenyl)thio)pyrazin-2-yl)-4-methylpiperidin-4-yl)methyl)carbamate (33 mg, 0.03 mmol) was suspended in DCM (2 ml). Then, 4 M HCl in dioxane (0.3 ml) was added and stirred at room temperature for 1 hour. A saturated aqueous solution of NaHCOs (10 ml) was added to the reaction solution, followed by extraction with 10% MeOH/DCM (10 ml × 2). The organic layer was washed with brine (10 ml × 2), dried over anhydrous magnesium sulfate, filtered, and concentrated under reduced pressure to obtain a residue. The obtained residue was subjected to MPLC (amine-silica, 0-5% MeOH/DCM) to obtain 23 mg (77%) of an ivory solid.

### Example 82. N-(3-((5-(4-(aminomethyl)-4-methylpiperidin-1-yl)pyrazin-2-yl)thio)-2-chlorophenyl)-6-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)nicotinamide

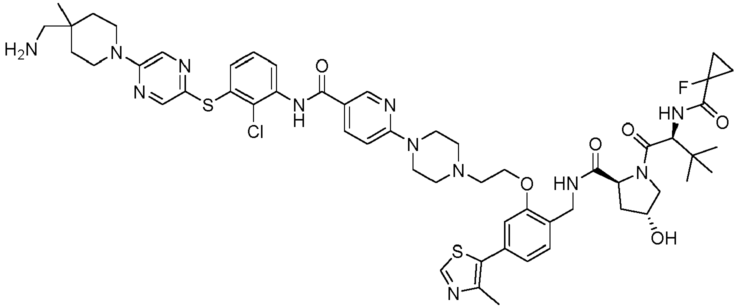

Example 82 was synthesized in the same way as in Example 81, using methyl 6-fluoropicolinate instead of methyl 5-fluoropicolinate.

### Example 83. N-(3-((5-(4-(aminomethyl)-4-methylpiperidin-1-yl)pyrazin-2-yl)thio)-2-chlorophenyl)-5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-2-carboxamide

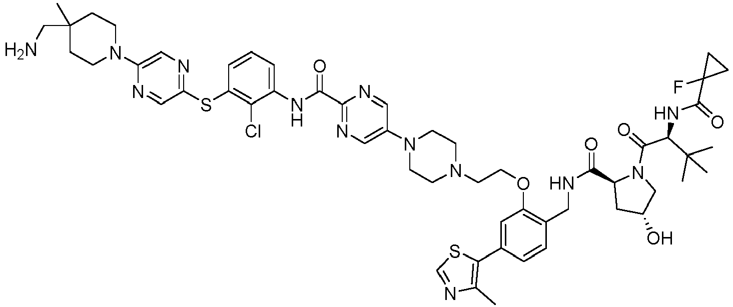

Example 84 was synthesized in the same way as in Example 81, using 5-chloropyrimidine-2-carboxylic acid instead of 5-fluoropicolinic acid.

### Example 84. N-(3-((5-(4-(aminomethyl)-4-methylpiperidin-1-yl)pyrazin-2-yl)thio)-2-chlorophenyl)-2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-5-carboxamide

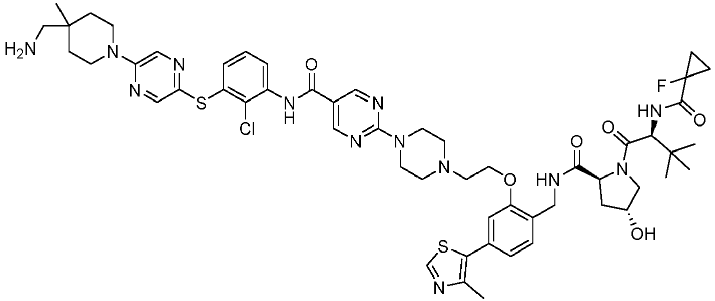

Example 84 was synthesized in the same way as in Example 81, using 2-chloropyrimidine-5-carboxylic acid instead of 5-fluoropicolinic acid.

### Example 85. N-(3-((5-(4-(aminomethyl)-4-methylpiperidin-1-yl)pyrazin-2-yl)thio)-2-chlorophenyl)-6-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyridazine-3-carboxamide

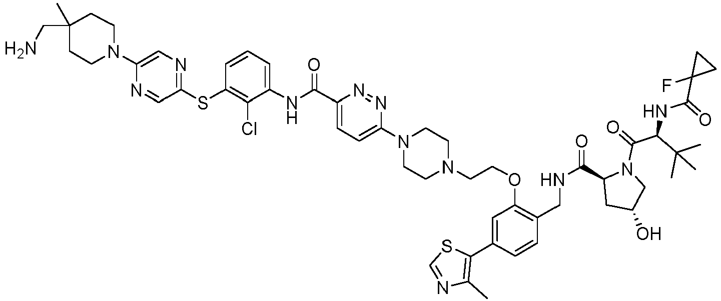

Example 85 was synthesized in the same way as in Example 81, using methyl 6-chloropyridazine-3-carboxylate instead of methyl 5-fluoropicolinate.

### Example 86. N-(3-((5-(4-(aminomethyl)-4-methylpiperidin-1-yl)pyrazin-2-yl)thio)-2-chlorophenyl)-2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-4-carboxamide

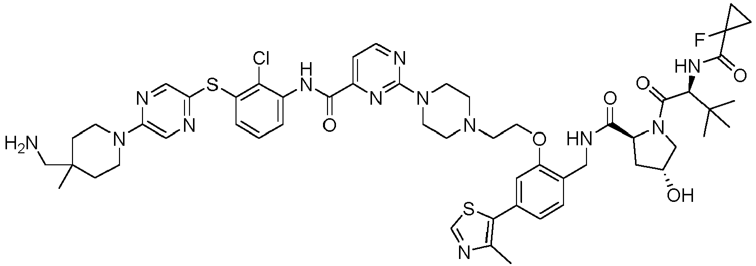

Example 86 was synthesized in the same way as in Example 81, using 2-chloropyrimidine-4-carboxylic acid instead of 5-fluoropicolinic acid.

### Example 87. N-(3-((5-(4-(aminomethyl)-4-methylpiperidin-1-yl)pyrazin-2-yl)thio)-2-chlorophenyl)-5-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)-7-azaspiro[3.5]nonan-7-yl)pyrimidine-2-carboxamide

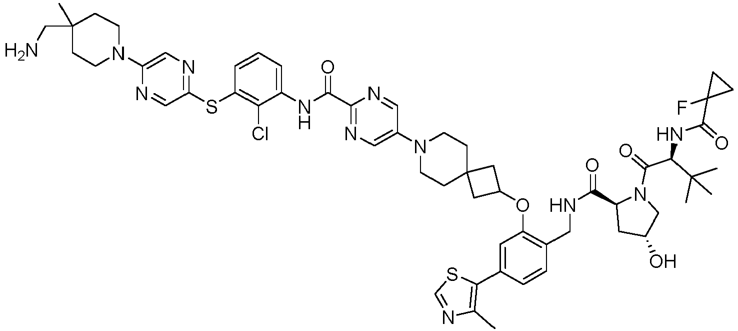

Example 87 was synthesized in the same way as in Example 81, using 5-chloropyrimidine-2-carboxylic acid and (2S,4R)-N-(2-(7-azaspiro[3.5]nonan-2-yloxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide instead of 5-fluoropicolinic acid and (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide, respectively.

### Example 88. N-(3-((5-(4-(aminomethyl)-4-methylpiperidin-1-yl)pyrazin-2-yl)thio)-2-chlorophenyl)-6-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)-7-azaspiro[3.5]nonan-7-yl)pyridazine-3-carboxamide

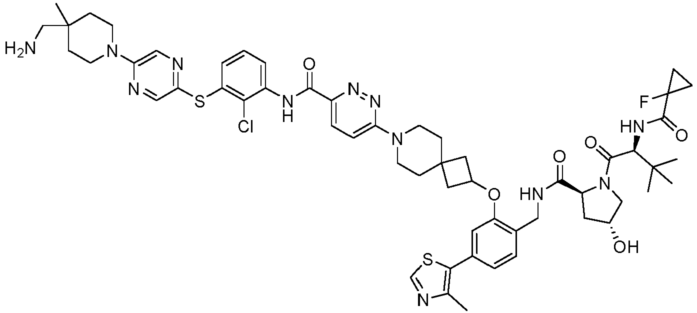

Example 88 was synthesized in the same way as in Example 81, using methyl 6-chloropyridazine-3-carboxylate and (2S,4R)-N-(2-((7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide instead of methyl 5-fluoropicolinate and (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide, respectively.

### Example 89. 2-(4-(4-((3-((5-(4-(aminomethyl)-4-methylpiperidin-1-yl)pyrazin-2-yl)thio)-2-chlorophenyl)amino)-4-oxobutyl)piperidin-1-yl)-N-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)pyrimidine-5-carboxamide

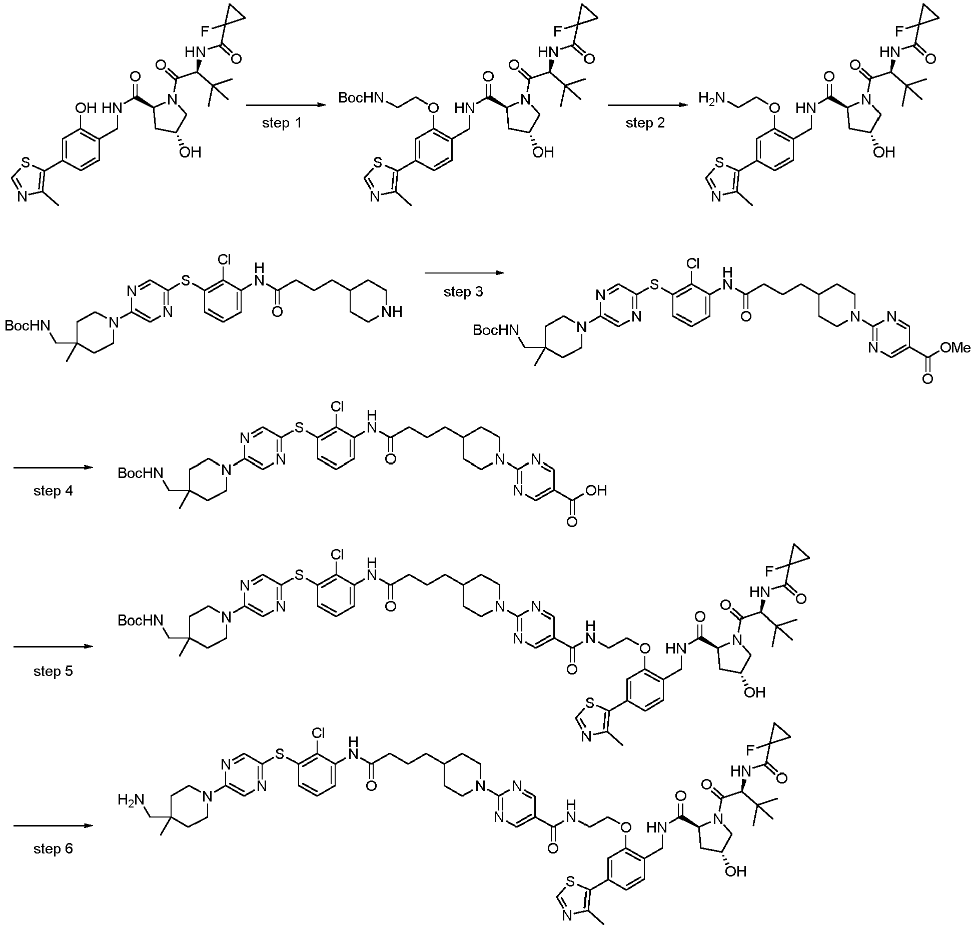

### Step 1: Synthesis of tert-butyl (2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)carbamate

(2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 7, 50 mg, 0.09 mmol), and tert-butyl (2-bromoethyl)carbamate (122 mg, 0.56 mmol) were suspended in DMF (0.2 ml). Potassium carbonate (39 mg, 0.28 mmol) was added and then stirred at 70 °C for 16 hours. Distilled water (10 ml) was added to the reaction solution, followed by extraction with EtOAc (5 ml × 2). The organic layer was washed with brine (5 ml × 2), dried over anhydrous magnesium sulfate, filtered, and concentrated under reduced pressure to obtain a residue. The obtained residue was subjected to MPLC (0-20% MeOH/DCM) to give 73 mg (quant.) of an ivory solid.

### Step 2: Synthesis of (2S,4R)-N-(2-(2-aminoethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl (2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)carbamate (73 mg, 0.11 mmol) was suspended in DCM (3 ml). Then, 4 M HCl in dioxane (1 ml) was added and then stirred at room temperature for 4 hours. A saturated aqueous solution of NaHCOs (5 ml) was added to the reaction solution, followed by extraction with 10% MeOH/DCM (5 ml × 2). The organic layer was washed with brine (5 ml × 2), dried over anhydrous magnesium sulfate, filtered, and concentrated under reduced pressure to obtain a residue. The obtained residue was subjected to MPLC (amine-silica, 0-5% MeOH/DCM) to obtain 22 mg (35%) of a white solid.

### Step 3: Synthesis of methyl 2-(4-(4-((3-((5-(4-(((tert-butoxycarbonyl)amino)methyl)-4-methylpiperidin-1-yl)pyrazin-2-yl)thio)-2-chlorophenyl)amino)-4-oxobutyl)piperidin-1-yl)pyrimidine-5-carboxylate

Tert-butyl ((1-(5-((2-chloro-3-(3-(piperidin-4-yl)butanamido)phenyl)thio)pyrazin-2-yl)-4-methylpiperidin-4-yl)methyl)carbamate (100 mg, 0.16 mmol), methyl 2-chloropyrimidine-5-carboxylate (28 mg, 0.16 mmol), and DIPEA (0.08 ml, 0.48 mmol) were suspended in DCM (0.6 ml) and then stirred at room temperature for 16 hours. Distilled water (10 ml) was added to the reaction solution, followed by extraction with EtOAc (5 ml × 2). The organic layer was washed with brine (5 ml × 2), dried over anhydrous magnesium sulfate, filtered, and concentrated under reduced pressure to obtain a residue. The obtained residue was subjected to MPLC (0-100% EtOAc/Hexane) to give 111 mg (91%) of an ivory solid.

### Step 4: Synthesis of 2-(4-(4-((3-((5-(4-(((tert-butoxycarbonyl)amino)methyl)-4-methylpiperidin-1-yl)pyrazin-2-yl)thio)-2-chlorophenyl)amino)-4-oxobutyl)piperidin-1-yl)pyrimidine-5-carboxylic acid

Methyl 2-(4-(4-((3-((5-(4-(((tert-butoxycarbonyl)amino)methyl)-4-methylpiperidin-1-yl)pyrazin-2-yl)thio)-2-chlorophenyl)amino)-4-oxobutyl)piperidin-1-yl)pyrimidine-5-carboxylate (110 mg, 0.13 mmol), and lithium hydroxide (20 mg, 0.53 mmol) were suspended in water : THF = 1 : 1 (0.8 ml) and then stirred at 60 °C for 5 hours. The reaction solution was adjusted to pH = 2 using 1N HCl aqueous solution, and extracted with EtOAc (10 ml × 2). The organic layer was dried over anhydrous sodium sulfate, filtered, and concentrated under reduced pressure to obtain 92 mg (85%) of an ivory solid.

### Step 5: Synthesis of tert-butyl ((1-(5-((2-chloro-3-(4-(1-(5-((2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)carbamoyl)pyrimidin-2-yl)piperidin-4-yl)butanamido)phenyl)thio)pyrazin-2-yl)-4-methylpiperidin-4-yl)methyl)carbamate

2-(4-(4-((3-((5-(4-(((tert-butoxycarbonyl)amino)methyl)-4-methylpiperidin-1-yl)pyrazin-2-yl)thio)-2-chlorophenyl)amino)-4-oxobutyl)piperidin-1-yl)pyrimidine-5-carboxylic acid (31 mg 0.04 mmol), (2S,4R)-N-(2-(2-aminoethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (20 mg, 0.03 mmol), EDCI (8 mg, 0.04 mmol), HOBt (6 mg, 0.04 mmol), and DIPEA (0.03 ml, 0.17 mmol) were suspended in DMF (0.1 ml) and then stirred at room temperature for 16 hours. Distilled water (10 ml) was added to the reaction solution, followed by extraction with EtOAc (5 ml × 2). The organic layer was washed with brine (5 ml × 2), dried over anhydrous sodium sulfate, filtered, and concentrated under reduced pressure to obtain a residue. The obtained residue was subjected to MPLC (10% MeOH/DCM) to give 36 mg (68%) of an ivory solid.

### Step 6: Synthesis of 2-(4-(4-((3-((5-(4-(aminomethyl)-4-methylpiperidin-1-yl)pyrazin-2-yl)thio)-2-chlorophenyl)amino)-4-oxobutyl)piperidin-1-yl)-N-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)pyrimidine-5-carboxamide

Tert-butyl ((1-(5-((2-chloro-3-(4-(1-(5-((2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)carbamoyl)pyrimidin-2-yl)piperidin-4-yl)butanamido)phenyl)thio)pyrazin-2-yl)-4-methylpiperidin-4-yl)methyl)carbamate (34.4 mg, 0.03 mmol) was suspended in DCM (1 ml). Then, 4 M HCl in dioxane (0.3 ml) was added and stirred at room temperature for 4 hours. A saturated aqueous solution of NaHCOs (5 ml) was added to the reaction solution, followed by extraction with 10% MeOH/DCM (5 ml × 2). The organic layer was washed with brine (5 ml × 2), dried over anhydrous magnesium sulfate, filtered, and concentrated under reduced pressure to obtain a residue. The obtained residue was subjected to MPLC (amine-silica, 0-5% MeOH/DCM) to obtain 12 mg (39%) of an ivory solid.

### Example 90. (2S,4R)-N-(2-(2-((2-(4-(4-((3-((5-(4-(aminomethyl)-4-methylpiperidin-1-yl)pyrazin-2-yl)thio)-2-chlorophenyl)amino)-4-oxobutyl)piperidin-1-yl)pyrimidin-5-yl)oxy)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

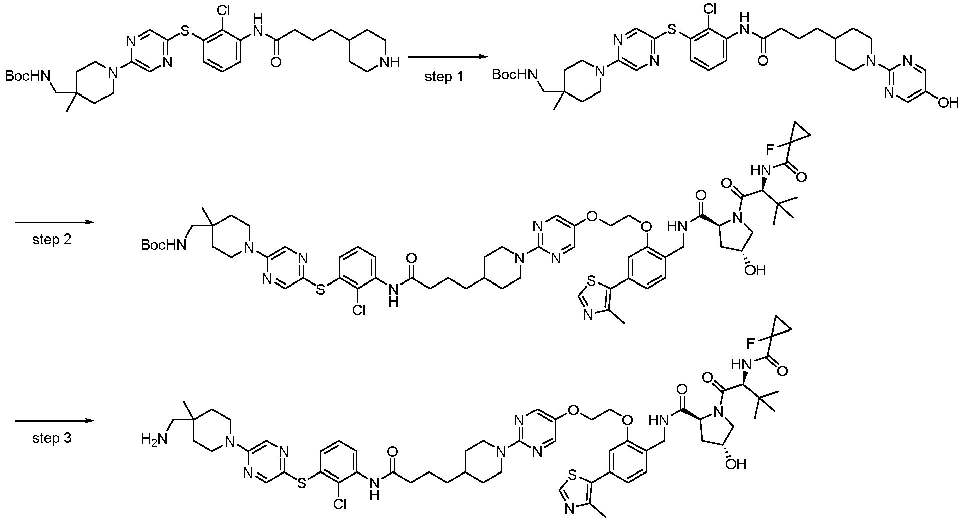

### Step 1: Synthesis of tert-butyl ((1-(5-((2-chloro-3-(4-(1-(5-hydroxypyrimidin-2-yl)piperidin-4-yl)butanamido)phenyl)thio)pyrazin-2-yl)-4-methylpiperidin-4-yl)methyl)carbamate

Tert-butyl ((1-(5-((2-chloro-3-(3-(piperidin-4-yl)butanamido)phenyl)thio)pyrazin-2-yl)-4-methylpiperidin-4-yl)methyl)carbamate (65 mg, 0.11 mmol), and 2-chloro-5-hydroxypyrimidine (21 mg, 0.16 mmol) were suspended in DMF (0.3 ml). Potassium carbonate (34 mg, 0.24 mmol) was added and stirred in a microwave at 180 °C for 16 hours. Distilled water (10 ml) was added to the reaction solution, followed by extraction with EtOAc (5 ml × 2). The organic layer was washed with brine (5 ml × 2), dried over anhydrous magnesium sulfate, filtered, and concentrated under reduced pressure to obtain a residue. The obtained residue was subjected to MPLC (0-100% EtOAc/Hexane) to give 20 mg (27%) of an ivory solid.

### Step 2: Synthesis of tert-butyl ((1-(5-((2-chloro-3-(4-(1-(5-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethoxy)pyrimidin-2-yl)piperidin-4-yl)butanamido)phenyl)thio)pyrazin-2-yl)-4-methylpiperidin-4-yl)methyl)carbamate

Tert-butyl ((1-(5-((2-chloro-3-(4-(1-(5-hydroxypyrimidin-2-yl)piperidin-4-yl)butanamido)phenyl)thio)pyrazin-2-yl)-4-methylpiperidin-4-yl)methyl)carbamate (20 mg, 0.03 mmol), and (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 10, 20 mg, 0.03 mmol) were suspended in DMF (0.05 ml). Potassium carbonate (12 mg, 0.08 mmol), and potassium iodide (9 mg, 0.06 mmol) were added and then stirred at 70 °C for 16 hours. Distilled water (10 ml) was added to the reaction solution, followed by extraction with EtOAc (5 ml × 2). The organic layer was washed with brine (5 ml × 2), dried over anhydrous magnesium sulfate, filtered, and concentrated under reduced pressure to obtain a residue. The obtained residue was subjected to MPLC (0-100% EtOAc/Hexane) to give 35 mg (98%) of an ivory solid.

### Step 3: Synthesis of (2S,4R)-N-(2-(2-((2-(4-(4-((3-((5-(4-(aminomethyl)-4-methylpiperidin-1-yl)pyrazin-2-yl)thio)-2-chlorophenyl)amino)-4-oxobutyl)piperidin-1-yl)pyrimidin-5-yl)oxy)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

Tert-butyl ((1-(5-((2-chloro-3-(4-(1-(5-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethoxy)pyrimidin-2-yl)piperidin-4-yl)butanamido)phenyl)thio)pyrazin-2-yl)-4-methylpiperidin-4-yl)methyl)carbamate (33 mg, 0.03 mmol) was suspended in DCM (1 ml). Then, 4 M HCl in dioxane (0.3 ml) was added and stirred at room temperature for 4 hours. A saturated aqueous solution of NaHCOs (5 ml) was added to the reaction solution, followed by extraction with 10% MeOH/DCM (5 ml × 2). The organic layer was washed with brine (5 ml × 2), dried over anhydrous magnesium sulfate, filtered, and concentrated under reduced pressure to obtain a residue. The obtained residue was subjected to PTLC (DCM : MeOH : NH₄OH = 15 : 1 : 0.1) to obtain 7.3 mg (24%) of an ivory solid.

The NMR and mass results of the compounds obtained in Example 1-90 are summarized in Table 3 below.

**[Table 3]**

| Exam ple (Com pound ) | NMR and m/z |
|---|---|
| 1 | ¹H NMR (600 MHz, CDCl₃) δ 8.68 (s, 1H), 8.24 (t, J = 7.3 Hz, 1H), 8.19 (dd, J = 4.2, 1.4 Hz, 1H), 8.14 (dd, J = 3.7, 1.4 Hz, 1H), 7.80 (s, 1H), 7.33 (d, J = 7.7 Hz, 1H), 7.31-7.28 (m, 1H), 7.12 (td, J = 8.1, 5.3 Hz, 1H), 7.01-6.97 (m, 1H), 6.96 (dd, J = 7.7, 1.5 Hz, 1H), 6.89 (d, J = 1.4 Hz, 1H), 6.76 (ddd, J = 19.3, 8.0, 1.4 Hz, 1H), 4.75 (t, J = 7.6 Hz, 1H), 4.57-4.37 (m, 4H), 4.14-4.05 (m, 2H), 4.01 (d, J = 10.4 Hz, 1H), 3.95-3.87 (m, 2H), 3.61 (dd, J = 11.2, 3.8 Hz, 1H), 3.39 (tdd, J = 13.7, 10.2, 3.5 Hz, 2H), 3.07 (m, 2H), 2.65-2.56 (m, 5H), 2.53 (s, 3H), 2.34 (m, 1H), 2.13-2.04 (m, 5H), 1.95-1.85 (m, 2H), 1.75-1.67 (m, 2H), 1.54-1.41 (m, 4H), 1.35-1.25 (m, 4H), 1.03 (s, 3H), 0.92 (s, 9H). m/z 1047.55 [M+H]⁺ |
| 2 | ¹H NMR (600 MHz, CDCl₃) δ 8.67 (s, 1H), 8.23 (d, J = 8.1 Hz, 1H), 8.18 (d, J = 1.4 Hz, 1H), 8.14 (d, J = 1.3 Hz, 1H), 7.80 (s, 1H), 7.32 (d, J = 7.7 Hz, 1H), 7.20 (t, J = 5.8 Hz, 1H), 7.12 (t, J = 8.1 Hz, 1H), 7.00 (dd, J = 8.5, 3.2 Hz, 1H), 6.95 (dd, J = 7.6, 1.5 Hz, 1H), 6.87 (d, J = 1.4 Hz, 1H), 6.77 (dd, J = 8.0, 1.4 Hz, 1H), 4.74 (t, J = 7.6 Hz, 1H), 4.59-4.38 (m, 4H), 4.10-4.02 (m, 2H), 3.99 (d, J = 11.3 Hz, 1H), 3.91 (dt, J = 13.4, 4.8 Hz, 2H), 3.63 (dd, J = 11.2, 4.0 Hz, 1H), 3.38 (ddd, J = 13.5, 10.1, 3.5 Hz, 2H), 3.03 (m, 2H), 2.62-2.54 (m, 3H), 2.53 (s, 3H), 2.44 (t, J = 7.3 Hz, 2H), 2.32 (t, J = 11.5 Hz, 1H), 2.10-1.97 (m, 5H), 1.92-1.86 (m, 4H), 1.75-1.72 (m, 2H), 1.61-1.41 (m, 4H), 1.37-1.23 (m, 4H), 1.03 (s, 3H), 0.93 (s, 9H). m/z 1061.56 [M+H]⁺ |
| 3 | ¹H NMR(600 MHz, CDCl₃) δ 8.68 (s, 1H), 8.27-8.20 (m, 1H), 8.18 (d, J = 1.4 Hz, 1H), 8.14 (d, J = 1.4 Hz, 1H), 7.80 (s, 1H), 7.32 (d, J = 7.7 Hz, 1H), 7.18-7.08 (m, 2H), 7.01 (dd, J = 8.6, 3.3 Hz, 1H), 6.95 (dd, J = 7.6, 1.6 Hz, 1H), 6.86 (d, J = 1.5 Hz, 1H), 6.77 (dd, J = 8.0, 1.4 Hz, 1H), 4.70 (t, J = 7.5 Hz, 1H), 4.59-4.38 (m, 4H), 4.09-3.98 (m, 2H), 3.96-3.89 (m, 3H), 3.64 (dd, J = 11.0, 4.1 Hz, 1H), 3.42-3.35 (m, 2H), 3.02 (t, J = 10.3 Hz, 2H), 2.60-2.54 (m, 3H), 2.53 (s, 3H), 2.40-2.29 (m, 3H), 2.09-1.95 (m, 5H), 1.93-1.80 (m, 4H), 1.73-1.38 (m, 10H), 1.37-1.22 (m, 4H), 1.03 (s, 3H), 0.93 (s, 9H). m/z 1089.48 [M+H]⁺ |
| 4 | ¹H NMR (600 MHz, CDCl₃) δ 8.68 (s, 1H), 8.24 (s, 1H), 8.19 (s, 1H), 8.15 (s, 1H), 7.80 (s, 1H), 7.32 (d, J = 7.7 Hz, 1H), 7.21 (m, 1H), 7.12 (td, J = 8.0, 4.3 Hz, 1H), 7.01 (d, J = 8.3 Hz, 1H), 6.95 (d, J = 7.6 Hz, 1H), 6.87 (s, 1H), 6.76 (dd, J = 14.6, 8.0 Hz, 1H), 4.72 (t, J = 7.5 Hz, 1H), 4.55-4.40 (m, 4H), 4.05-3.86 (m, 5H), 3.63 (dd, J = 11.1, 3.6 Hz, 1H), 3.40 (dt, J = 23.9, 6.8 Hz, 2H), 3.02-3.01 (m, 2H), 2.61-2.54 (m, 3H), 2.53 (s, 3H), 2.34-2.32 (m, 3H), 2.10-1.92 (m, 5H), 1.92-1.77 (m, 4H), 1.77-1.43 (m, 14H), 1.43-1.19 (m, 4H), 1.03 (s, 3H), 0.93 (s, 9H). m/z 1117.56 [M+H]⁺ |
| 5 | ¹H NMR (600 MHz, CDCl₃) δ 8.68 (s, 1H), 8.19-8.14 (m, 3H), 7.79 (s, 1H), 7.37-7.35 (m, 1H), 7.33 (d, J = 7.7 Hz, 1H), 7.12 (td, J = 8.1, 4.3 Hz, 1H), 7.01 (dd, J = 8.3, 3.0 Hz, 1H), 6.96 (dd, J = 7.6, 1.5 Hz, 1H), 6.89 (d, J = 1.4 Hz, 1H), 6.76 (dd, J = 8.0, 1.4 Hz, 1H), 4.70 (t, J = 7.8 Hz, 1H), 4.53-4.43 (m, 4H), 4.20-4.10 (m, 2H), 4.01 (d, J = 11.3 Hz, 1H), 3.90 (dd, J = 13.3, 5.0 Hz, 2H), 3.64 (dd, J = 11.2, 3.6 Hz, 1H), 3.39 (tdd, J = 13.6, 10.2, 3.5 Hz, 2H), 3.05-3.03 (m, 2H), 2.91-2.81 (m, 2H), 2.58 (s, 2H), 2.53 (s, 3H), 2.48-2.44 (m, 1H), 2.39-2.28 (m, 2H), 2.21-2.13 (m, 2H), 2.12-2.06 (m, 1H), 2.05-1.99 (m, 1H), 1.96-1.87 (m, 2H), 1.87-1.75 (m, 2H), 1.73-1.37 (m, 4H), 1.36-1.19 (m, 4H), 1.03 (s, 3H), 0.94 (s, 9H). m/z 1047.58 [M+H]⁺ |
| 6 | ¹H NMR (600 MHz, CDCl₃) δ 8.68 (s, 1H), 8.24-8.10 (m, 3H), 7.68 (s, 1H), 7.33-7.27 (m, 2H), 7.13 (t, J = 8.1 Hz, 1H), 7.00 (dd, J = 8.5, 3.1 Hz, 1H), 6.95 (d, J = 7.7 Hz, 1H), 6.89 (s, 1H), 6.77 (d, J = 8.0 Hz, 1H), 4.74 (t, J = 7.6 Hz, 1H), 4.59-4.35 (m, 4H), 4.12-4.03 (m, 2H), 4.00 (d, J = 11.3 Hz, 1H), 3.91 (dt, J = 13.3, 4.7 Hz, 2H), 3.61 (dd, J = 11.2, 3.9 Hz, 1H), 3.38 (ddd, J = 13.5, 10.1, 3.5 Hz, 2H), 2.96 (m, 2H), 2.64-2.47 (m, 8H), 2.34 (d, J = 7.1 Hz, 2H), 2.09-2.00 (m, 5H), 1.95-1.90 (m, 1H), 1.84-1.73 (m, 2H), 1.57-1.21 (m, 10H), 1.03 (s, 3H), 0.92 (s, 9H). m/z 1061.69 [M+H]⁺ |
| 7 | ¹H NMR (600 MHz, CDCl₃) δ 8.68 (s, 1H), 8.24-8.12 (m, 3H), 7.69 (s, 1H), 7.32 (d, J = 7.7 Hz, 1H), 7.19-7.09 (m, 2H), 7.02 (dd, J = 8.7, 3.2 Hz, 1H), 6.95 (dd, J = 7.7, 1.6 Hz, 1H), 6.87 (d, J = 1.5 Hz, 1H), 6.77-6.73 (m, 1H), 4.72 (t, J = 7.6 Hz, 1H), 4.57-4.38 (m, 4H), 4.08-4.00 (m, 2H), 3.96 (d, J = 11.2 Hz, 1H), 3.94-3.87 (m, 2H), 3.63 (dd, J = 11.1, 4.1 Hz, 1H), 3.40 (tdd, J = 13.6, 10.1, 3.5 Hz, 2H), 2.95-2.94 (m, 2H), 2.61-2.49 (m, 6H), 2.42 (t, J = 7.4 Hz, 2H), 2.33 (d, J = 7.1 Hz, 2H), 2.10-2.03 (m, 1H), 2.03-1.95 (m, 2H), 1.93-1.43 (m, 13H), 1.43-1.24 (m, 4H), 1.03 (s, 3H), 0.93 (s, 9H). m/z 1075.65 [M+H]⁺ |
| 8 | ¹H NMR (600 MHz, CDCl₃) δ 8.68 (s, 1H), 8.27-8.11 (m, 3H), 7.69 (s, 1H), 7.32 (d, J = 7.7 Hz, 1H), 7.13 (t, J = 8.1 Hz, 1H), 7.00 (dd, J = 8.5, 3.2 Hz, 1H), 6.95 (dd, J = 7.7, 1.4 Hz, 1H), 6.89 (d, J = 1.4 Hz, 1H), 6.78 (dd, J = 8.0, 1.3 Hz, 1H), 4.73 (t, J = 7.6 Hz, 1H), 4.54 (s, 1H), 4.53-4.47 (m, 2H), 4.41 (dd, J = 14.7, 5.2 Hz, 1H), 4.05-4.09 (m, 2H), 3.99 (d, J = 11.3 Hz, 1H), 3.79-3.72 (m, 3H), 3.70-3.58 (m, 4H), 2.97 (s, 2H), 2.54-2.59 (m, 2H), 2.53 (s, 3H), 2.34 (d, J = 7.1 Hz, 2H), 2.11-1.98 (m, 5H), 1.98-1.88 (m, 2H), 1.80 (d, J = 11.8 Hz, 2H), 1.62-1.67 (m, 3H), 1.57-1.51 (m, 2H), 1.47 (t, J = 7.3 Hz, 1H), 1.36-1.39 (m, 2H), 1.36-1.24 (m, 6H), 1.22 (s, 3H), 0.92 (s, 9H). m/z 1047.49 [M+H]⁺ |
| 9 | ¹H NMR (600 MHz, CDCl₃) δ 8.68 (s, 1H), 8.13 (s, 1H), 7.66 (s, 1H), 7.63 (s, 1H), 7.33-7.31 (m, 2H), 7.09 (t, J = 8.1 Hz, 1H), 6.99 (d, J = 5.1 Hz, 1H), 6.95 (d, J = 7.6 Hz, 1H), 6.89 (s, 1H), 6.57 (d, J = 8.0 Hz, 1H), 4.82 (s, 2H), 4.74 (t, J = 7.6 Hz, 1H), 4.60-4.36 (m, 4H), 4.09-4.06 (m, 2H), 4.00 (d, J = 11.5 Hz, 1H), 3.74-3.68 (m, 2H), 3.64-3.57 (m, 3H), 2.97 (m, 2H), 2.62-2.49 (m, 6H), 2.35 (d, J = 6.8 Hz, 2H), 2.10-1.98 (m, 5H), 1.95 (m, 1H), 1.80 (m, 2H), 1.68-1.37 (m, 6H), 1.33-1.25 (m, 4H), 1.21 (s, 3H), 0.92 (s, 9H). m/z 1062.53 [M+H]⁺ |
| 10 | ¹H NMR (600 MHz, CDCl₃) δ 8.68 (s, 1H), 8.13 (d, J = 7.1 Hz, 1H), 7.67 (s, 1H), 7.62 (s, 1H), 7.33-7.32 (m, 2H), 7.08 (t, J = 8.1 Hz, 1H), 6.99 (d, J = 5.6 Hz, 1H), 6.95 (d, J = 7.6 Hz, 1H), 6.89 (s, 1H), 6.57 (d, J = 8.0 Hz, 1H), 4.82 (s, 2H), 4.74 (t, J = 7.6 Hz, 1H), 4.59-4.37 (m, 4H), 4.09-4.06 (m, 2H), 4.00 (d, J = 10.9 Hz, 1H), 3.88 (dt, J = 9.7, 4.6 Hz, 2H), 3.62 (dd, J = 11.2, 3.8 Hz, 1H), 3.38-3.30 (m, 2H), 2.97 (m, 2H), 2.64-2.48 (m, 8H), 2.34 (d, J = 6.8 Hz, 2H), 2.11-1.98 (m, 5H), 1.93 (m, 1H), 1.84-1.76 (m, 2H), 1.57-1.23 (m, 10H), 1.02 (s, 3H), 0.92 (s, 9H). m/z 1076.51 [M+H]⁺ |
| 11 | ¹H NMR (600 MHz, CDCl₃) δ 8.68 (s, 1H), 8.22 (d, J = 1.2 Hz, 1H), 8.19 (d, J = 7.9 Hz, 1H), 7.92 (d, J = 1.2 Hz, 1H), 7.69 (s, 1H), 7.32 (d, J = 7.7 Hz, 1H), 7.30-7.27 (m, 1H), 7.11 (t, J = 8.1 Hz, 1H), 7.01 (dd, J = 8.5, 3.1 Hz, 1H), 6.95 (dd, J = 7.7, 1.4 Hz, 1H), 6.89 (d, J = 1.3 Hz, 1H), 6.71 (dd, J = 8.0, 1.3 Hz, 1H), 4.73 (t, J = 7.6 Hz, 1H), 4.57-4.38 (m, 4H), 4.11-4.03 (m, 2H), 3.98 (d, J = 11.3 Hz, 1H), 3.74-3.71 (m, 1H), 3.70-3.59 (m, 2H), 3.51 (dt, J = 10.5, 7.8 Hz, 1H), 3.25 (dd, J = 10.6, 7.5 Hz, 1H), 3.00-2.99 (m, 2H), 2.88-2.79 (m, 2H), 2.64-2.49 (m, 6H), 2.46-2.40 (m, 1H), 2.34 (d, J = 7.0 Hz, 2H), 2.25-2.20 (m, 1H), 2.13-2.00 (m, 4H), 1.99-1.85 (m, 2H), 1.85-1.75 (m, 3H), 1.43-1.27 (m, 6H), 0.92 (s, 9H). m/z 1033.51 [M+H]⁺ |
| 12 | ¹H NMR (600 MHz, CDCl₃) δ 8.68 (s, 1H), 8.23-8.12 (m, 3H), 7.70 (s, 1H), 7.32 (d, J = 7.7 Hz, 1H), 7.25-7.21 (m, 1H), 7.12 (t, J = 8.1 Hz, 1H), 6.95 (dd, J = 7.7, 1.6 Hz, 1H), 6.88 (d, J = 1.5 Hz, 1H), 6.77 (dd, J = 8.0, 1.4 Hz, 1H), 4.71 (t, J = 7.7 Hz, 1H), 4.53 (s, 1H), 4.52-4.45 (m, 2H), 4.41 (dd, J = 14.7, 5.3 Hz, 1H), 4.10-4.03 (m, 2H), 3.95-3.87 (m, 2H), 3.59 (dd, J = 11.3, 3.8 Hz, 1H), 3.36-3.41 (m, 2H), 2.98 (s, 2H), 2.59 (s, 2H), 2.58-2.53 (m, 1H), 2.53 (d, J = 3.2 Hz, 3H), 2.35 (d, J = 7.0 Hz, 2H), 2.12-2.02 (m, 4H), 1.99 (s, 3H), 1.97-1.89 (m, 2H), 1.81 (d, J = 11.5 Hz, 2H), 1.60-1.54 (m, 6H), 1.48-1.44 (m, 3H), 1.43-1.35 (m, 2H), 1.03 (s, 3H), 0.90 (s, 9H). m/z 1017.52 [M+H]⁺ |
| 13 | ¹H NMR (600 MHz, CDCl₃) δ 8.70 (s, 1H), 8.26-8.12 (m, 3H), 7.77 (s, 1H), 7.52-7.46 (m, 1H), 7.36 (d, J = 7.7 Hz, 1H), 7.15 (t, J = 8.1 Hz, 1H), 7.05 (dd, J = 8.7, 3.3 Hz, 1H), 6.98 (dd, J = 7.7, 1.5 Hz, 1H), 6.90 (d, J = 1.4 Hz, 1H), 6.83-6.78 (m, 1H), 4.72 (t, J = 7.9 Hz, 1H), 4.55-4.51 (m, 2H), 4.47-4.43 (m, 2H), 4.25-4.17 (m, 2H), 4.01 (d, J = 11.4 Hz, 1H), 3.97-3.88 (m, 2H), 3.65 (dd, J = 11.2, 3.7 Hz, 1H), 3.42-3.37 (m, 2H), 3.13 (d, J = 10.1 Hz, 2H), 3.01-2.91 (m, 2H), 2.62 (s, 2H), 2.54 (s, 3H), 2.51-2.43 (m, 3H), 2.24 (s, 2H), 2.16-2.09 (m, 1H), 1.75-1.69 (m, 4H), 1.62-1.54 (m, 3H), 1.51-1.45 (m, 2H), 1.42-1.39 (m, 2H), 1.35-1.28 (m, 4H), 1.06 (s, 3H), 0.95 (s, 9H). m/z 1061.52 [M+H]⁺ |
| 14 | ¹H NMR (600 MHz, CDCl₃) δ 8.69 (s, 1H), 8.27-8.13 (m, 3H), 7.73 (s, 1H), 7.34 (d, J = 7.7 Hz, 1H), 7.33-7.29 (m, 1H), 7.14 (t, J = 8.1 Hz, 1H), 7.05 (dd, J = 8.7, 3.3 Hz, 1H), 6.97 (dd, J = 7.7, 1.5 Hz, 1H), 6.90 (d, J = 1.5 Hz, 1H), 6.78 (dd, J = 8.0, 1.4 Hz, 1H), 4.75 (t, J = 7.7 Hz, 1H), 4.58-4.49 (m, 3H), 4.42 (dd, J = 14.7, 5.3 Hz, 1H), 4.10-4.08 (m, 2H), 4.00 (d, J = 11.3 Hz, 1H), 3.95-3.91 (m, 2H), 3.63 (dd, J = 11.2, 3.9 Hz, 1H), 3.42-3.38 (m, 2H), 3.05 (d, J = 9.9 Hz, 2H), 2.71-2.62 (m, 2H), 2.60 (s, 2H), 2.57-2.51 (m, 4H), 2.50-2.43 (m, 2H), 2.16-2.03 (m, 3H), 1.78-1.71 (m, 5H), 1.60-1.55 (m, 2H), 1.49-1.46 (m, 2H), 1.40-1.38 (m, 2H), 1.37-1.34 (m, 2H), 1.33-1.27 (m, 4H), 1.05 (s, 3H), 0.93 (s, 9H). m/z 1075.55 [M+H]⁺ |
| 15 | ¹H NMR (600 MHz, CDCl₃) δ 8.68 (s, 1H), 8.26-8.10 (m, 3H), 7.70 (s, 1H), 7.31 (d, J = 7.7 Hz, 1H), 7.18-7.09 (m, 2H), 7.03-6.97 (m, 1H), 6.95 (dd, J = 7.6, 1.6 Hz, 1H), 6.86 (d, J = 1.5 Hz, 1H), 6.74 (dd, J = 8.0, 1.4 Hz, 1H), 4.72 (t, J = 7.6 Hz, 1H), 4.54-4.40 (m, 4H), 4.09-4.00 (m, 2H), 3.97 (d, J = 10.5 Hz, 1H), 3.94-3.87 (m, 2H), 3.63 (dd, J = 11.2, 4.0 Hz, 1H), 3.45-3.35 (m, 2H), 2.94 (m, 2H), 2.61-2.49 (m, 6H), 2.46-2.40 (m, 4H), 2.11-2.03 (m, 1H), 1.96-1.84 (m, 4H), 1.77-1.42 (m, 12H), 1.40-1.23 (m, 4H), 1.03 (s, 3H), 0.93 (s, 9H). m/z 1089.54 [M+H]⁺ |
| 16 | ¹H NMR (600 MHz, CDCl₃) δ 8.68 (s, 1H), 8.22-8.15 (m, 3H), 7.70 (s, 1H), 7.39-7.35 (m, 1H), 7.33 (d, J = 7.7 Hz, 1H), 7.15-7.10 (m, 1H), 7.02 (dd, J = 8.7, 3.3 Hz, 1H), 6.96 (dd, J = 7.6, 1.6 Hz, 1H), 6.89 (d, J = 1.5 Hz, 1H), 6.77 (dd, J = 8.0, 1.4 Hz, 1H), 4.73-4.68 (m, 1H), 4.52-4.48 (m, 2H), 4.48 (d, J = 6.5 Hz, 1H), 4.43 (dd, J = 14.5, 5.5 Hz, 1H), 4.18-4.15 (m, 2H), 3.99 (d, J = 11.4 Hz, 1H), 3.94-3.88 (m, 2H), 3.63 (dd, J = 11.3, 3.8 Hz, 1H), 3.42-3.35 (m, 2H), 3.03 (d, J = 11.0 Hz, 2H), 2.93-2.83 (m, 2H), 2.59 (s, 2H), 2.55-2.48 (m, 3H), 2.44-2.39 (m, 2H), 2.17-2.00 (m, 3H), 1.77-1.69 (m, 6H), 1.56-1.53 (m, 3H), 1.47-1.43 (m, 2H), 1.38-1.23 (m, 6H), 1.03 (s, 3H), 0.93 (s, 9H). m/z 1075.61 [M+H]⁺ |
| 17 | ¹H NMR (600 MHz, CDCl₃) δ 8.68 (s, 1H), 8.25-8.12 (m, 3H), 7.69 (s, 1H), 7.31 (d, J = 7.7 Hz, 1H), 7.16 (s, 1H), 7.14-7.10 (m, 1H), 7.00 (dd, J = 8.2, 3.2 Hz, 1H), 6.95 (d, J = 7.6 Hz, 1H), 6.86 (s, 1H), 6.76 (dd, J = 8.0, 1.3 Hz, 1H), 4.74-4.71 (m, 1H), 4.54 (s, 1H), 4.52 (d, J = 8.8 Hz, 1H), 4.43-4.39 (m, 2H), 4.07-4.01 (m, 2H), 3.95 (d, J = 10.4 Hz, 1H), 3.93-3.87 (m, 2H), 3.63 (dd, J = 11.1, 4.1 Hz, 1H), 3.42-3.35 (m, 2H), 2.95 (s, 2H), 2.59 (s, 2H), 2.55-2.53 (s, 4H), 2.44-2.38 (m, 2H), 2.11-1.99 (m, 3H), 1.87 (s, 2H), 1.80-1.72 (m, 2H), 1.72-1.67 (m, 2H), 1.68-1.49 (m, 9H), 1.49-1.43 (m, 2H), 1.40-1.23 (m, 6H), 1.03 (s, 3H), 0.92 (s, 9H). m/z 1103.47 [M+H]⁺ |
| 18 | ¹H NMR (600 MHz, CDCl₃) δ 8.68 (s, 1H), 8.26-8.13 (m, 3H), 7.68 (s, 1H), 7.31 (d, J = 7.7 Hz, 1H), 7.15-7.10 (m, 1H), 7.06-7.02 (m, 2H), 6.95 (dd, J = 7.6, 1.5 Hz, 1H), 6.86 (d, J = 1.3 Hz, 1H), 6.76 (dd, J = 8.0, 1.4 Hz, 1H), 4.69-4.64 (m, 1H), 4.59-4.51 (m, 3H), 4.39 (dd, J = 14.3, 5.1 Hz, 1H), 4.04-3.97 (m, 3H), 3.93-3.86 (m, 3H), 3.67-3.65 (m, 2H), 3.41-3.36 (m, 2H), 2.99-2.89 (m, 2H), 2.59 (s, 2H), 2.55-2.47 (m, 4H), 2.44-2.37 (m, 2H), 2.33-2.29 (m, 2H), 2.04-1.99 (m, 3H), 1.97-1.83 (m, 5H), 1.76-1.73 (m, 2H), 1.70-1.68 (m, 2H), 1.65-1.49 (m, 7H), 1.49-1.41 (m, 2H), 1.41-1.36 (m, 2H), 1.36-1.30 (m, 4H), 1.30-1.23 (m, 4H), 1.03 (s, 3H), 0.92 (s, 9H). m/z 1131.56 [M+H]⁺ |
| 19 | ¹H NMR (600 MHz, CDCl₃) δ 9.59 (s, 1H), 8.67 (s, 1H), 8.28-8.13 (m, 3H), 7.32 (d, J = 7.7 Hz, 1H), 7.19 (m, 1H), 7.14-7.10 (m, 1H), 7.05-6.99 (m, 1H), 6.94 (d, J = 7.7 Hz, 1H), 6.87 (s, 1H), 6.74-6.70 (m, 1H), 4.71 (t, J = 7.6 Hz, 1H), 4.57-4.38 (m, 4H), 4.07-3.87 (m, 5H), 3.63 (dd, J = 11.1, 4.1 Hz, 1H), 3.50 (m, 2H), 3.44-3.34 (m, 4H), 3.11 (m, 1H), 2.62-2.46 (m, 8H), 2.09-2.01 (m, 3H), 1.92-1.78 (m, 2H), 1.72-1.26 (m, 12H), 1.03 (s, 3H), 0.93 (s, 9H). m/z 1061.51 [M+H]⁺ |
| 20 | ¹H NMR (400 MHz, CDCl₃) δ 8.68 (s, 1H), 8.61 (s, 1H), 8.19-8.14 (m, 3H), 7.33 (d, J = 7.7 Hz, 1H), 7.30-7.27 (m, 1H), 7.15-7.10 (m, 1H), 7.05-6.99 (m, 1H), 6.96 (dd, J = 7.7, 1.5 Hz, 1H), 6.90 (s, 1H), 6.79-6.74 (M, 1H), 4.72 (t, J = 7.6 Hz, 1H), 4.59-4.38 (m, 4H), 4.09 (t, J = 5.1 Hz, 2H), 3.98 (d, J = 11.5 Hz, 1H), 3.91 (dt, J = 9.3, 4.4 Hz, 2H), 3.63 (dd, J = 11.3, 3.9 Hz, 1H), 3.44-3.35 (m, 2H), 2.73-2.43 (m, 14H), 2.10-2.05 (m, 3H), 1.59-1.42 (m, 8H), 1.35-1.22 (m, 4H), 1.03 (s, 3H), 0.93 (s, 9H). m/z 1077.56 [M+H]⁺ |
| 21 | ¹H NMR (600 MHz, CDCl₃) δ 8.68 (s, 1H), 8.20-8.12 (m, 3H), 7.77 (s, 1H), 7.39-7.29 (m, 2H), 7.13 (t, J = 8.1 Hz, 1H), 7.03-6.97 (m, 1H), 6.95 (d, J = 7.7 Hz, 1H), 6.89 (s, 1H), 6.78 (dd, J = 8.0, 1.3 Hz, 1H), 4.75-4.66 (m, 2H), 4.58-4.32 (m, 4H), 4.14-4.03 (m, 3H), 3.99-3.88 (m, 3H), 3.65-3.58 (m, 1H), 3.38 (ddd, J = 13.5, 10.1, 3.4 Hz, 2H), 3.14 (dd, J = 25.3, 12.1 Hz, 1H), 2.78-2.69 (m, 1H), 2.66-2.51 (m, 8H), 2.23-2.21 (m, 2H), 2.06-2.00 (m, 3H), 1.85-1.71 (m, 3H), 1.59-1.42 (m, 4H), 1.38-1.22 (m, 4H), 1.04 (s, 3H), 0.91 (d, J = 13.8 Hz, 9H). m/z 1075.53 [M+H]⁺ |
| 22 | ¹H NMR (600 MHz, CDCl₃) δ 8.68 (s, 1H), 8.18-8.13 (m, 3H), 7.94-7.91 (m, 1H), 7.79 (d, J = 8.5 Hz, 1H), 7.35 (d, J = 7.7 Hz, 1H), 7.13 (t, J = 8.1 Hz, 1H), 7.01 (m, 2H), 6.93 (s, 1H), 6.80 (t, J = 7.2 Hz, 1H), 4.87-4.77 (m, 2H), 4.75-4.41 (m, 6H), 3.93-3.90 (m, 4H), 3.70-3.64 (m, 1H), 3.38 (t, J = 10.5 Hz, 2H), 3.18-3.09 (m, 1H), 2.71-2.63 (m, 1H), 2.60 (s, 2H), 2.52 (s, 3H), 2.43-2.26 (m, 3H), 2.14-1.79 (m, 4H), 1.61-1.38 (m, 6H), 1.38-1.18 (m, 4H), 1.04 (s, 3H), 0.94 (d, J = 9.1 Hz, 9H). m/z 1061.57 [M+H]⁺ |
| 23 | ¹H NMR (600 MHz, CDCl₃) δ 8.67 (s, 1H), 8.30 (s, 1H), 8.17-8.11 (m, 3H), 7.80 (s, 1H), 7.56-7.48 (m, 1H), 7.49-7.42 (m, 1H), 7.33-7.31 (m, 1H), 7.14 (t, J = 8.1 Hz, 1H), 7.03-6.99 (m, 1H), 6.94 (d, J = 7.8 Hz, 1H), 6.90-6.85 (m, 1H), 4.73-4.69 (m, 1H), 4.64 (d, J = 11.9 Hz, 1H), 4.53-4.47 (m, 2H), 4.42-4.37 (m, 1H), 4.13-4.04 (m, 2H), 3.94-3.87 (m, 3H), 3.63-3.59 (m, 1H), 3.38-3.37 (m, 2H), 3.11-3.00 (m, 2H), 2.75 (s, 2H), 2.69-2.64 (m, 1H), 2.63-2.55 (m, 2H), 2.53 (s, 3H), 2.44-2.38 (m, 2H), 2.30-2.26 (m, 1H), 2.13-2.11 (m, 3H), 2.08-2.04 (m, 1H), 1.91-1.83 (m, 1H), 1.79 (d, J = 12.3 Hz, 1H), 1.62-1.59 (m, 2H), 1.56-1.51 (m, 2H), 1.35-1.19 (m, 8H), 1.14 (s, 3H), 0.91 (s, 9H). m/z 1089.52 [M+H]⁺ |
| 24 | ¹H NMR(600 MHz, CDCl₃) δ 8.68 (s, 1H), 8.23-8.12 (m, 3H), 7.77 (s, 1H), 7.71 (s, 1H), 7.40-7.33 (m, 1H), 7.31 (d, J = 7.7 Hz, 1H), 7.16-7.10 (m, 1H), 7.02-6.97 (m, 1H), 6.95 (d, J = 7.6 Hz, 1H), 6.89 (d, J = 1.3 Hz, 1H), 6.76 (d, J = 7.9 Hz, 1H), 4.72 (dd, J = 12.7, 7.6 Hz, 1H), 4.63 (d, J = 12.8 Hz, 1H), 4.55-4.47 (m, 3H), 4.44-4.37 (m, 1H), 4.14-4.07 (m, 2H), 4.02-3.94 (m, 2H), 3.94-3.88 (m, 2H), 3.64-3.58 (m, 1H), 3.40-3.36 (m, 2H), 3.02-2.94 (m, 2H), 2.73-2.66 (m, 1H), 2.62-2.58 (m, 2H), 2.56-2.53 (m, 1H), 2.53 (s, 3H), 2.47-2.42 (m, 2H), 2.22-2.17 (m, 2H), 2.10-2.06 (m, 1H), 1.76 (d, J = 12.2 Hz, 2H), 1.72-1.67 (m, 2H), 1.60-1.54 (m, 5H), 1.48-1.43 (m, 2H), 1.34-1.25 (m, 4H), 1.18-1.08 (m, 2H), 1.03 (s, 3H), 0.90 (s, 9H). m/z 1103.57 [M+H]⁺ |
| 25 | ¹H NMR (600 MHz, CDCl₃) δ 8.67 (s, 1H), 8.20-8.14 (m, 3H), 7.69 (d, J = 6.5 Hz, 1H), 7.40-7.38 (m, 1H), 7.36-7.32 (m, 1H), 7.31 (d, J = 7.5 Hz, 1H), 7.13 (t, J = 8.1 Hz, 1H), 7.01-6.98 (m, 1H), 6.95-6.94 (m, 1H), 6.89-6.86 (m, 1H), 6.77 (dd, J = 8.0, 1.3 Hz, 1H), 4.74-4.69 (m, 1H), 4.61 (d, J = 12.6 Hz, 1H), 4.53-4.49 (m, 3H), 4.43-4.37 (m, 1H), 4.09-4.05 (m, 3H), 3.96 (d, J = 11.3 Hz, 1H), 3.93-3.89 (m, 2H), 3.64-3.59 (m, 2H), 3.40-3.36 (m, 2H), 3.01-2.94 (m, 2H), 2.71-2.66 (m, 1H), 2.62-2.57 (m, 2H), 2.53 (s, 4H), 2.46-2.36 (m, 2H), 2.23-2.17 (m, 2H), 2.12-1.98 (m, 2H), 1.81-1.68 (m, 4H), 1.59-1.54 (m, 2H), 1.53-1.50 (m, 1H), 1.48-1.45 (m, 2H), 1.38-1.31 (m, 2H), 1.27-1.21 (m, 4H), 1.14-1.06 (m, 2H), 1.04 (s, 3H), 0.92-0.89 (m, 9H). m/z 1117.46 [M+H]⁺ |
| 26 | ¹H NMR (600 MHz, CDCl₃) δ 8.67 (s, 1H), 8.22-8.15 (m, 3H), 7.68 (s, 1H), 7.32 (d, J = 7.6 Hz, 1H), 7.22-7.17 (m, 1H), 7.15-7.10 (m, 1H), 7.07-7.03 (m, 1H), 6.95 (d, J = 7.6 Hz, 1H), 6.86 (s, 1H), 6.76 (d, J = 7.9 Hz, 1H), 4.74-4.68 (m, 1H), 4.59-4.42 (m, 6H), 4.07-3.99 (m, 2H), 3.98-3.79 (m, 4H), 3.64 (d, J = 7.9 Hz, 1H), 3.42-3.35 (m, 2H), 3.02-2.95 (m, 1H), 2.59 (s, 2H), 2.55-2.47 (m, 4H), 2.45-2.40 (m, 2H), 2.40-2.35 (m, 2H), 2.14-2.07 (m, 1H), 1.94-1.85 (m, 2H), 1.78-1.73 (m, 2H), 1.74-1.68 (m, 3H), 1.65-1.51 (m, 6H), 1.47-1.45 (m, 2H), 1.35-1.33 (m, 2H), 1.32-1.26 (m, 4H), 1.39-1.05 (m, 2H), 1.03 (s, 3H), 1.00-0.84 (m, 9H). m/z 1145.50 [M+H]⁺ |
| 27 | ¹H NMR (400 MHz, CDCl₃) δ 9.76 (d, J = 5.7 Hz, 1H), 8.69 (s, 1H), 8.25-8.12 (m, 3H), 7.36-7.30 (m, 2H), 7.14 (t, J = 8.0 Hz, 1H), 7.03-6.93 (m, 2H), 6.87 (d, J = 1.2 Hz, 1H), 6.81-6.74 (m, 1H), 5.21-5.07 (m, 1H), 4.74 (t, J = 7.1 Hz, 1H), 4.62 (d, J = 12.8 Hz, 1H), 4.58-4.35 (m, 4H), 4.13-3.98 (m, 3H), 3.97-3.85 (m, 2H), 3.65-3.55 (m, 1H), 3.40 (dt, J = 11.8, 5.4 Hz, 2H), 3.26-3.12 (m, 1H), 2.83 (t, J = 12.8 Hz, 1H), 2.67-2.56 (m, 3H), 2.53 (s, 3H), 2.12-1.44 (m, 12H), 1.40-1.17 (m, 4H), 1.07 (s, 3H), 0.91 (s, 9H). m/z 1061.42 [M+H]⁺ |
| 28 | ¹H NMR (400 MHz, CDCl₃) δ 9.76 (s, 1H), 8.68 (s, 1H), 8.24-8.13 (m, 3H), 7.34-7.28 (m, 2H), 7.15 (t, J = 8.2 Hz, 1H), 7.03-6.93 (m, 2H), 6.86 (s, 1H), 6.82 (d, J = 8.0 Hz, 1H), 5.13-5.09 (m, 1H), 4.74 (t, J = 7.7 Hz, 1H), 4.65-4.37 (m, 5H), 4.03-3.89 (m, 5H), 3.61 (dd, J = 11.4, 3.7 Hz, 1H), 3.39 (ddd, J = 13.5, 10.0, 3.5 Hz, 2H), 3.14 (t, J = 12.3 Hz, 1H), 2.82-2.76 (m, 1H), 2.66-2.55 (m, 3H), 2.53 (s, 3H), 2.13-2.00 (m, 1H), 1.93-1.87 (m, 4H), 1.62-1.25 (m, 13H), 1.04 (s, 3H), 0.92 (s, 9H). m/z 1075.44 [M+H]⁺ |
| 29 | ¹H NMR (400 MHz, CDCl₃) δ 10.57 (d, J = 36.8 Hz, 1H), 8.68 (s, 1H), 8.23-8.15 (m, 3H), 7.35-7.32 (m, 2H), 7.12 (t, J = 7.9 Hz, 1H), 7.03-6.96 (m, 2H), 6.84 (t, J = 1.8 Hz, 1H), 6.81-6.78 (m, 1H), 4.80-4.69 (m, 2H), 4.59-4.48 (m, 3H), 4.42-4.30 (m, 1H), 4.06-3.82 (m, 4H), 3.75-3.52 (m, 7H), 3.20 (t, J = 13.3 Hz, 1H), 2.76-2.70 (m, 1H), 2.61-2.55 (m, 1H), 2.52 (s, 3H), 2.18 (m, 1H), 2.09-1.90 (m, 3H), 1.66-1.25 (m, 10H), 1.22 (s, 3H), 0.91 (d, J = 12.8 Hz, .9H). m/z 1047.47 [M+H]⁺ |
| 30 | ¹H NMR (400 MHz, CDCl₃) δ 10.50 (d, J = 35.9 Hz, 1H), 8.68 (s, 1H), 8.15-8.11 (m, 1H), 7.63 (d, J = 4.5 Hz, 1H), 7.34 (dd, J = 7.6, 2.1 Hz, 1H), 7.23 (d, J = 5.5 Hz, 1H), 7.11-6.95 (m, 3H), 6.86-6.81 (m, 1H), 6.58 (dd, J = 8.0, 1.3 Hz, 1H), 4.86-4.78 (m, 3H), 4.70 (t, J = 7.6 Hz, 1H), 4.59-4.45 (m, 4H), 4.40-4.29 (m, 4H), 3.72-3.56 (m, 7H), 3.20 (t, J = 13.3 Hz, 1H), 2.73 (dd, J = 12.7, 10.4 Hz, 1H), 2.60-2.54 (m, 1H), 2.52 (s, 3H), 2.18-2.17 (m, 1H), 2.07-1.88 (m, 5H), 1.66-1.24 (m, 8H), 1.22 (s, 3H), 0.91 (d, J = 8.9 Hz, 9H). m/z 1062.37 [M+H]⁺ |
| 31 | ¹H NMR (600 MHz, CDCl₃) δ 10.59 (s, 1H), 10.51 (s, 1H), 8.68 (s, 1H), 8.23-8.14 (m, 3H), 7.35-7.33 (m, 1H), 7.30 (s, 1H), 7.45-7.11 (m, 1H), 7.03-7.01 (m, 1H), 6.97 (d, J = 6.6 Hz, 1H), 6.84 (s, 1H), 6.82-6.80 (m, 1H), 4.78 (d, J = 13.1 Hz, 1H), 4.73-4.70 (m, 1H), 4.57-4.48 (m, 3H), 4.39 (dd, J = 14.9, 5.1 Hz, 1H), 4.33 (dd, J = 14.6, 4.7 Hz, 1H), 4.07-4.03 (m, 1H), 3.98 (dd, J = 21.2, 11.4 Hz, 1H), 3.91-3.88 (m, 3H), 3.86-3.82 (m, 1H), 3.61 (d, J = 8.9 Hz, 1H), 3.57-3.52 (m, 2H), 3.39-3.35 (m, 2H), 3.21-3.17 (m, 2H), 2.75-2.71 (m, 1H), 2.61-2.55 (m, 3H), 2.52 (s, 3H), 2.08-2.00 (m, 2H), 1.96-1.90 (m, 2H), 1.57-1.42 (m, 2H), 1.47-1.40 (m, 2H), 1.33-1.25 (m, 4H), 1.04 (s, 3H), 0.93-0.85 (m, 9H). m/z 1061.31 [M+H]⁺ |
| 32 | ¹H NMR(400 MHz, CDCl₃) δ 10.54-10.42 (m, 1H), 8.68 (s, 1H), 8.14-8.11 (m, 1H), 7.64-7.62 (m, 1H), 7.36-7.33 (m, 1H), 7.25-7.23 (m, 1H), 7.10-6.96 (m, 3H), 6.84-6.83 (m, 1H), 6.59-6.56 (m, 1H), 4.89-4.75 (m, 3H), 4.70 (t, J = 7.6 Hz, 1H), 4.59-4.46 (m, 3H), 4.41-4.28 (m, 1H), 4.10-3.81 (m, 6H), 3.65-3.53 (m, 3H), 3.40-3.33 (m, 2H), 3.25-3.14 (m, 1H), 2.79-2.70 (m, 1H), 2.60-2.54 (m, 3H), 2.52 (s, 3H), 2.18-2.17 (m, 1H), 2.07-1.89 (m, 5H), 1.70-1.24 (m, 8H), 1.05 (s, 3H), 0.91 (d, J = 8.9 Hz, 9H). m/z 1076.56 [M+H]⁺ |
| 33 | ¹H NMR (400 MHz, CDCl₃) δ 10.71-10.45 (m, 1H), 8.68 (s, 1H), 8.24-8.13 (m, 3H), 7.35 (dd, J = 7.7, 5.2 Hz, 1H), 7.24-7.17 (m, 1H), 7.15-7.10 (m, 1H), 6.96 (dd, J = 7.7, 1.4 Hz, 1H), 6.84 (s, 1H), 6.82-6.78 (m, 1H), 6.68-6.36 (m, 1H), 4.79 (t, J = 12.9 Hz, 1H), 4.70-4.64 (m, 1H), 4.54-4.31 (m, 4H), 4.12-4.04 (m, 2H), 3.96-3.90 (m 4H), 3.84-3.72 (m, 1H), 3.59-3.55 (m, 2H), 3.41-3.34 (m, 2H), 3.18 (t, J = 12.9 Hz, 1H), 2.77-2.67 (m, 1H), 2.60 (s, 2H), 2.58-2.48 (m, 4H), 2.19-2.10 (m, 1H), 2.08 (s, 3H), 1.64-1.31 (m, 9H), 1.04 (s, 3H), 0.88 (s, 9H). m/z 1017.48 [M+H]⁺ |
| 34 | ¹H NMR (400 MHz, CDCl₃) δ 10.73 (d, J = 3.6 Hz, 1H), 8.69 (s, 1H), 8.28-8.12 (m, 3H), 7.38-7.29 (m, 2H), 7.12 (t, J = 8.1 Hz, 1H), 7.02-6.92 (m, 2H), 6.86 (s, 1H), 6.80 (dd, J = 8.0, 1.4 Hz, 1H), 4.74-4.69 (m, 2H), 4.54-4.49 (m, 3H), 4.42-4.37 (m, 1H), 4.15-4.04 (m, 2H), 4.01-3.94 (m, 2H), 3.80-3.46 (m, 7H), 3.15 (dd, J = 24.1, 12.0 Hz, 1H), 2.74-2.56 (m, 2H), 2.53 (s, 3H), 2.12-2.00 (m, 1H), 2.00-1.77 (m, 4H), 1.74-1.47 (m, 7H), 1.36-1.24 (m, 4H), 1.22 (s, 3H), 0.90 (d, J = 1.0 Hz, 9H). m/z 1061.47 [M+H]⁺ |
| 35 | ¹H NMR (400 MHz, CDCl₃) δ 10.65 (s, 1H), 8.69 (s, 1H), 8.15-8.12 (m, 1H), 7.63 (s, 1H), 7.37-7.29 (m, 2H), 7.08 (t, J = 8.1 Hz, 1H), 7.00-6.96 (m, 2H), 6.87 (s, 1H), 6.62-6.57 (m, 1H), 4.84 (s, 2H), 4.74-4.70 (m, 2H), 4.58-4.47 (m, 3H), 4.44-4.36 (m, 1H), 4.13-3.93 (m, 4H), 3.74-3.55 (m, 5H), 3.52 (s, 2H), 3.22-3.09 (m, 1H), 2.75-2.56 (m, 2H), 2.53 (s, 3H), 2.13-2.00 (m, 2H), 1.97-1.81 (m, 5H), 1.68-1.45 (m, 5H), 1.38-1.17 (m, 4H), 1.00 (s, 3H), 0.91 (s, 9H). m/z 1076.37 [M+H]⁺ |
| 36 | ¹H NMR (600 MHz, CDCl₃) δ 10.70 (d, J = 6.8 Hz, 1H), 8.68 (s, 1H), 8.22 (d, J = 8.3 Hz, 1H), 8.17 (s, 1H), 8.13 (s, 1H), 7.36-7.29 (m, 2H), 7.12 (t, J = 8.1 Hz, 1H), 6.97-6.96 (m, 2H), 6.86 (s, 1H), 6.80 (d, J = 7.9 Hz, 1H), 4.73-4.69 (m, 2H), 4.54-4.49 (m, 3H), 4.44-4.37 (m, 1H), 4.09-4.07 (m, 2H), 4.00 (d, J = 11.3 Hz, 1H), 3.96 (d, J = 13.5 Hz, 1H), 3.92-3.88 (m, 2H), 3.61-3.58 (m, 1H), 3.51 (s, 2H), 3.40-3.35 (m, 2H), 3.15 (dd, J = 30.0, 12.4 Hz, 1H), 2.69 (t, J = 12.0 Hz, 1H), 2.65-2.56 (m, 3H), 2.53 (s, 3H), 2.10-2.00 (m, 1H), 1.95-1.82 (m, 4H), 1.60-1.42 (m, 7H), 1.38-1.20 (m, 4H), 1.03 (s, 3H), 0.91 (s, 9H). m/z 1075.45 [M+H]⁺ |
| 37 | ¹H NMR (400 MHz, CDCl₃) δ 10.64 (s, 1H), 8.69 (s, 1H), 8.13 (d, J = 8.1 Hz, 1H), 7.61 (s, 1H), 7.36-7.28 (m, 2H), 7.07 (t, J = 8.1 Hz, 1H), 7.01-6.93 (m, 2H), 6.87 (s, 1H), 6.64-6.55 (m, 1H), 4.83 (s, 2H), 4.74-4.70 (m, 2H), 4.55-4.48 (m, 3H), 4.45-4.35 (m, 1H), 4.11-4.06 (m, 2H), 4.02-3.95 (m, 2H), 3.91-3.85 (m, 2H), 3.60 (dd, J = 11.5, 3.8 Hz, 1H), 3.52 (s, 2H), 3.39-3.30 (m, 2H), 3.20-3.11 (m, 1H), 2.73-2.66 (m, 1H), 2.65-2.55 (m, 3H), 2.53 (s, 3H), 2.15-1.84 (m, 7H), 1.53-1.36 (m, 5H), 1.34-1.23 (m, 4H), 1.03 (s, 3H), 0.91 (s, 9H). m/z 1090.36 [M+H]⁺ |
| 38 | ¹H NMR (600 MHz, CDCl₃) δ 10.85 (s, 1H), 10.64 (s, 1H), 8.68 (s, 1H), 8.22-8.13 (m, 3H), 7.35 (dd, J = 12.9, 7.7 Hz, 1H), 7.18-7.16 (m, 1H), 7.12 (t, J = 8.1 Hz, 1H), 6.97 (d, J = 7.6 Hz, 1H), 6.86 (s, 1H), 6.81 (dd, J = 12.6, 7.9 Hz, 1H), 6.67 (d, J = 8.6 Hz, 1H), 6.34 (d, J = 8.6 Hz, 1H), 4.74-4.68 (m, 2H), 4.52 (s, 1H), 4.47-4.43 (m, 2H), 4.16 (d, J = 11.5 Hz, 1H), 4.10-4.06 (m, 2H), 3.99 (d, J = 13.4 Hz, 1H), 3.93-3.89 (m, 2H), 3.63-3.49 (m, 3H), 3.40-3.36 (m, 2H), 3.16-3.10 (m, 1H), 2.72-2.67 (m, 1H), 2.59-2.54 (m, 2H), 2.52 (s, 3H), 2.08-2.04 (m, 1H), 2.01-1.89 (m, 3H), 1.93-1.89 (m, 2H), 1.85-1.83 (m, 2H), 1.65-1.63 (m, 1H), 1.58-1.54 (m, 2H), 1.47-1.45 (m, 2H), 1.33-1.29 (m, 4H), 1.03 (s, 3H), 0.92-0.79 (m, 9H). m/z 1031.54 [M+H]⁺ |
| 39 | ¹H NMR (400 MHz, CDCl₃) δ 10.69 (d, J = 2.3 Hz, 1H), 8.68 (s, 1H), 8.27-8.12 (m, 3H), 7.33 (d, J = 7.7 Hz, 1H), 7.30-7.27 (m, 1H), 7.12 (t, J = 8.1 Hz, 1H), 7.05-6.93 (m, 2H), 6.85 (d, J = 1.4 Hz, 1H), 6.82-6.76 (m, 1H), 4.75-4.67 (m, 2H), 4.60-4.38 (m, 4H), 4.03-3.93 (m, 4H), 3.80-3.69 (m, 2H), 3.69-3.57 (m, 3H), 3.51 (s, 2H), 3.10 (t, J = 12.1 Hz, 1H), 2.72-2.55 (m, 2H), 2.53 (s, 3H), 2.12-1.99 (m, 1H), 1.94-1.78 (m, 4H), 1.71-1.45 (m, 7H), 1.35-1.24 (m, 6H), 1.22 (s, 3H), 0.92 (s, 9H). m/z 1075.55 [M+H]⁺ |
| 40 | ¹H NMR (400 MHz, CDCl₃) δ 10.58 (s, 1H), 8.66 (s, 1H), 8.11 (d, J = 7.6 Hz, 1H), 7.62 (d, J = 1.4 Hz, 1H), 7.31 (d, J = 7.8 Hz, 1H), 7.26 (d, J = 1.6 Hz, 1H), 7.06 (t, J = 8.1 Hz, 1H), 6.99-6.93 (m, 2H), 6.84 (s, 1H), 6.57 (dd, J = 7.9, 1.2 Hz, 1H), 4.82 (s, 2H), 4.73-4.67 (m, 2H), 4.61-4.31 (m, 4H), 4.05-3.89 (m, 4H), 3.75-3.64 (m, 2H), 3.61-3.57 (m, 3H), 3.50 (s, 2H), 3.09 (t, J = 12.3 Hz, 1H), 2.71-2.54 (m, 2H), 2.51 (s, 3H), 2.07-1.99 (m, 1H), 1.94-1.78 (m, 4H), 1.61-1.48 (m, 4H), 1.38-1.13 (m, 12H), 0.91 (s, 9H). m/z 1090.52 [M+H]⁺ |
| 41 | ¹H NMR (600 MHz, CDCl₃) δ 10.68 (s, 1H), 8.68 (s, 1H), 8.26-8.11 (m, 3H), 7.33-7.27 (m, 2H), 7.12 (t, J = 8.1 Hz, 1H), 7.01-6.94 (m, 2H), 6.85 (d, J = 1.4 Hz, 1H), 6.80 (dd, J = 7.9, 1.3 Hz, 1H), 4.77-4.66 (m, 2H), 4.59-4.47 (m, 3H), 4.42-4.39 (m, 1H), 4.08-3.98 (m, 3H), 3.98-3.87 (m, 3H), 3.60 (dd, J = 11.2, 3.9 Hz, 1H), 3.52 (s, 2H), 3.40-3.35 (m, 2H), 3.10 (t, J = 12.8 Hz, 1H), 2.66 (t, J = 11.6 Hz, 1H), 2.61-2.56 (m, 3H), 2.53 (s, 3H), 2.09-2.04 (m, 1H), 1.93-1.81 (m, 4H), 1.58-1.44 (m, 5H), 1.35-1.19 (m, 8H), 1.03 (s, 3H), 0.92 (s, 9H). m/z 1089.43 [M+H]⁺ |
| 42 | ¹H NMR (400 MHz, CDCl₃) δ 10.57 (s, 1H), 8.68 (s, 1H), 8.13 (d, J = 8.1 Hz, 1H), 7.62 (d, J = 1.9 Hz, 1H), 7.33 (d, J = 7.7 Hz, 1H), 7.07 (t, J = 8.1 Hz, 1H), 7.02-6.93 (m, 2H), 6.85 (s, 1H), 6.58 (t, J = 6.0 Hz, 1H), 4.83 (s, 2H), 4.74-4.68 (m, 2H), 4.57-4.46 (m, 3H), 4.40 (dd, J = 14.7, 5.1 Hz, 1H), 4.02-3.94 (m, 4H), 3.90-3.85 (m, 2H), 3.60 (dd, J = 11.2, 3.9 Hz, 1H), 3.52 (s, 2H), 3.39-3.28 (m, 2H), 3.16-3.05 (m, 1H), 2.66 (t, J = 11.6 Hz, 1H), 2.60-2.55 (m, 3H), 2.52 (s, 3H), 2.09-1.99 (m, 2H), 1.93-1.83 (m, 5H), 1.46-1.40 (m, 2H), 1.35-1.31 (m, 4H), 1.30-1.24 (m, 4H), 1.20-1.18 (m, 2H), 1.02 (s, 3H), 0.92 (s, 9H). m/z 1104.57 [M+H]⁺ |
| 43 | ¹H NMR (400 MHz, CDCl₃) δ 10.65 (s, 1H), 8.67 (s, 1H), 8.23-8.14 (m, 3H), 7.32 (dd, J = 7.7, 1.7 Hz, 1H), 7.24-7.07 (m, 2H), 6.95 (d, J = 7.7 Hz, 1H), 6.85 (s, 1H), 6.81 (d, J = 8.0 Hz, 1H), 6.24 (dd, J = 12.5, 8.7 Hz, 1H), 4.70 (t, J = 7.8 Hz, 2H), 4.57-4.39 (m, 4H), 4.10 (d, J = 11.2 Hz, 1H), 4.02-3.99 (m, 3H), 3.95-3.88 (m, 2H), 3.62-3.52 (m, 3H), 3.41-3.34 (m, 2H), 3.11 (t, J = 13.1 Hz, 1H), 2.66 (t, J = 11.7 Hz, 1H), 2.59 (s, 2H), 2.52 (s, 3H), 2.12-2.07 (m, 2H), 2.02 (d, J = 4.6 Hz, 3H), 1.93-1.79 (m, 5H), 1.59-1.53 (m, 4H), 1.49-1.43 (m, 4H), 1.03 (s, 3H), 0.91 (s, 9H). m/z 1045.55 [M+H]⁺ |
| 44 | ¹H NMR (600 MHz, CDCl₃) δ 8.68-8.66 (m, 1H), 8.53 (s, 1H), 8.23-8.10 (m, 3H), 7.42 (s, 1H), 7.35 (dd, J = 14.3, 7.7 Hz, 1H), 7.10-7.08 (m, 1H), 7.01 (dd, J = 16.9, 7.2 Hz, 1H), 6.98-6.96 (m, 1H), 6.84 (d, J = 6.3 Hz, 1H), 6.73 (dd, J = 16.7, 7.9 Hz, 1H), 4.75 (d, J = 12.9 Hz, 1H), 4.72-4.69 (m, 1H), 4.57-4.52 (m, 2H), 4.36 (dd, J = 14.9, 4.6 Hz, 1H), 4.00-3.96 (m, 2H), 3.92-3.84 (m, 3H), 3.62 (d, J = 11.0 Hz, 1H), 3.39-3.36 (m, 2H), 3.12 (t, J = 13.0 Hz, 1H), 2.92-2.88 (m, 1H), 2.83-2.75 (m, 2H), 2.77-2.74 (m, 1H), 2.68-2.64 (m, 1H), 2.58-2.54 (m, 2H), 2.52-2.46 (m, 3H), 2.23-2.21 (m, 1H), 2.13 (s, 1H), 2.05-2.00 (m, 2H), 1.97-1.93 (m, 1H), 1.89-1.85 (m, 2H), 1.65-1.63 (m, 2H), 1.58-1.53 (m, 2H), 1.49-1.40 (m, 3H), 1.33-1.31 (m, 2H), 1.27-1.25 (m, 4H), 1.03 (s, 3H), 0.93-0.83 (m, 9H). m/z 1075.34 [M+H]⁺ |
| 45 | ¹H NMR (600 MHz, CDCl₃) δ 8.68 (s, 1H), 8.57 (d, J = 11.3 Hz, 1H), 8.28-8.13 (m, 3H), 7.33 (d, J = 7.7 Hz, 1H), 7.30 (d, J = 5.7 Hz, 1H), 7.11-7.08 (m, 1H), 6.98-6.96 (m, 1H), 6.87 (d, J = 1.5 Hz, 1H), 6.75 (dd, J = 7.9, 2.1 Hz, 1H), 4.74-4.70 (m, 1H), 4.64 (d, J = 12.6 Hz, 1H), 4.53-4.49 (m, 2H), 4.42-4.38 (m, 1H), 4.10-4.03 (m, 2H), 4.00 (d, J = 11.1 Hz, 1H), 3.92-3.88 (m, 2H), 3.59 (dd, J = 11.1, 3.4 Hz, 1H), 3.40-3.36 (m, 2H), 3.11-3.05 (m, 2H), 2.78-2.75 (m, 4H), 2.65-2.60 (m, 1H), 2.58 (s, 2H), 2.53 (s, 3H), 2.09-2.05 (m, 1H), 1.89-1.82 (m, 3H), 1.73-1.53 (m, 9H), 1.47-1.42 (m, 2H), 1.34-1.23 (m, 4H), 1.03 (s, 3H), 0.91-0.78 (m, 9H). m/z 1089.44 [M+H]⁺ |
| 46 | ¹H NMR (400 MHz, CDCl₃) δ 8.67 (s, 1H), 8.43 (s, 1H), 8.25-8.12 (m, 3H), 7.41-7.30 (m, 2H), 7.15-7.08 (m, 1H), 7.05-6.98 (m, 1H), 6.96 (dd, J = 7.7, 1.5 Hz, 1H), 6.83 (d, J = 1.3 Hz, 1H), 6.73-6.64 (m, 1H), 4.70 (t, J = 7.4 Hz, 1H), 4.57-4.16 (m, 5H), 4.04-3.76 (m, 6H), 3.60-3.58 (m, 1H), 3.48-3.32 (m, 2H), 3.07 (m, 3H), 2.60-2.46 (m, 5H), 2.13-1.24 (m, 18H), 1.06 (s, 3H), 0.88 (s, 9H). m/z 1087.45 [M+H]⁺ |
| 47 | ¹H NMR (400 MHz, CDCl₃) δ 8.68 (s, 1H), 8.38 (s, 1H), 8.23-8.11 (m, 3H), 7.33-7.30 (m, 2H), 7.16-7.07 (m, 1H), 7.02-6.92 (m, 2H), 6.85 (s, 1H), 6.76-6.66 (m, 1H), 4.70 (t, J = 7.7 Hz, 1H), 4.53-4.49 (m, 4H), 4.40-4.35 (m, 1H), 4.05 (m, 2H), 3.97-3.90 (m, 4H), 3.59 (dd, J = 11.2, 4.0 Hz, 1H), 3.45-3.34 (m, 2H), 3.07 (m, 1H), 2.65-2.45 (m, 7H), 2.08-1.25 (m, 20H), 1.03 (s, 3H), 0.88 (s, 9H). m/z 1101.51 [M+H]⁺ |
| 48 | ¹H NMR (400 MHz, CDCl₃) δ 8.67 (s, 1H), 8.39 (s, 1H), 8.24-8.13 (m, 3H), 7.38-7.31 (m, 2H), 7.10 (t, J = 8.2 Hz, 1H), 7.01 (dd, J = 7.6, 2.6 Hz, 1H), 6.95 (dd, J = 7.7, 1.4 Hz, 1H), 6.85 (d, J = 1.5 Hz, 1H), 6.67 (d, J = 7.0 Hz, 1H), 4.72 (t, J = 7.7 Hz, 1H), 4.59-4.47 (m, 4H), 4.39-4.34 (m, 1H), 4.04-3.88 (m, 6H), 3.60 (dd, J = 11.2, 3.9 Hz, 1H), 3.46-3.34 (m, 2H), 3.08 (m, 3H), 2.55-2.50 (m, 5H), 2.04-1.17 (m, 22H), 1.07 (s, 3H), 0.92 (s, 9H). m/z 1115.82 [M+H]⁺ |
| 49 | ¹H NMR (400 MHz, CDCl₃) δ 8.67 (s, 1H), 8.21-8.06 (m, 3H), 7.70 (s, 1H), 7.32 (d, J = 7.7 Hz, 1H), 7.23 (d, J = 6.9 Hz, 1H), 7.13 (t, J = 8.1 Hz, 1H), 7.03-6.98 (m, 1H), 6.95 (dd, J = 7.7, 1.6 Hz, 1H), 6.84 (d, J = 1.5 Hz, 1H), 6.72 (dd, J = 8.0, 1.4 Hz, 1H), 4.68 (t, J = 7.7 Hz, 2H), 4.53-4.47 (m, 3H), 4.38-4.33 (m, 1H), 4.09-3.85 (m, 5H), 3.85-3.67 (m, 1H), 3.62-3.52 (m, 1H), 3.38 (ddd, J = 13.5, 10.0, 3.6 Hz, 2H), 2.98-2.65 (m, 2H), 2.62-2.48 (m, 6H), 2.06-2.02 (m, 1H), 1.83-1.42 (m, 17H), 1.35-1.21 (m, 4H), 1.03 (s, 3H), 0.89 (s, 9H). m/z 1103.55 [M+H]⁺ |
| 50 | ¹H NMR (400 MHz, CDCl₃) δ 10.60 (d, J = 10.8 Hz, 1H), 8.68 (s, 1H), 8.24-8.12 (m, 3H), 7.36 (t, J = 6.0 Hz, 1H), 7.33 (d, J = 7.7 Hz, 1H), 7.13-7.08 (m, 1H), 7.01-6.98 (m, 1H), 6.95 (dd, J = 7.7, 1.5 Hz, 1H), 6.76-6.74 (m, 1H), 6.66 (s, 1H), 4.78-4.73 (m, 2H), 4.56-4.41 (m, 4H), 4.04-4.00 (m, 1H), 3.94-3.88 (m, 2H), 3.73-3.68 (m, 1H), 3.63-3.59 (m, 2H), 3.52-3.50 (m, 3H), 3.46-3.37 (m, 3H), 3.07 (s, 2H), 2.66-2.59 (m, 1H), 2.52 (s, 3H), 2.50-2.45 (m, 2H), 2.21-2.17 (m, 1H), 2.10-2.05 (m, 2H), 1.79-1.66 (m, 6H), 1.55-1.50 (m, 2H), 1.37-1.28 (m, 4H), 1.06 (s, 3H), 0.93 (s, 9H). m/z 1087.48 [M+H]⁺ |
| 51 | ¹H NMR (400 MHz, CDCl₃) δ 8.68 (s, 1H), 8.26 (d, J = 3.5 Hz, 1H), 8.19 (d, J = 4.0 Hz, 1H), 7.33-7.31 (m, 2H), 7.20-7.14 (m, 2H), 7.04-6.92 (m, 2H), 6.88 (d, J = 7.4 Hz, 1H), 6.66 (s, 1H), 4.74- 4.73 (m, 2H), 4.59-4.38 (m, 4H), 4.02-3.93 (m, 3H), 3.67-3.04 (m, 12H), 2.69-2.37 (m, 8H), 2.10-2.02 (m, 3H), 1.58-1.45 (m, 8H), 1.38-1.23 (m, 4H), 1.04 (s, 3H), 0.93 (s, 9H). m/z 1101.51 [M+H]⁺ |
| 52 | ¹H NMR (400 MHz, CDCl₃) δ 10.62 (s, 1H), 8.69 (s, 1H), 8.20-8.13 (m, 3H), 7.35-7.30 (m, 2H), 7.11 (t, J = 8.1 Hz, 1H), 7.01 (dd, J = 9.0, 2.4 Hz, 1H), 6.98 (dd, J = 7.7, 1.5 Hz, 1H), 6.88 (d, J = 1.2 Hz, 1H), 6.79 (dd, J = 7.9, 1.3 Hz, 1H), 4.69 (t, J = 7.7 Hz, 1H), 4.53-4.23 (m, 5H), 4.18 (t, J = 5.3 Hz, 2H), 3.98 (d, J = 11.3 Hz, 1H), 3.94-3.88 (m, 2H), 3.75-3.71 (m, 2H), 3.64-3.57 (m, 4H), 3.51 (s, 2H), 3.41-3.34 (m, 2H), 3.07-3.04 (m, 1H), 2.99-2.90 (m, 2H), 2.71-2.66 (m, 2H), 2.65-2.63 (m, 2H), 2.60-2.57 (m, 3H), 2.53 (s, 3H), 2.10-2.04 (m, 1H), 1.59-1.52 (m, 2H), 1.48-1.42 (m, 2H), 1.34-1.31 (m, 4H), 1.03 (s, 3H), 0.92 (s, 9H). m/z 1076.47 [M+H]⁺ |
| 53 | ¹H NMR (400 MHz, CDCl₃) δ 10.61 (s, 1H), 8.69 (s, 1H), 8.27-8.12 (m, 3H), 7.35-7.31 (m, 2H), 7.12 (t, J = 8.1 Hz, 1H), 7.06-6.94 (m, 2H), 6.88 (s, 1H), 6.80 (dd, J = 7.9, 1.2 Hz, 1H), 4.69 (t, J = 7.8 Hz, 1H), 4.59-4.46 (m, 3H), 4.44-4.39 (m, 1H), 4.18 (t, J = 5.5 Hz, 2H), 3.99 (d, J = 11.2 Hz, 1H), 3.81-3.69 (m, 4H), 3.69-3.54 (m, 5H), 3.51 (s, 2H), 3.03-2.86 (m, 2H), 2.74-2.59 (m, 4H), 2.59-2.48 (m, 4H), 2.12-2.00 (m, 1H), 1.60-1.47 (m, 4H), 1.38-1.24 (m, 4H), 1.22 (s, 3H), 0.92 (s, 9H). m/z 1062.49 [M+H]⁺ |
| 54 | ¹H NMR (400 MHz, CDCl₃) δ 10.54 (s, 1H), 8.69 (s, 1H), 8.11 (dd, J = 8.2, 1.2 Hz, 1H), 7.63 (s, 1H), 7.35-7.30 (m, 2H), 7.07 (t, J = 8.1 Hz, 1H), 7.03-6.97 (m, 2H), 6.88 (d, J = 1.3 Hz, 1H), 6.59 (dd, J = 8.0, 1.4 Hz, 1H), 4.84 (s, 2H), 4.69 (t, J = 7.7 Hz, 1H), 4.59-4.45 (m, 3H), 4.44-4.39 (m, 1H), 4.18 (t, J = 5.2 Hz, 2H), 3.99 (d, J = 11.4 Hz, 1H), 3.76-3.67 (m, 4H), 3.65-3.55 (m, 5H), 3.52 (s, 2H), 3.02-2.88 (m, 2H), 2.75-2.59 (m, 4H), 2.59-2.49 (m, 4H), 2.09-2.02 (m, 1H), 1.66-1.50 (m, 4H), 1.38-1.23 (m, 4H), 1.21 (s, 3H), 0.92 (s, 9H). m/z 1077.53 [M+H]⁺ |
| 55 | ¹H NMR (400 MHz, CDCl₃) δ 10.53 (s, 1H), 8.69 (s, 1H), 8.11 (dd, J = 8.2, 1.2 Hz, 1H), 7.62 (s, 1H), 7.35-7.31 (m, 2H), 7.13-6.95 (m, 3H), 6.88 (d, *J* = 1.4 Hz, 1H), 6.59 (dd, J = 8.0, 1.4 Hz, 1H), 4.84 (s, 2H), 4.68 (t, *J* = 7.7 Hz, 1H), 4.57-4.46 (m, 3H), 4.44-4.39 (m, 1H), 4.18 (t, *J* = 5.4 Hz, 2H), 3.98 (d, *J* = 11.4 Hz, 1H), 3.88 (dt, J = 13.3, 4.8 Hz, 2H), 3.80-3.68 (m, 2H), 3.66-3.56 (m, 3H), 3.52 (s, 2H), 3.33 (ddd, J = 13.5, 10.1, 3.6 Hz, 2H), 3.00-2.88 (m, 2H), 2.74-2.61 (m, 4H), 2.58 (s, 2H), 2.56-2.49 (m, 4H), 2.11-2.03 (m, 1H), 1.56-1.40 (m, 4H), 1.37-1.22 (m, 4H), 1.02 (s, 3H), 0.92 (s, 9H). m/z 1091.56 [M+H]⁺ |
| 56 | ¹H NMR (400 MHz, CDCl₃) δ 10.71 (s, 1H), 8.68 (s, 1H), 8.23-8.11 (m, 3H), 7.38 (d, J = 7.7 Hz, 1H), 7.34 (t, J = 5.8 Hz, 1H), 7.12 (t, J = 8.1 Hz, 1H), 6.98 (dd, J = 7.7, 1.5 Hz, 2H), 6.88 (d, J = 1.3 Hz, 1H), 6.82 (dd, J = 7.9, 1.3 Hz, 1H), 4.67 (t, J = 7.9 Hz, 1H), 4.48-4.38 (m, 4H), 4.23-4.09 (m, 3H), 3.93-3.88 (m, 2H), 3.83-3.67 (m, 4H), 3.67-3.51 (m, 4H), 3.40-3.34 (m, 2H), 2.94-2.92 (m, 2H), 2.78 (s, 1H), 2.73-2.64 (m, 2H), 2.62 (s, 2H), 2.53 (s, 3H), 2.52-2.44 (m, 1H), 2.10-2.06 (m, 1H), 1.61-1.53 (m, 2H), 1.50-1.43 (m, 2H), 1.05 (s, 3H), 0.95 (s, 9H), 0.92-0.82 (m, 2H), 0.71-0.67 (m, 2H). m/z 1058.57 [M+H]⁺ |
| 57 | ¹H NMR (400 MHz, CDCl₃) δ 10.72 (s, 1H), 8.69 (s, 1H), 8.21-8.10 (m, 3H), 7.38 (d, J = 7.7 Hz, 1H), 7.23 (dd, J = 5.5, 3.0 Hz, 1H), 7.12 (td, J = 8.1, 5.3 Hz, 1H), 6.99 (dd, J = 7.7, 1.6 Hz, 1H), 6.91-6.73 (m, 3H), 4.66 (t, J = 7.9 Hz, 1H), 4.51-4.38 (m, 4H), 4.25-4.12 (m, 3H), 3.91 (dt, J = 13.2, 4.8 Hz, 2H), 3.85-3.65 (m, 3H), 3.65-3.53 (m, 4H), 3.47-3.33 (m, 2H), 2.98-2.88 (m, 2H), 2.85-2.74 (m, 1H), 2.70-2.64 (m, 3H), 2.59 (s, 2H), 2.56-2.46 (m, 4H), 2.10-1.95 (m, 4H), 1.63-1.39 (m, 4H), 1.03 (s, 3H), 0.94 (s, 9H). m/z 1032.47 [M+H]⁺ |
| 58 | ¹H NMR (400 MHz, CDCl₃) δ 10.03 (d, J = 17.0 Hz, 1H), 8.68 (s, 1H), 8.19-8.11 (m, 3H), 7.36-7.28 (m, 2H), 7.10 (t, J = 7.7 Hz, 1H), 7.06-6.95 (m, 2H), 6.88 (d, J = 1.4 Hz, 1H), 6.76 (dd, J = 8.0, 1.4 Hz, 1H), 4.69 (dd, J = 13.5, 7.7 Hz, 1H), 4.60-4.33 (m, 4H), 4.18 (m, 2H), 4.02-3.77 (m, 4H), 3.73-3.59 (m, 5H), 3.44-3.37 (m, 2H), 2.98-2.88 (m, 2H), 2.71-2.49 (m, 10H), 2.06-2.04 (m, 1H), 1.70-1.43 (m, 7H), 1.37-1.22 (m, 4H), 1.07 (s, 3H), 0.92 (s, 9H). m/z 1090.53 [M+H]⁺ |
| 59 | ¹H NMR (400 MHz, CDCl₃) δ 8.67 (s, 1H), 8.20-8.13 (m, 2H), 8.05 (dd, J = 8.2, 1.4 Hz, 1H), 7.72 (s, 1H), 7.32 (d, J = 7.7 Hz, 1H), 7.30-7.27 (m, 1H), 7.13 (t, J = 8.1 Hz, 1H), 7.01 (dd, J = 8.7, 3.2 Hz, 1H), 6.96 (dd, J = 7.7, 1.6 Hz, 1H), 6.86 (d, J = 1.6 Hz, 1H), 6.74 (dd, J = 8.0, 1.4 Hz, 1H), 4.66 (t, J = 7.7 Hz, 1H), 4.57-4.44 (m, 3H), 4.36-4.31 (m, 1H), 4.17-4.06 (m, 2H), 3.96-3.89 (m, 3H), 3.79-3.63 (m, 2H), 3.63-3.35 (m, 5H), 2.86-3.83 (m, 2H), 2.66-2.45 (m, 10H), 2.07-1.97 (m, 1H), 1.61-1.42 (m, 10H), 1.39-1.22 (m, 4H), 1.03 (s, 3H), 0.90 (s, 9H). m/z 1104.55 [M+H]⁺ |
| 60 | ¹H NMR (400 MHz, CDCl₃) δ 10.61 (s, 1H), 8.68 (s, 1H), 8.22-8.14 (m, 3H), 7.33 (d, J = 7.7 Hz, 1H), 7.30 (t, J = 6.0 Hz, 1H), 7.12 (dd, J = 10.1, 6.1 Hz, 1H), 7.00-6.98 (m, 1H), 6.97-6.95 (m, 1H), 6.88 (d, J = 1.5 Hz, 1H), 6.79 (dd, J = 7.9, 1.4 Hz, 1H), 4.74 (t, J = 7.7 Hz, 1H), 4.55-4.49 (m, 4H), 4.40 (dd, J = 14.7, 5.1 Hz, 1H), 4.11-4.06 (m, 2H), 4.01 (d, J = 11.4 Hz, 1H), 3.94-3.88 (m, 2H), 3.73-3.70 (m, 2H), 3.62-3.59 (m, 1H), 3.59-3.56 (m, 2H), 3.51 (s, 2H), 3.41-3.34 (m, 2H), 2.63-2.59 (m, 2H), 2.58-2.56 (m, 2H), 2.53 (s, 3H), 2.51-2.49 (m, 1H), 2.09-2.02 (m, 4H), 1.59-1.52 (m, 2H), 1.48-1.43 (m, 2H), 1.34-1.29 (m, 6H), 1.03 (s, 3H), 0.92 (s, 9H). m/z 1090.48 [M+H]⁺ |
| 61 | ¹H NMR (400 MHz, CDCl₃) δ 10.56 (s, 1H), 8.68 (s, 1H), 8.20-8.14 (m, 3H), 7.33 (d, J = 7.7 Hz, 1H), 7.19 (t, J = 5.8 Hz, 1H), 7.12 (t, J = 8.1 Hz, 1H), 6.96 (d, J = 7.7 Hz, 1H), 6.87 (s, 1H), 6.79 (d, J = 8.0 Hz, 1H), 6.30 (d, J = 8.7 Hz, 1H), 4.69 (t, J = 7.8 Hz, 1H), 4.52-4.40 (m, 4H), 4.09-4.06 (m, 3H), 3.93-3.88 (m, 2H) 3.72 (s, 2H), 3.64-3.55 (m, 3H), 3.53 (s, 2H), 3.44-3.32 (m, 2H), 2.64-2.56 (m, 3H), 2.52-2.47 (m, 9H), 2.15-2.02 (m, 2H), 2.02-2.00 (m, 4H), 1.60-1.50 (m, 2H), 1.48-1.43 (m, 2H), 1.03 (s, 3H), 0.91 (s, 9H). m/z 1046.58 [M+H]⁺ |
| 62 | ¹H NMR (400 MHz, CDCl₃) δ 10.63 (d, J = 26.7 Hz, 1H), 8.69 (s, 1H), 8.21-8.14 (m, 3H), 7.34 (d, J = 7.7 Hz, 1H), 7.30-7.29 (m, 1H), 7.12 (t, J = 8.1 Hz, 1H), 7.04-6.97 (m, 2H), 6.88 (d, J = 1.4 Hz, 1H), 6.80 (d, J = 7.8 Hz, 1H), 4.83 (s, 1H), 4.72-4.67 (m, 1H), 4.54-4.46 (m, 4H), 4.42-4.36 (m, 1H), 4.16 (t, J = 5.3 Hz, 2H), 4.13-4.10 (m, 1H), 3.99 (d, J = 11.5 Hz, 1H), 3.91-3.88 (m, 2H), 3.70-3.59 (m, 2H), 3.55-3.46 (m, 2H), 3.43-3.34 (m, 2H), 3.07-2.99 (m, 2H), 2.94-2.81 (m, 4H), 2.59 (s, 2H), 2.53 (s, 4H), 2.46-2.36 (m, 1H), 2.24-2.17 (m, 2H), 2.07 (t, J = 10.6 Hz, 2H), 1.59-1.53 (m, 4H), 1.48-1.44 (m, 3H), 1.39-1.37 (m, 3H), 1.35-1.29 (m, 4H), 1.03 (s, 3H), 0.91-0.90 (m, 9H). m/z 1090.48 [M+H]⁺ |
| 63 | ¹H NMR (400 MHz, CDCl₃) δ 10.63 (d, J = 11.4 Hz, 1H), 8.69 (s, 1H), 8.24-8.10 (m, 3H), 7.36-7.29 (m, 2H), 7.12 (dd, J = 11.4, 4.9 Hz, 1H), 7.01-6.97 (m, 2H), 6.88 (d, J = 1.6 Hz, 1H), 6.84-6.78 (m, 1H), 4.76-4.65 (m, 1H), 4.59-4.37 (m, 5H), 4.24-4.06 (m, 3H), 3.99 (d, J = 11.9 Hz, 1H), 3.94-3.88 (m, 2H), 3.73-3.31 (m, 6H), 3.04 (t, J = 12.1 Hz, 2H), 2.93-2.76 (m, 4H), 2.64-2.50 (m, 6H), 2.13-2.00 (m, 1H), 1.62-1.42 (m, 4H), 1.38 (d, J = 6.6 Hz, 3H), 1.36-1.21 (m, 4H), 1.04 (s, 3H), 0.93 (d, J = 2.6 Hz, 9H). m/z 1090.50 [M+H]⁺ |
| 64 | ¹H NMR (400 MHz, CDCl₃) δ 10.68-10.62 (m, 1H), 8.69 (s, 1H), 8.22-8.14 (m, 3H), 7.33 (d, J = 7.8 Hz, 1H), 7.30-7.28 (m, 1H), 7.12 (t, J = 8.1 Hz, 1H), 7.00-6.97 (m, 1H), 6.96-6.94 (m, 1H), 6.88 (s, 1H), 6.79 (d, J = 7.6 Hz, 1H), 4.74 (t, J = 7.6 Hz, 1H), 4.54-4.49 (m, 3H), 4.42 (dd, J = 14.8, 5.2 Hz, 1H), 4.13-4.09 (m, 3H), 4.02 (d, J = 11.3 Hz, 1H), 3.94-3.88 (m, 2H), 3.61 (dd, J = 11.5, 3.9 Hz, 1H), 3.54-3.49 (m, 1H), 3.47-3.43 (m, 1H), 3.40-3.34 (m, 3H), 2.92-2.90 (m, 1H), 2.79 (t, J = 11.7 Hz, 1H), 2.63-2.57 (m, 4H), 2.52 (s, 4H), 2.23-2.13 (m, 1H), 2.10-1.98 (m, 5H), 1.59-1.52 (m, 4H), 1.47-1.44 (m, 3H), 1.37 (d, J = 6.7 Hz, 2H), 1.33-1.31 (m, 3H), 1.29-1.23 (m, 4H), 1.03 (s, 3H), 0.93 (s, 9H). m/z 1104.43 [M+H]⁺ |
| 65 | ¹H NMR (400 MHz, CDCl₃) δ 10.67-10.62 (m, 1H), 8.68 (s, 1H), 8.23-8.11 (m, 3H), 7.35-7.29 (m, 2H), 7.12 (t, J = 8.1 Hz, 1H), 7.00-6.94 (m, 2H), 6.88-6.86 (m, 1H), 6.81 (d, J = 7.9 Hz, 1H), 4.75 (t, J = 7.7 Hz, 1H), 4.55-4.48 (m, 4H), 4.45-4.38 (m, 1H), 4.11-4.07 (m, 3H), 4.03-4.01 (m, 1H), 3.94-3.88 (m, 2H), 3.68-3.64 (m, 1H), 3.61 (dd, J = 11.2, 3.7 Hz, 1H), 3.56-3.54 (m, 1H), 3.49-3.43 (m, 2H), 3.41-3.34 (m, 2H), 2.96-2.89 (m, 1H), 2.79 (t, J = 10.3 Hz, 1H), 2.63-2.57 (m, 3H), 2.53-2.48 (m, 4H), 2.27-2.20 (m, 1H), 2.15-2.07 (m, 2H), 2.06-2.00 (m, 3H), 1.59-1.53 (m, 4H), 1.49-1.45 (m, 2H), 1.37-1.35 (m, 3H), 1.29-1.27 (m, 4H), 1.04 (s, 3H), 0.92 (s, 9H). m/z 1104.43 [M+H]⁺ |
| 66 | ¹H NMR (400 MHz, CDCl₃) δ 10.52 (d, J = 59.1 Hz, 1H), 8.69 (s, 1H), 8.24-8.09 (m, 3H), 7.38 (t, J = 6.6 Hz, 1H), 7.35 (d, J = 7.7 Hz, 1H), 7.12 (t, J = 8.1 Hz, 1H), 7.03 (d, J = 7.6 Hz, 1H), 6.97 (d, J = 7.8 Hz, 1H), 6.87 (d, J = 2.0 Hz, 1H), 6.81 (dd, J = 7.8, 6.7 Hz, 1H), 4.73-4.67 (m, 1H), 4.53-4.47 (m, 3H), 4.44-4.34 (m, 1H), 4.26-4.20 (m, 1H), 4.17-4.11 (m, 2H), 4.08-4.07 (m, 1H), 3.99 (d, J = 10.4 Hz, 1H), 3.93-3.88 (m, 2H), 3.74 (s, 2H), 3.73-3.65 (m, 3H), 3.64-3.58 (m, 2H), 3.52 (s, 3H), 3.40-3.34 (m, 2H), 3.29-3.01 (m, 3H), 2.77-2.70 (m, 1H), 2.62 (s, 2H), 2.55-2.46 (m, 4H), 2.24-2.17 (m, 1H), 1.57-1.41 (m, 4H), 1.33-1.28 (m, 4H), 1.05 (s, 3H), 0.92 (s, 9H). m/z 1134.57 [M+H]⁺ |
| 67 | ¹H NMR (400 MHz, CDCl₃) δ 10.52 (d, J = 73.0 Hz, 1H), 8.68 (s, 1H), 8.25-8.11 (m, 3H), 7.37-7.34 (m, 2H), 7.12 (t, J = 8.2 Hz, 1H), 7.05-6.95 (m, 2H), 6.87 (dd, J = 4.3, 1.4 Hz, 1H), 6.81-6.78 (m, 1H), 4.73-4.68 (m, 1H), 4.53-4.48 (m, 3H), 4.40-4.34 (m, 1H), 4.32-4.08 (m, 4H), 4.03-3.86 (m, 3H), 3.77-3.66 (m, 4H), 3.64-3.55 (m, 2H), 3.52 (d, J = 3.7 Hz, 2H), 3.41-3.34 (m, 2H), 3.25-3.00 (m, 4H), 2.81-2.68 (m, 1H), 2.61-2.50 (m, 6H), 2.12-2.03 (m,1H), 1.59-1.43 (m, 4H), 1.38-1.23 (m, 4H), 1.03 (s, 3H), 0.90 (s, 9H). m/z 1134.57 [M+H]⁺ |
| 68 | ¹H NMR (400 MHz, CDCl₃) δ 10.82 (d, J = 38.9 Hz, 1H), 8.67 (s, 1H), 8.23-8.09 (m,3 H), 7.33-7.30 (m, 2H), 7.10 (td, J = 8.1, 2.5 Hz, 1H), 7.06-6.97 (m, 1H), 6.95 (dt, J = 7.7, 1.6 Hz, 1H), 6.84 (d, J = 1.5 Hz, 1H), 6.79 (dd, J = 7.9, 1.2 Hz, 1H), 4.78-4.64 (m, 2H), 4.58-4.44 (m, 3H), 4.43-4.32 (m, 1H), 4.23-4.21 (m, 1H), 4.12 (t, J = 4.2 Hz, 2H), 4.00-3.83 (m, 3H), 3.66-3.56 (m, 1H), 3.50-3.44 (m, 3H), 3.36 (ddd, J = 13.5, 10.1, 3.5 Hz, 2H), 3.01-2.76 (m, 4H), 2.60 (s, 2H), 2.58-2.42 (m, 6H), 2.16-1.89 (m, 5H), 1.62-1.38 (m, 4H), 1.37-1.21 (m, 4H), 1.03 (s, 3H), 0.91 (d, J = 10.9 Hz, 9H). m/z 1102.43 [M+H]⁺ |
| 69 | ¹H NMR (400 MHz, DMSO-d₆) δ 10.14 (d, J = 14.5 Hz, 1H), 9.03 (s, 1H), 8.62-8.58 (m, 1H), 8.44 (d, J = 1.1 Hz, 1H), 8.25 (d, J = 1.3 Hz, 1H), 8.01 (s, 3H), 7.65 (d, J = 7.7 Hz, 1H), 7.44 (d, J = 7.9 Hz, 1H), 7.29 (d, J = 9.3 Hz, 1H), 7.21 (t, J = 8.1 Hz, 1H), 7.08-6.99 (m, 2H), 6.72-6.64 (m, 1H), 4.64-4.43 (m, 5H), 4.42-4.24 (m, 5H), 3.94-3.58 (m, 11H), 3.49-3.41 (m, 3H), 2.78-2.75 (m, 2H), 2.47 (s, 3H), 2.11 (dd, J = 11.7, 6.4 Hz, 1H), 1.97-1.85 (m, 1H), 1.59-1.32 (m, 6H), 1.23-1.20 (m, 2H), 1.08 (s, 3H), 0.94 (s, 9H). m/z 1120.54 [M+H]⁺ |
| 70 | ¹H NMR (400 MHz, DMSO-d₆) δ 10.14 (d, J = 18.2 Hz, 1H), 9.02 (s, 1H), 8.65-8.55 (m, 1H), 8.44 (s, 1H), 8.25 (d, J = 1.1 Hz, 1H), 7.95 (s, 3H), 7.66 (d, J = 7.8 Hz, 1H), 7.50-7.43 (m, 1H), 7.30 (d, J = 9.0 Hz, 1H), 7.20 (t, J = 8.1 Hz, 1H), 7.08-6.98 (m, 2H), 6.68 (d, J = 8.1 Hz, 1H), 4.65-4.18 (m, 10H), 3.75-3.32 (m, 14H), 2.78-2.74 (m, 2H), 2.46 (s, 3H), 2.13-2.05 (m, 1H), 1.96-1.86 (m, 1H), 1.61-1.20 (m, 8H), 1.08 (s, 3H), 0.94 (s, 9H). m/z 1120.49 [M+H]⁺ |
| 71 | ¹H NMR (600 MHz, CDCl₃) δ 10.17 (s, 1H), 8.68 (s, 1H), 8.35-8.32 (m, 1H), 8.18 (d, J = 1.4 Hz, 1H), 8.14 (d, J = 1.4 Hz, 1H), 7.33 (d, J = 7.7 Hz, 1H), 7.31-7.27 (m, 1H), 7.15-7.11 (m, 1H), 7.00-6.95 (m, 2H), 6.87 (d, J = 1.5 Hz, 1H), 6.77 (dd, J = 7.9, 1.4 Hz, 1H), 4.76 (t, J = 7.6 Hz, 1H), 4.58-4.49 (m, 3H), 4.43-4.40 (m, 1H), 4.04-3.99 (m, 3H), 3.93-3.89 (m, 2H), 3.62 (dd, J = 11.2, 4.0 Hz, 1H), 3.43-3.36 (m, 2H), 3.14 (s, 2H), 2.94 (d, J = 11.1 Hz, 2H), 2.66-2.56 (m, 3H), 2.54 (s, 3H), 2.30 (t, J = 10.1 Hz, 2H), 2.10-2.05 (m, 1H), 1.93-1.88 (m, 2H), 1.83-1.81 (m, 2H), 1.58-1.25 (m, 13H), 1.03 (s, 3H), 0.92 (s, 9H). m/z 1061.46 [M+H]⁺ |
| 72 | ¹H NMR (400 MHz, CDCl₃) δ 10.04 (s, 1H), 8.68 (s, 1H), 8.31 (dd, J = 8.2, 1.4 Hz, 1H), 8.25-8.10 (m, 2H), 7.38-7.29 (m, 2H), 7.13 (td, J = 8.1, 3.2 Hz, 1H), 7.04-6.94 (m, 2H), 6.90 (d, J = 1.4 Hz, 1H), 6.73 (dd, J = 8.0, 1.4 Hz, 1H), 4.73 (t, J = 7.7 Hz, 1H), 4.61-4.36 (m, 4H), 4.24-4.09 (m, 2H), 4.02-3.85 (m, 3H), 3.63 (dd, J = 11.2, 3.9 Hz, 1H), 3.48-3.34 (m, 2H), 3.18 (s, 2H), 3.02-2.83 (m, 2H), 2.71 (s, 8H), 2.62-2.47 (m, 6H), 2.13-2.05 (m, 1H), 1.68-1.41 (m, 4H), 1.35-1.23 (m, 4H), 1.06 (s, 3H), 0.92 (s, 9H). m/z 1048.61 [M+H]⁺ |
| 73 | ¹H NMR (400 MHz, CDCl₃) δ 10.08 (s, 1H), 8.69 (s, 1H), 8.33 (dd, J = 8.2, 1.4 Hz, 1H), 8.22-8.11 (m, 2H), 7.34-7.29 (m, 2H), 7.14 (t, J = 8.1 Hz, 1H), 7.00-6.95 (m, 2H), 6.89 (d, J = 1.5 Hz, 1H), 6.77 (dd, J = 7.9, 1.4 Hz, 1H), 4.75 (t, J = 7.6 Hz, 1H), 4.59-4.37 (m, 4H), 4.16-4.04 (m, 2H), 3.99 (d, J = 11.3 Hz, 1H), 3.91 (dt, J = 13.3, 4.7 Hz, 2H), 3.62 (dd, J = 11.2, 4.0 Hz, 1H), 3.38 (ddd, J = 13.5, 10.0, 3.6 Hz, 2H), 3.18 (s, 2H), 2.78-2.56 (m, 13H), 2.53 (s, 3H), 2.10-2.05 (m, 3H), 1.59-1.39 (m, 4H), 1.35-1.23 (m, 4H), 1.03 (s, 3H), 0.91 (s, 9H). m/z 1062.63 [M+H]⁺ |
| 74 | ¹H NMR (600 MHz, CDCl₃) δ 10.94 (s, 1H), 8.68 (s, 1H), 8.21-8.13 (m, 3H), 7.32 (d, J = 7.7 Hz, 1H), 7.29-7.26 (m, 1H), 7.11 (t, J = 8.1 Hz, 1H), 6.99 (dd, J = 8.6, 3.3 Hz, 1H), 6.95 (dd, J = 7.7, 1.6 Hz, 1H), 6.88 (d, J = 1.5 Hz, 1H), 6.70 (dd, J = 7.9, 1.4 Hz, 1H), 4.74 (t, J = 7.7 Hz, 1H), 4.58-4.46 (m, 3H), 4.43-4.40 (m, 1H), 4.13-4.03 (m, 2H), 3.99 (d, J = 11.4 Hz, 1H), 3.91 (dt, J = 13.4, 4.8 Hz, 2H), 3.61 (dd, J = 11.2, 4.0 Hz, 1H), 3.39 (ddd, J = 13.4, 10.1, 3.3 Hz, 2H), 3.11 (d, J = 10.5 Hz, 2H), 2.72-2.63 (m, 2H), 2.63-2.56 (m, 5H), 2.53 (s, 3H), 2.09-2.02 (m, 3H), 1.87-1.82 (m, 4H), 1.68-1.44 (m, 7H), 1.38-1.22 (m, 4H), 1.03 (s, 3H), 0.92 (s, 9H). m/z 1061.41 [M+H]⁺ |
| 75 | ¹H NMR (600 MHz, CDCl₃) δ 10.96 (s, 1H), 8.68 (s, 1H), 8.22-8.13 (m, 3H), 7.34 (d, J = 7.7 Hz, 1H), 7.29-7.26 (m, 1H), 7.13-7.08 (m, 1H), 7.03-6.98 (m, 1H), 6.95 (dd, J = 7.7, 1.6 Hz, 1H), 6.87 (d, J = 1.5 Hz, 1H), 6.68 (dd, J = 8.0, 1.3 Hz, 1H), 4.74 (t, J = 7.6 Hz, 1H), 4.57-4.51 (m, 3H), 4.42-4.39 (m, 1H), 4.03-3.97 (m, 3H), 3.93-3.89 (m, 2H), 3.61 (dd, J = 11.1, 4.1 Hz, 1H), 3.42-3.34 (m, 2H), 3.12-3.07 (m, 2H), 2.68 (m, 2H), 2.63-2.55 (m, 5H), 2.53 (s, 3H), 2.10-1.97 (m, 3H), 1.90-1.87 (m, 2H), 1.85-1.77 (m, 2H), 1.63-1.38 (m, 9H), 1.37-1.20 (m, 4H), 1.03 (s, 3H), 0.92 (s, 9H). m/z 1075.42 [M+H]⁺ |
| 76 | ¹H NMR (400 MHz, CDCl₃) δ 8.68-8.66 (m, 2H), 8.20-8.08 (m, 3H), 7.41-7.33 (m, 2H), 7.11 (t, J = 8.1 Hz, 1H), 7.04 (dd, J = 8.7, 3.2 Hz, 1H), 7.01-6.96 (m, 1H), 6.88 (d, J = 1.2 Hz, 1H), 6.80-6.74 (m, 1H), 4.57-4.41 (m, 5H), 4.36 (d, J = 15.4 Hz, 1H), 4.22-4.11 (m, 2H), 4.01-3.87 (m, 4H), 3.65-3.61 (m, 2H), 3.57-3.33 (m, 5H), 3.09-2.86 (m, 4H), 2.58 (s, 2H), 2.53 (s, 3H), 2.46-2.37 (m, 1H), 2.07-1.99 (m, 1H), 1.60-1.50 (m, 2H), 1.48-1.42 (m, 2H), 1.36-1.23 (m, 4H), 1.03 (s, 3H), 0.95 (s, 9H). m/z 1062.56 [M+H]⁺ |
| 77 | ¹H NMR (400 MHz, CDCl₃) δ 8.71 (s, 1H), 8.68 (s, 1H), 8.17-8.14 (m, 3H), 7.33 (d, J = 7.7 Hz, 1H), 7.30 (s, 1H), 7.12 (t, J = 8.1 Hz, 1H), 7.07 (d, J = 5.8 Hz, 1H), 6.96 (d, J = 7.5 Hz, 1H), 6.88 (s, 1H), 6.78 (d, J = 7.9 Hz, 1H), 4.72 (t, J = 7.6 Hz, 1H), 4.53-4.49 (m, 3H), 4.41 (dd, J = 14.8, 5.2 Hz, 1H), 4.23-4.15 (m, 2H), 4.12-4.07 (m, 2H), 3.99 (d, J = 11.4 Hz, 1H), 3.95-3.86 (m, 2H), 3.61 (dd, J = 11.3, 3.6 Hz, 1H), 3.53-3.51 (m, 2H), 3.41-3.35 (m, 2H), 3.33-3.28 (m, 2H), 2.83 (t, J = 5.3 Hz, 2H), 2.71 (t, J = 6.9 Hz, 2H), 2.58 (s, 2H), 2.57-2.55 (m, 1H), 2.52 (s, 3H), 2.10-2.04 (s, 3H), 1.60-1.52 (m, 2H), 1.48-1.42 (m, 2H), 1.33-1.60 (m, 4H), 1.03 (s, 3H), 0.91 (s, 3H). m/z 1076.51 [M+H]⁺ |
| 78 | ¹H NMR (400 MHz, CDCl₃) δ 8.72 (s, 1H), 8.68 (s, 1H), 8.20-8.11 (m, 3H), 7.33 (d, J = 7.8 Hz, 1H), 7.12 (t, J = 8.1 Hz, 1H), 7.03 (dd, J = 8.6, 3.1 Hz, 1H), 6.96 (dd, J = 7.7, 1.4 Hz, 1H), 6.87 (d, J = 0.9 Hz, 1H), 6.78 (dd, J = 7.9, 1.1 Hz, 1H), 4.71 (t, J = 7.7 Hz, 1H), 4.56-4.51 (m, 3H), 4.41 (dd, J = 14.7, 5.3 Hz, 1H), 4.23-4.14 (m, 1H), 4.05 (t, J = 6.1 Hz, 2H), 3.97 (d, J = 11.6 Hz, 1H), 3.93-3.88 (m, 2H), 3.61 (dd, J = 11.2, 3.9 Hz, 1H), 3.50 (t, J = 5.3 Hz, 2H), 3.43-3.33 (m, 2H), 3.28 (d, J = 2.7 Hz, 2H), 2.83-2.74 (m, 2H), 2.58 (s, 2H), 2.57-2.55 (m, 1H), 2.53 (s, 3H), 2.09-2.00 (m, 2H), 1.95-1.89 (m, 3H), 1.80-1.73 (m, 2H), 1.59-1.52 (m, 2H), 1.48-1.43 (m, 2H), 1.33-1.28 (m, 4H), 1.03 (s, 3H), 0.92 (s, 9H). m/z 1090.54 [M+H]⁺ |
| 79 | ¹H NMR (400 MHz, CDCl₃) δ 8.69 (s, 1H), 8.65 (s, 1H), 8.19 (d, J = 1.3 Hz, 1H), 8.14 (d, J = 1.3 Hz, 1H), 8.03 (d, J = 7.5 Hz, 1H), 7.54 (t, J = 6.1 Hz, 1H), 7.36 (d, J = 7.7 Hz, 1H), 7.11 (t, J = 8.1 Hz, 1H), 6.97 (dd, J = 7.7, 1.4 Hz, 1H), 6.87 (d, J = 1.4 Hz, 1H), 6.79-6.72 (m, 2H), 4.53-4.47 (m, 4H), 4.44-4.31 (m, 2H), 4.20-4.11 (m, 2H), 4.02 (d, J = 15.5 Hz, 1H), 3.98-3.86 (m, 3H), 3.65-3.50 (m, 4H), 3.46-3.32 (m, 3H), 3.11-2.92 (m, 4H), 2.59 (s, 2H), 2.53 (s, 3H), 2.51-2.43 (m, 1H), 2.01-1.87 (m, 1H), 1.66-1.43 (m, 5H), 1.03 (s, 3H), 0.94-0.80 (m, 11H), 0.79-0.64 (m, 2H). m/z 1044.50 [M+H]⁺ |
| 80 | ¹H NMR (400 MHz, CDCl₃) δ 8.68 (s, 1H), 8.60 (s, 1H), 8.20-8.10 (m, 3H), 7.76 (m, 1H), 7.52 (d, J = 8.0 Hz, 1H), 7.32 (d, J = 7.6 Hz, 1H), 7.15-7.09 (m, 1H), 6.93 (dd, J = 7.6, 1.3 Hz, 1H), 6.87 (s, 1H), 6.81-6.74 (m, 1H), 4.76 (t, J = 7.8 Hz, 1H), 4.57-4.23 (6H), 4.01-3.89 (m, 5H), 3.66-3.32 (m, 5H), 3.21-3.05 (m, 2H), 2.99-2.96 (m, 1H), 2.92-2.78 (m, 1H), 2.75-2.56 (m, 5H), 2.52 (s, 3H), 2.27-2.18 (m, 1H), 2.11-2.03 (m, 2H), 1.81-1.63 (m, 3H), 1.62-1.39 (m, 2H), 1.06 (s, 3H), 0.93-0.80 (m, 2H), 0.76 (s, 9H), 0.67-0.65 (m, 2H). m/z 1058.53 [M+H]⁺ |
| 81 | ¹H NMR (400 MHz, CDCl₃) δ 10.58 (s, 1H), 8.69 (s, 1H), 8.52 (dd, J = 8.3, 1.4 Hz, 1H), 8.27 (d, J = 2.8 Hz, 1H), 8.20 (d, J = 1.3 Hz, 1H), 8.15 (d, J = 1.3 Hz, 1H), 8.11 (d, J = 8.8 Hz, 1H), 7.39-7.34 (m, 2H), 7.26-7.22 (m, 2H), 7.18 (t, J = 8.1 Hz, 1H), 6.99 (dd, J = 7.7, 1.6 Hz, 2H), 6.91 (d, J = 1.4 Hz, 1H), 6.79 (dd, J = 7.9, 1.4 Hz, 1H), 4.74 (t, J = 7.8 Hz, 1H), 4.59-4.39 (m, 5H), 4.22 (t, J = 4.9 Hz, 2H), 4.02 (d, J = 11.4 Hz, 1H), 3.94-3.88 (m, 2H), 3.61 (dd, J = 11.3, 3.7 Hz, 1H), 3.46-3.39 (m, 4H), 3.39-3.32 (m, 2H), 3.00-2.96 (m, 2H), 2.82-2.80 (m, 4H), 2.61-2.56 (m, 3H), 2.54 (s, 3H), 2.12-2.03 (m, 1H), 1.59-1.52 (m, 4H), 1.48-1.44 (m, 2H), 1.35-1.25 (m, 6H), 1.04 (s, 3H), 0.92 (s, 9H). m/z 1111.46 [M+H]⁺ |
| 82 | ¹H NMR (400 MHz, CDCl₃) δ 8.73 (s, 1H), 8.69 (s, 1H), 8.39 (d, J = 8.3 Hz, 1H), 8.36 (s, 1H), 8.20 (s, 1H), 8.15 (s, 1H), 8.00 (dd, J = 9.0, 2.3 Hz, 1H), 7.35-7.32 (m, 2H), 7.17 (t, J = 8.1 Hz, 1H), 7.01-6.97 (m, 2H), 6.90 (s, 1H), 6.79 (d, J = 7.9 Hz, 1H), 6.68 (d, J = 9.1 Hz, 1H), 4.73 (t, J = 7.7 Hz, 1H), 4.55-4.41 (m, 4H), 4.22 (t, J = 5.4 Hz, 2H), 4.01 (d, J = 11.3 Hz, 1H), 3.94-3.89 (m, 2H), 3.73-3.72 (m, 4H), 3.62 (dd, J = 11.0, 3.6 Hz, 1H), 3.42-3.35 (m, 2H), 2.99-2.92 (m, 2H), 2.73 (t, J = 4.5 Hz, 4H), 2.59 (s, 3H), 2.54 (s, 3H), 2.11-2.02 (m, 1H), 1.59-1.53 (m, 2H), 1.48-1.43 (m, 2H), 1.35-1.25 (m, 4H), 1.03 (s, 3H), 0.93 (s, 9H). m/z 1111.45 [M+H]⁺ |
| 83 | ¹H NMR (400 MHz, CDCl₃) δ 10.50 (s, 1H), 8.69 (s, 1H), 8.57 (dd, J = 8.3, 1.4 Hz, 1H), 8.46 (s, 2H), 8.21 (d, J = 1.3 Hz, 1H), 8.16 (d, J = 1.4 Hz, 1H), 7.39-7.34 (m, 2H), 7.19 (t, J = 8.1 Hz, 1H), 6.99 (dd, J = 7.7, 1.4 Hz, 2H), 6.90 (d, J = 1.4 Hz, 1H), 6.80 (dd, J = 8.0, 1.4 Hz, 1H), 4.73 (t, J = 7.8 Hz, 1H), 4.56-4.49 (m, 4H), 4.44 (dd, J = 14.7, 5.4 Hz, 1H), 4.22 (t, J = 5.3 Hz, 2H), 4.01 (d, J = 11.4 Hz, 1H), 3.95-3.89 (m, 2H), 3.61 (dd, J = 11.3, 3.7 Hz, 1H), 3.48-3.45 (m, 4H), 3.42-3.35 (m, 2H), 3.03-2.94 (m, 2H), 2.83 (t, J = 5.1 Hz, 4H), 2.59 (s, 2H), 2.57-2.51 (m, 4H), 2.11-2.05 (m, 1H), 1.60-1.52 (m, 2H), 1.49-1.42 (m, 2H), 1.32-1.24 (m, 4H), 1.04 (s, 3H), 0.93 (s, 9H). m/z 1112.51 [M+H]⁺ |
| 84 | ¹H NMR (400 MHz, CDCl₃) δ 8.83 (s, 2H), 8.69 (s, 1H), 8.36-8.31 (m, 1H), 8.25-8.14 (m, 3H), 7.35-7.32 (m, 2H), 7.20-7.15 (m, 1H), 7.03-6.96 (m, 2H), 6.91 (s, 1H), 6.81 (dd, J = 8.0, 1.4 Hz, 1H), 4.73 (t, J = 7.7 Hz, 1H), 4.59-4.40 (m, 4H), 4.22 (t, J = 5.6 Hz, 2H), 4.06-3.87 (m, 7H), 3.62 (dd, J = 11.3, 3.8 Hz, 1H), 3.47-3.33 (m, 2H), 3.01-2.93 (m, 2H), 2.75-2.64 (m, 4H), 2.62-2.50 (m, 6H), 2.14-2.05 (m, 1H), 1.58-1.40 (m, 4H), 1.38-1.22 (m, 4H), 1.03 (s, 3H), 0.93 (s, 9H). m/z 1112.48 [M+H]⁺ |
| 85 | ¹H NMR (400 MHz, CDCl₃) δ 10.53 (s, 1H), 8.69 (s, 1H), 8.45 (dd, J = 8.3, 1.4 Hz, 1H), 8.21 (d, J = 1.3 Hz, 1H), 8.16 (d, J = 1.4 Hz, 1H), 8.07 (d, J = 9.6 Hz, 1H), 7.36-7.34 (m, 2H), 7.18 (t, J = 8.1 Hz, 1H), 7.04-6.95 (m, 3H), 6.91 (d, J = 1.5 Hz, 1H), 6.79 (dd, J = 8.0, 1.4 Hz, 1H), 4.73 (t, J = 7.8 Hz, 1H), 4.54-4.41 (m, 4H), 4.22 (t, J = 5.5 Hz, 2H), 4.01 (d, J = 11.4 Hz, 1H), 3.95-3.85 (m, 6H), 3.62 (dd, J = 11.2, 3.8 Hz, 1H), 3.42-3.35 (m, 2H), 3.04-2.89 (m, 2H), 2.78 (t, J = 4.9 Hz, 4H), 2.62-2.50 (m, 6H), 2.15-2.04 (m, 1H), 1.62-1.41 (m, 4H), 1.37-1.23 (m, 4H), 1.03 (s, 3H), 0.93 (s, 9H). m/z 1112.41 [M+H]⁺ |
| 86 | ¹H NMR (400 MHz, CDCl₃) δ 10.69 (s, 1H), 8.69 (s, 1H), 8.59 (d, J = 4.8 Hz, 1H), 8.48 (dd, J = 8.2, 1.3 Hz, 1H), 8.24-8.13 (m, 2H), 7.38-7.32 (m, 3H), 7.19 (t, J = 8.1 Hz, 1H), 7.04-6.96 (m, 2H), 6.91 (d, J = 1.3 Hz, 1H), 6.76 (dd, J = 8.0, 1.3 Hz, 1H), 4.74 (t, J = 7.4 Hz, 1H), 4.61-4.37 (m, 4H), 4.25-4.22 (m, 2H), 4.02-3.88 (m, 7H), 3.64 (dd, J = 11.1, 4.2 Hz, 1H), 3.44-3.33 (m, 2H), 3.08-2.90 (m, 2H), 2.81-2.68 (m, 4H), 2.62-2.49 (m, 6H), 2.08-2.00 (m, 1H), 1.59-1.42 (m, 4H), 1.35-1.23 (m, 4H), 1.03 (s, 3H), 0.91 (s, 9H). m/z 1112.62 [M+H]⁺ |
| 87 | ¹H NMR(400 MHz, CDCl₃) δ 10.49 (d, J = 6.2 Hz, 1H), 8.69 (s, 1H), 8.55 (t, J = 7.4 Hz, 1H), 8.46 (d, J = 3.7 Hz, 2H), 8.20 (s, 1H), 8.15 (s, 1H), 7.41-7.38 (m, 1H), 7.33 (d, J = 7.7 Hz, 1H), 7.22-7.16 (m, 1H), 7.00-6.95 (m, 2H), 6.79 (t, J = 7.2 Hz, 1H), 6.68 (s, 1H), 4.82-4.74 (m, 2H), 4.56-4.46 (m, 4H), 4.05-3.97 (m, 1H), 3.97-3.83 (m, 2H), 3.68-3.61 (m, 2H), 3.47-3.36 (m, 6H), 3.18-3.14 (m, 1H), 2.65 (s, 2H), 2.53-2.49 (m, 6H), 2.27-2.18 (m, 1H), 2.13-2.10 (m, 2H), 2.05-2.03 (m, 2H), 1.89-1.87 (m, 2H), 1.84-1.82 (m, 2H), 1.37-1.31 (m, 4H), 1.05-0.99 (m, 3H), 0.94 (s, 9H). m/z 1123.45 [M+H]⁺ |
| 88 | ¹H NMR (400 MHz, CDCl₃) δ 10.51 (s, 1H), 8.69 (s, 1H), 8.47-8.41 (m, 1H), 8.21 (d, J = 1.3 Hz, 1H), 8.15 (d, J = 1.2 Hz, 1H), 8.04 (d, J = 9.6 Hz, 1H), 7.37-7.33 (m, 2H), 7.20-7.13 (m, 1H), 7.04-6.94 (m, 3H), 6.80 (dd, J = 8.0, 1.3 Hz, 1H), 6.69 (s, 1H), 4.83-4.74 (m, 2H), 4.56-4.43 (m, 4H), 4.03 (d, J = 11.4 Hz, 1H), 3.98-3.67 (m, 6H), 3.62 (dd, J = 11.3, 3.8 Hz, 1H), 3.46-3.34 (m, 2H), 2.70-2.49 (m, 8H), 2.26-2.05 (m, 3H), 1.82-1.42 (m, 8H), 1.36-1.25 (m, 4H), 1.04 (s, 3H), 0.94 (s, 9H). m/z 1123.57 [M+H]⁺ |
| 89 | ¹H NMR (600 MHz, CDCl₃) δ 8.84 (s, 2H), 8.70 (s, 1H), 8.25 (d, J = 7.9 Hz, 1H), 8.20-8.16 (m, 2H), 7.69 (s, 1H), 7.46-7.44 (m, 1H), 7.15 (t, J = 8.1 Hz, 1H), 7.00 (dd, J = 7.6, 1.5 Hz, 1H), 6.94 (dd, J = 8.5, 3.4 Hz, 1H), 6.89 (d, J = 1.4 Hz, 1H), 6.80 (dd, J = 7.9, 1.2 Hz, 1H), 4.81 (d, J = 11.9 Hz, 1H), 4.66 (t, J = 7.7 Hz, 1H), 4.57 (dd, J = 13.9, 6.3 Hz, 1H), 4.53-4.51 (m, 1H), 4.40 (dd, J = 13.9, 5.9 Hz, 1H), 4.25-4.23 (m, 1H), 4.17-4.15 (m, 1H), 4.02-3.99 (m, 1H), 3.95-3.91 (m, 1H), 3.78-3.76 (m, 1H), 3.59 (dd, J = 11.2, 3.9 Hz, 1H), 3.42-3.38 (m, 1H), 2.94-2.90 (m, 2H), 2.61 (s, 2H), 2.59-2.54 (m, 3H), 2.46-2.44 (m, 2H), 2.06-2.03 (m, 1H), 1.84-1.78 (m, 4H), 1.60-1.55 (m, 3H), 1.50-1.47 (m, 2H), 1.40-1.36 (m, 2H), 1.35-1.30 (m, 2H), 1.29-1.21 (m, 4H), 1.19-1.14 (m, 2H), 1.05 (s, 3H), 0.91 (s, 9H). m/z 1196.60 [M+H]⁺ |
| 90 | ¹H NMR (600 MHz, CDCl₃) δ 8.70 (s, 1H), 8.25-8.16 (m, 4H), 7.70 (s, 1H), 7.37 (d, J = 7.7 Hz, 1H), 7.16-7.13 (m, 1H), 7.05 (d, J = 7.3 Hz, 1H), 7.02 (d, J = 7.6 Hz, 1H), 6.94 (s, 1H), 6.79 (d, J = 7.8 Hz, 1H), 4.71-4.69 (m, 1H), 4.63 (d, J = 13.3 Hz, 2H), 4.57-4.51 (m, 2H), 4.43-4.36 (m, 3H), 4.02 (d, J = 11.0 Hz, 1H), 3.94-3.91 (m, 2H), 3.62 (dd, J = 11.2, 3.7 Hz, 1H), 3.42-3.38 (m, 2H), 2.88-2.83 (m, 2H), 2.61 (s, 1H), 2.56-2.51 (m, 3H), 2.47-2.44 (m, 2H), 2.09-2.04 (m, 2H), 1.83-1.79 (m, 4H), 1.60-1.55 (m, 3H), 1.49-1.47 (m, 2H), 1.39-1.31 (m, 4H), 1.29-1.27 (m, 2H), 1.23-1.17 (m, 2H), 1.05 (s, 3H), 0.94 (s, 9H). m/z 1169.52 [M+H]⁺ |

### Evaluation of the compounds of the present invention

### Experimental Example 1: Measurement of SHP2 protein degradation in KYSE-520 cells

KYSE-520 cells were treated with the synthesized Example compound, and the amount of SHP2 protein present in the cells was measured using western blotting detection. The protocol of the experiment conducted using KYSE-520 cells was as follows:
[Culture] KYSE-520 cells resuspended in RPMI1640 MEDIUM (Hyclone, SH30027.01) containing 5% FBS (Hyclone, SV30207.02) and 1% Penicillin-streptomycin (Welgene, LS 202-02) were seeded at a density of 2.5 × 10⁵/well in a 6-well plate in an amount of 2 ml each and incubated for 2 days.
[Compound treatment] Cells were treated with compounds at a final concentration of 0.1, 1, 10 mM or 0.001, 0.01, 0.1 mM made by serial dilution of 10 mM stock with DMSO in 1/10 increments (3 mL + 27 mL DMSO). After 24 hours, cells were harvested. For the negative control group, DMSO diluted 1/10 to the media (3 mL DMSO + 27 mL media) was added.
[Cell Lysis and Protein Quantification] Lysis buffer was prepared as follows: 60 mL of RIPA buffer (Biosesang, RC2038-050-00) + 0.5 mM PMSF (SIGMA, P7626) + 1x Protease/Phosphatase Inhibitor (Cell signaling, 5872S) per well was added and placed on ice for 30 minutes (vortexing at 0 and 30 minutes, respectively). After sonication process (10 seconds pulse, 30 seconds rest, 5 cycles, 70% amplification), centrifugation (15000 g, 15 min, 4 °C) was performed, and then only the supernatant was taken and transferred to a new microtube. The sample was diluted 1/4 with RIPA buffer in a 96-well plate, and mixed with A:B = 50:1 of the BCA Protein Assay Kit (iNtRON, 21071). 200 ml each of the mixture was left at 37 °C for 30 minutes, and cooled for 10 minutes. Then, absorbance was measured at 562 nm with the SYNERGY H1 microplate reader from BioTek. After making a sample by quantifying the protein with the measured value, boiling was performed at 70 °C for 10 minutes. At this time, the sample buffer used was a mixture of NuPAGE or Bolt 4x sample buffer (Invitrogen) and each 10x sample reducing agent according to the gel to be used, and RIPA buffer was used for protein dilution.
[Western blotting detection method] The same amount of sample was loaded on NuPAGE or Bolt Bis-tris 4-12% gradient gel and run (200 V, 35 minutes). It was transferred to 0.2 mm NC membrane with Trans-blot Turbo (BIO-RAD) (1.3 A constant, 25 V limit, 15 minutes). Blocking was performed at room temperature for 1 hour with Skim milk or Intercept Blocking Buffer (LI-COR, 927-60001):0.1% TBST = 1:1. Anti SHPTP2 Mouse (1:500 in 5% Skim milk/TBST, size: 70 kDa, Santacruz sc-7384) was incubated overnight at 4 °C, and Anti GAPDH Rabbit (1:2,000 in 5% BSA/0.2% TBST, size: 37 kDa, CST #2118) was incubated at room temperature for 1 hour 30 minutes or 3 hours. It was washed 3 times for 5 minutes each with 0.2% TBST washing buffer. Secondary antibody Anti-Rabbit IgG (1:5,000 in 5% Skim milk/0.2% TBST, CST), and IRDye^{®} 680RD Goat anti-Rabbit IgG Secondary Antibody (1:10,000 in 5% BSA/0.2% TBST, LI -COR 926-68071) were incubated for 45 minutes on a rocker at room temperature. It was washed 5 times for 5 minutes each with 0.2% TBST washing buffer and detected with LI-COR's Odyssey. At this time, SHPTP2 was detected using SuperSignal West Pico PLUS Chemiluminescent Substrate, and house-keeping genes were also detected.

### Experimental Example 2: Measurement of SHP2 protein degradation in MV-4-11 cells

MV-4-11 cells were treated with the synthesized Example compound, and the amount of SHP2 protein present in the cells was measured using western blotting detection. The protocol of the experiment conducted using MV-4-11 cells was as follows:
[Culture] MV-4-11 cells resuspended in IMDM MEDIUM (Hyclone, SH30228.01) containing 5% FBS (Hyclone, SV30207.02) and 1% Penicillin-streptomycin (Welgene, LS 202-02) were seeded at a density of 1 × 10⁶/mL/well in a 12-well plate in an amount of 1 ml each.
[Compound treatment] The 12-well plate was treated as follows: Cells were treated with compounds at a final concentration of 0.01, 0.1, 1 mM or 0.001, 0.01, 0.1 mM made by serial dilution of 10 mM stock with DMSO in 1/100 increments (3 mL + 297 mL DMSO). After 24 hours, cells were harvested. For the negative control group, DMSO diluted 1/100 to the media (3 mL DMSO + 297 mL media) was added.
[Harvest] Each well was collected in a 1.5 mL microtube by pipetting with a 1 mL pipette and centrifuged (500 g, 5 min, 4 °C). The supernatant was removed. It was washed with PBS, and then centrifuged again (500 g, 5 min, 4 °C). Thereafter, the supernatant was removed to prepare a pellet.
[Cell Lysis and Sample preparation] Lysis buffer was prepared as follows: 50 mL of RIPA buffer (Biosesang, RC2038-050-00) + 0.5 mM PMSF (SIGMA, P7626) + 1x Protease/Phosphatase Inhibitor (Cell signaling, 5872S) per well was added and placed on ice for 30 minutes (vortexing at 0 and 30 minutes, respectively). After sonication process (10 seconds pulse, 30 seconds rest, 5 cycles, 70% amplification), centrifugation (15000 g, 15 min, 4 °C) was performed, and then only the supernatant was taken and transferred to a microtube. The sample was diluted 1/4 with RIPA buffer in a 96-well plate, and mixed with A:B = 50:1 of the BCA Protein Assay Kit (iNtRON, 21071). 200 ml each of the mixture was left at 37 °C for 30 minutes, and cooled for 10 minutes. Then, absorbance was measured at 562 nm with the SYNERGY H1 microplate reader from BioTek. After making a sample by quantifying the protein with the measured value, boiling was performed at 70 °C for 10 minutes. At this time, the sample buffer used was a mixture of NuPAGE or Bolt 4x sample buffer (Invitrogen) and each 10x sample reducing agent according to the gel to be used, and RIPA buffer was used for protein dilution.
[Western blotting detection method] The same amount of sample was loaded on NuPAGE or Bolt Bis-tris 4-12% gradient gel and run (200 V, 35 minutes). It was transferred to 0.2 mm NC membrane with Trans-blot Turbo (BIO-RAD) (1.3 A constant, 25 V limit, 15 minutes). Blocking was performed at room temperature for 1 hour with Skim milk or Intercept Blocking Buffer (LI-COR, 927-60001):0.1% TBST = 1:1. Anti-SHPTP2 Mouse (1:500 in 2% BSA/0.2% TBST, size: 70 kDa, Santacruz sc-7384) was incubated overnight at 4 °C, and Anti-GAPDH Rabbit (1:2,000 in 2% BSA/0.2% TBST, size: 37 kDa, CST #2118) was incubated at room temperature for 1 hour 30 minutes or 3 hours. It was washed 3 times for 5 minutes each with 0.2% TBST washing buffer. Secondary antibody Anti-Rabbit IgG (1:5,000 in 2% BSA/0.2% TBST, CST), IRDye^{®} 680RD Goat anti-Rabbit IgG Secondary Antibody (1:10,000 in 2% BSA/0.2% TBST, LI-COR 926-68071) were added and incubated for 45 minutes on a rocker at room temperature. It was washed 5 times for 5 minutes each with 0.2% TBST washing buffer and detected with LI-COR's Odyssey. At this time, SHPTP2 was detected using SuperSignal West Pico PLUS Chemiluminescent Substrate, and house-keeping genes were also detected.

The results of the SHP2 protein degradation by the compounds of the present invention are summarized in Table 4 below. In the results of Table 4 below, Compounds 1-7, 13-19, and 21-26 are results in KYSE-520 cells, and the others are results in MV-4-11 cells.

**[Table 4]**

| Example no. (Compound no.) | SHP2 degradation (%) | | |
|---|---|---|---|
| | 10 nM | 100 nM | 1000 nM |
| 1 | 28.9 | 64.5 | 74.5 |
| 2 | 23.3 | 81.8 | 94.3 |
| 3 | -15.6 | 48.8 | 84.0 |
| 4 | 4.1 | 60.7 | 90.7 |
| 5 | 3.4 | 56.7 | 90.6 |
| 6 | 19.5 | 82.4 | 95.0 |
| 7 | 25.4 | 75.2 | 93.8 |
| 8 | 39.5 | 92.6 | 96.5 |
| 9 | 39.6 | 93.3 | 95.6 |
| 10 | 33.7 | 90.9 | 95.0 |
| 11 | -8.9 | 19.2 | 35.9 |
| 12 | 23.9 | 88.4 | 93.3 |
| 13 | -6.1 | 49.6 | 69.2 |
| 14 | 14.2 | 60.1 | 69.1 |
| 15 | 21.7 | 58.3 | 74.6 |
| 16 | -24.3 | 45.1 | 84.0 |
| 17 | | 71.8 | 94.9 |
| 18 | -9.0 | 56.9 | 91.8 |
| 19 | | 31.5 | 78.0 |
| 20 | 54.9 | 99.0 | |
| 21 | 11.7 | 51.7 | 66.6 |
| 22 | 13.7 | 34.9 | 69.6 |
| 23 | 8.4 | 44.3 | 64.2 |
| 24 | 15.1 | 52.1 | 74.7 |
| 25 | | 61.8 | 94.7 |
| 26 | 40.6 | 83.2 | 95.0 |
| 27 | 23.5 | 72 | 91.5 |
| 28 | 19 | 76.5 | 94 |
| 29 | 84 | 95.5 | |
| 30 | 71.5 | 95.5 | |
| 31 | 76 | 97 | 97.5 |
| 32 | 75 | 95 | |
| 33 | 90 | 96.5 | |
| 34 | 77 | 93.5 | 94 |
| 35 | 74 | 93 | 93 |
| 36 | 83.0 | 93.5 | 93.5 |
| 37 | 80.5 | 89.5 | 90.5 |
| 38 | 86 | 93 | 93.5 |
| 39 | 51.0 | 94.5 | |
| 40 | 67.0 | 95.0 | |
| 41 | 77.0 | 92.5 | 91.5 |
| 42 | 73.5 | 95.0 | |
| 43 | 80.0 | 96.5 | |
| 44 | 71.5 | 95 | 95.5 |
| 45 | 68.0 | 91.0 | 93.0 |
| 46 | 0.1 | 52.3 | |
| 47 | -2.5 | 21.1 | |
| 48 | 18.6 | 57.3 | |
| 49 | 4.0 | 10.6 | |
| 50 | 91.5 | 98.4 | |
| 51 | 4.7 | 74.1 | |
| 52 | 72 | 96 | 94.5 |
| 53 | 83.9 | 97.8 | |
| 54 | 85.8 | 98.3 | |
| 55 | 85.9 | 97.8 | |
| 56 | 91.4 | 98.9 | |
| 57 | 95.7 | 99.8 | 99.7 |
| 58 | 90.0 | 99.9 | |
| 59 | -2.0 | 3.0 | |
| 60 | 59 | 94 | 95.5 |
| 61 | 87.3 | 99.4 | |
| 62 | 82.5 | 96 | |
| 63 | 90.5 | 97 | |
| 64 | 45.9 | 96.6 | |
| 65 | 31.6 | 95.4 | |
| 66 | 79 | 98.1 | |
| 67 | 68.3 | 98.1 | |
| 68 | 83.3 | 98.3 | |
| 69 | 20 | 81.9 | |
| 70 | 14.4 | 59.2 | |
| 71 | -1.0 | 29.0 | 87.5 |
| 72 | 5.1 | 85.6 | |
| 73 | 8.2 | 81.2 | |
| 74 | 29.0 | 80.5 | 94.5 |
| 75 | 19.5 | 75.5 | 93.5 |
| 76 | 94.2 | 99.5 | |
| 77 | 92.4 | 99.5 | |
| 78 | 86.6 | 99.5 | |
| 79 | 78.4 | 98.6 | |
| 80 | 83.1 | 98.2 | |
| 81 | 22.7 | 93.2 | |
| 82 | 16.2 | 96.2 | |
| 83 | 79.9 | 97.5 | |
| 84 | 48.7 | 97.1 | |
| 85 | 73 | 97.5 | |
| 86 | -29.2 | 23.8 | |
| 87 | 9.1 | 45.5 | |
| 88 | 16.5 | 66.1 | |
| 89 | 4 | 95 | 97 |
| 90 | 6 | 97 | 98 |

As shown in Table 4, the compounds according to the present invention exhibited excellent activity in SHP2 protein degradation.

In particular, Compounds 20, 29-45, 50, 52-58, 60-64, 66-68, 76-80, and 83-85 of the present invention exhibit SHP2 protein degradation activity superior to other compounds having a similar structure. Compounds 29, 31, 33, 36-38, 43, 50, 52-58, 61-63, 66, 68, 76-80, and 83 of the present invention showed significantly superior SHP2 protein degradation activity than other compounds.

On the other hand, when there are two substituents or cyclopropyl substitution on the carbon present between the two carbonyl groups of the malonamide included in the 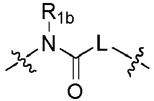 part in Chemical Formula 1 of the present invention (for example, Compounds 46, 47, 48, 49, 59), the degradation activity was somewhat decreased. Accordingly, in an even more preferred embodiment of the present invention, compounds having two substituents or cyclopropyl substitution on the carbon present between two carbonyl groups of the malonamide structure included in the 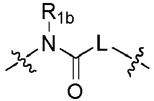 moiety in Chemical Formula 1 of the present invention are excluded.

## Claims

1. A compound of Chemical Formula 1: 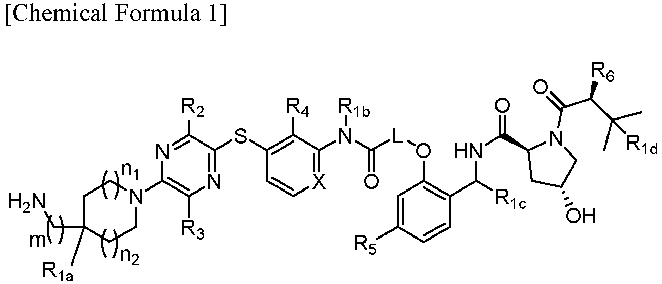 or a pharmaceutically acceptable salt thereof,
in the Chemical Formula 1
R₁ₐ, R_{1b}, R_{1c}, and R_{1d} are each independently H or C₁₋₆alkyl,
R₂ is H, C₁₋₆alkyl, or -NH₂,
R₃ is H, C₁₋₆alkyl, or C₁₋₆hydroxyalkyl,
R₄ is H, C₁₋₆alkyl, halogen, or C₁₋₆haloalkyl,
R₅ is H, C₁₋₆alkyl, C₁₋₆alkynyl, halogen, -CN, or heteroaryl, wherein one or more hydrogens in the heteroaryl are optionally substituted with C₁₋₆alkyl, halogen, or C₁₋₃haloalkyl,
R₆ is -NHC(O)R₇ or heteroaryl, wherein one or more hydrogens in the heteroaryl are optionally substituted with C₁₋₆alkyl, halogen, or C₁₋₃haloalkyl,
R₇ is C₁₋₆alkyl, C₁₋₆haloalkyl, C₃₋₁₀cycloalkyl, or heterocycle, wherein one or more hydrogens in the cycloalkyl and heterocycle are each independently and optionally substituted with C₁₋₆alkyl, halogen, or -CN,
X is CH or N,
n₁ and n₂ are each independently 0, 1, or 2,
m is 0 or 1,
L is the following Chemical Formula 2, 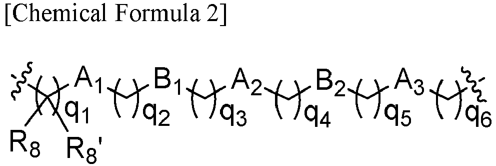 in the Chemical Formula 2,
A₁, A₂, and A₃ are each independently direct bond, -O-, -N(R₉)- -C(O)-, -C(O)N(R₉)-, or -N(R₉)C(O)-,
B₁ and B₂ are each independently direct bond, C₃₋₁₀cycloalkyl, heterocycle, aryl, or heteroaryl, wherein one or more hydrogens in the cycloalkyl, heterocycle, aryl, or heteroaryl are optionally substituted with C₁₋₆alkyl, halogen, C₁₋₆haloalkyl, -OH, =O, or -CO₂R₉,
R₈ and R₈' are each independently H or C₁₋₆alkyl, or R₈ and R₈' are linked to each other to form C₃₋₁₀cycloalkyl,
R₉ is H or C₁₋₆alkyl,
q₁, q₂, q₃, q₄, and q₅ are each independently an integer of from 0 to 10.

2. The compound of Claim 1 or a pharmaceutically acceptable salt thereof, in the Chemical Formula 1,
R₁ₐ, R_{1b}, R_{1c}, and R_{1d} are each independently H or C₁₋₆alkyl,
R₂ is H, C₁₋₆alkyl, or -NH₂,
R₃ is H, C₁₋₆alkyl, or C₁₋₆hydroxyalkyl,
R₄ is H, C₁₋₆alkyl, or halogen,
R₅ is heteroaryl, wherein one or more hydrogens in the heteroaryl are optionally substituted with C₁₋₆alkyl or halogen,
R₆ is -NHC(O)R₇,
R₇ is C₁₋₆alkyl or C₃₋₁₀cycloalkyl, wherein one or more hydrogens in the cycloalkyl are optionally substituted with C₁₋₆alkyl, halogen, or -CN,
X is CH or N,
n₁ and n₂ is each independently 0 or 1,
m is 0 or 1,
L is the following Chemical Formula 2, 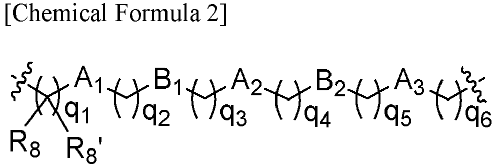 in the Chemical Formula 2,
A₁, A₂, and A₃ are each independently direct bond, -O-, -N(R₉)- -C(O)-, -C(O)N(R₉)-, or -N(R₉)C(O)-,
B₁ and B₂ are each independently direct bond, heterocycle, or heteroaryl, wherein one or more hydrogens in the heterocycle or heteroaryl are optionally substituted with C₁₋₆alkyl, halogen, -OH, =O, or -CO₂R₉,
R₈ and R₈' are each independently H or C₁₋₆alkyl, or R₈ and R₈' are linked to each other to form C₃₋₁₀cycloalkyl,
R₉ is H or C₁₋₆alkyl,
q₁, q₂, q₃, q₄, and q₅ are each independently an integer of from 0 to 10.

3. The compound of Claim 1 or a pharmaceutically acceptable salt thereof, in the Chemical Formula 1,
R₁ₐ, R_{1b}, R_{1c}, and R_{1d} are each independently H or C₁₋₆alkyl,
R₂ is H or -NH₂,
R₃ is H,
R₄ is H or halogen,
R₅ is thiazole, wherein one or more hydrogens in the thiazole are optionally substituted with C₁₋₆alkyl,
R₆ is -NHC(O)R₇,
R₇ is C₁₋₆alkyl or cyclopropyl, wherein one or more hydrogens in the cyclopropyl are optionally substituted with halogen or -CN,
X is CH,
n₁ and n₂ are each independently 0 or 1,
m is 0 or 1,
L is the following Chemical Formula 2, 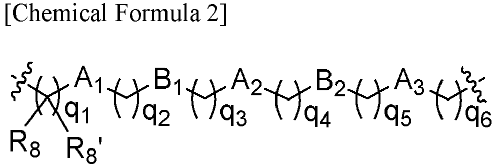 in the Chemical Formula 2,
A₁, A₂, and A₃ are each independently direct bond, -O-, -N(R₉)- -C(O)-, -C(O)N(R₉)-, or -N(R₉)C(O)-,
B₁ and B₂ are each independently direct bond, azetidine, pyrrolidine, piperidine, piperazine, azaspiro[3.5]nonane, diazabicyclo[3.2.1]octane, pyridine, pyrimidine, pyridazine, or pyrazine, wherein one or more hydrogens in the azetidine, pyrrolidine, piperidine, piperazine, azaspiro[3.5]nonane, diazabicyclo[3.2.1]octane, pyridine, pyrimidine, pyridazine, or pyrazine are optionally substituted with C₁₋₆alkyl, -OH, =O, or -CO₂R₉,
at least one of B₁ and B₂ is azetidine, pyrrolidine, piperidine, piperazine, azaspiro[3.5]nonane, diazabicyclo[3.2.1]octane, pyridine, pyrimidine, pyridazine, or pyrazine,
R₈ and R₈' are each independently H or C₁₋₆alkyl, or R₈ and R₈' are linked to each other to form C₃₋₁₀cycloalkyl,
R₉ is H or C₁₋₆alkyl,
q₁, q₂, q₃, q₄, and q₅ are each independently an integer of from 0 to 10.

4. The compound of Claim 1 or a pharmaceutically acceptable salt thereof, wherein L is any one of the followings:

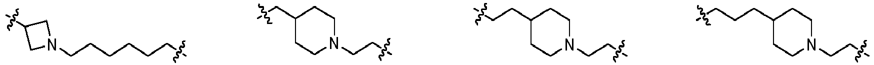

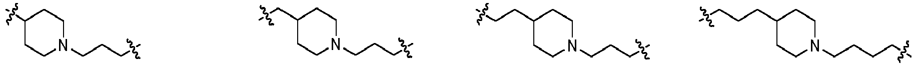

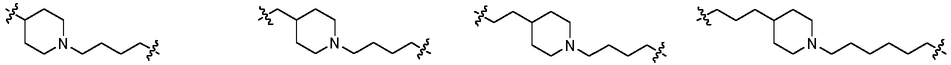

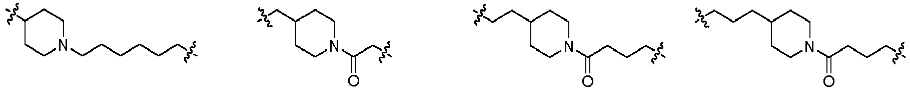

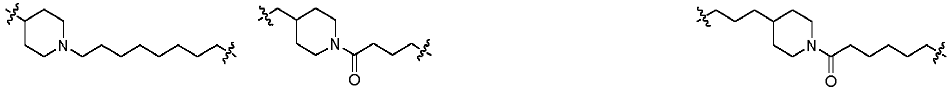

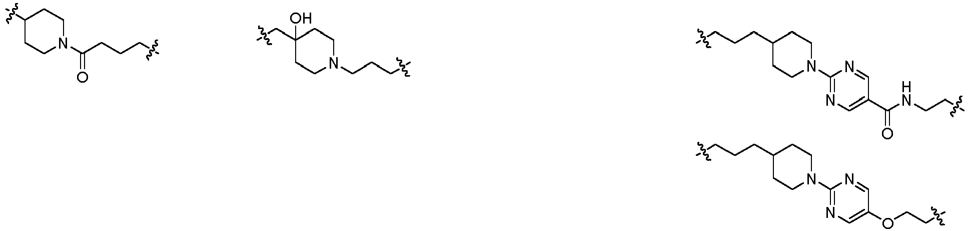

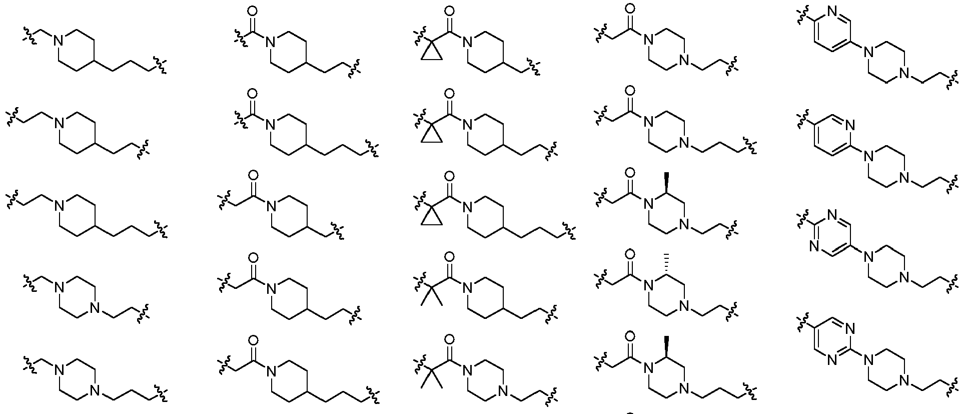

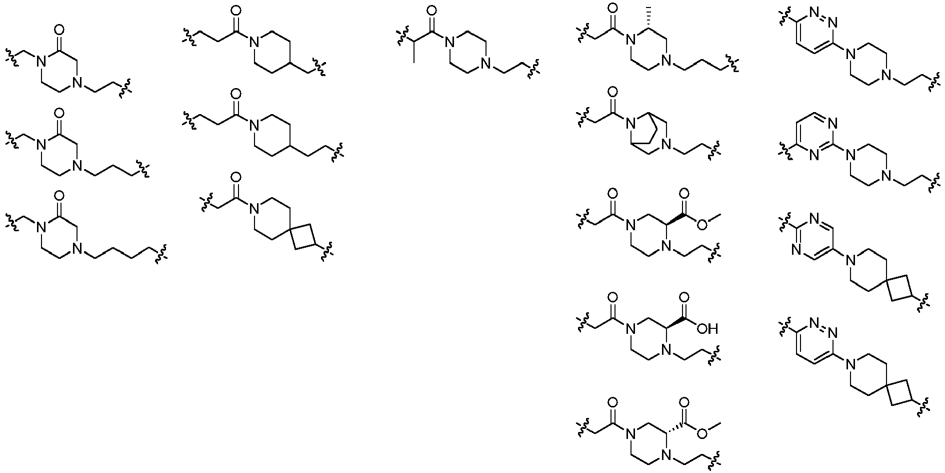

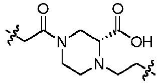

5. The compound of Claim 1 or a pharmaceutically acceptable salt thereof, wherein the compound is any one of the following compounds:
| Comp ound no. | IUPAC Name |
|---|---|
| 1 | N-(3-((5-(4-(aminomethyl)-4-methylpiperidin-1-yl)pyrazin-2-yl)thio)-2-chlorophenyl)-1-(3-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)propyl)piperidine-4-carboxamide |
| 2 | N-(3-((5-(4-(aminomethyl)-4-methylpiperidin-1-yl)pyrazin-2-yl)thio)-2-chlorophenyl)-1-(4-(2-(((2S,4R)-1 -((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butyl)piperidine-4-carboxamide |
| 3 | N-(3-((5-(4-(aminomethyl)-4-methylpiperidin-1-yl)pyrazin-2-yl)thio)-2-chlorophenyl)-1-(6-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)hexyl)piperidine-4-carboxamide |
| 4 | N-(3-((5-(4-(aminomethyl)-4-methylpiperidin-1-yl)pyrazin-2-yl)thio)-2-chlorophenyl)-1-(8-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)octyl)piperidine-4-carboxamide |
| 5 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 6 | (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 7 | (2S,4R)-N-(2-(4-(4-(2-((3-((5-(4-(aminomethyl)-4-methylpiperidin-1-yl)pyrazin-2-yl)thio)-2-chlorophenyl)amino)-2-oxoethyl)piperidin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 8 | (2S,4R)-N-(2-(3-(4-(2-((3-((5-(4-amino-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 9 | (2S,4R)-N-(2-(3-(4-(2-((3-((3-amino-5-(4-amino-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 10 | (2S,4R)-N-(2-(3-(4-(2-((3-((3-amino-5-(4-amino-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 11 | (2S,4R)-N-(2-(3-(4-(2-((3-((5-((R)-3-(aminomethyl)pyrrolidin-1-yl)pyrazin-2-yl)thio)-2-chlorophenyl)amino)-2-oxoethyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 12 | (2S,4R)-1-((S)-2-acetamido-3,3-dimethylbutanoyl)-N-(2-(3-(4-(2-((3-((5-(4-(aminomethyl)-4-methylpiperidin-1-yl)pyrazin-2-yl)thio)-2-chlorophenyl)amino)-2-oxoethyl)piperidin-1-yl)propoxy)-4-(4-methylthiazol-5-yl)benzyl)-4-hydroxypyrrolidine-2-carboxamide |
| 13 | (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 14 | (2S,4R)-N-(2-(3-(4-(3-((3-((5-(4-(aminomethyl)-4-methylpiperidin-1-yl)pyrazin-2-yl)thio)-2-chlorophenyl)amino)-3-oxopropyl)piperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 15 | (2S,4R)-N-(2-(4-(4-(3-((3-((5-(4-(aminomethyl)-4-methylpiperidin-1-yl)pyrazin-2-yl)thio)-2-chlorophenyl)amino)-3-oxopropyl)piperidin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 16 | (2S,4R)-N-(2-(2-(4-(4-((3-((5-(4-(aminomethyl)-4-methylpiperidin-1-yl)pyrazin-2-yl)thio)-2-chlorophenyl)amino)-4-oxobutyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 17 | (2S,4R)-N-(2-(4-(4-(4-((3-((5-(4-(aminomethyl)-4-methylpiperidin-1-yl)pyrazin-2-yl)thio)-2-chlorophenyl)amino)-4-oxobutyl)piperidin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 18 | (2S,4R)-N-(2-((6-(4-(4-((3-((5-(4-(aminomethyl)-4-methylpiperidin-1-yl)pyrazin-2-yl)thio)-2-chlorophenyl)amino)-4-oxobutyl)piperidin-1-yl)hexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 19 | (2S,4R)-N-(2-((6-(3-((3-((5-(4-(aminomethyl)-4-methylpiperidin-1-yl)pyrazin-2-yl)thio)-2-chlorophenyl)carbamoyl)azetidin-1-yl)hexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 20 | ((2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-4-hydroxypiperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 21 | N-(3-((5-(4-(aminomethyl)-4-methylpiperidin-1-yl)pyrazin-2-yl)thio)-2-chlorophenyl)-1-(4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)butanoyl)piperidine-4-carboxamide |
| 22 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)piperidin-1-yl)-2-oxoethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 23 | (2S,4R)-N-(2-(4-(4-(2-((3-((5-(4-(aminomethyl)-4-methylpiperidin-1-yl)pyrazin-2-yl)thio)-2-chlorophenyl)amino)-2-oxoethyl)piperidin-1-yl)-4-oxobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 24 | (2S,4R)-N-(2-(4-(4-(3-((3-((5-(4-(aminomethyl)-4-methylpiperidin-1-yl)pyrazin-2-yl)thio)-2-chlorophenyl)amino)-3-oxopropyl)piperidin-1-yl)-4-oxobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 25 | (2S,4R)-N-(2-(4-(4-(4-((3-((5-(4-(aminomethyl)-4-methylpiperidin-1-yl)pyrazin-2-yl)thio)-2-chlorophenyl)amino)-4-oxobutyl)piperidin-1-yl)-4-oxobutoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 26 | (2S,4R)-N-(2-((6-(4-(4-((3-((5-(4-(aminomethyl)-4-methylpiperidin-1-yl)pyrazin-2-yl)thio)-2-chlorophenyl)amino)-4-oxobutyl)piperidin-1-yl)-6-oxohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 27 | (2S,4R)-N-(2-(2-(1-(2-((3-((5-(4-(aminomethyl)-4-methylpiperidin-1-yl)pyrazin-2-yl)thio)-2-chlorophenyl)amino)-2-oxoacetyl)piperidin-4-yl)ethoxy)-4-(4-methylthiazol-5-yl)benzyl)-1-((8)-2-(1-fluorocyclopropane-1-carboxamido)-3,3-dimethylbutanoyl)-4-hydroxypyrrolidine-2-carboxamide |
| 28 | (2S,4R)-N-(2-(3-(1-(2-((3-((5-(4-(aminomethyl)-4-methylpiperidin-1-yl)pyrazin-2-yl)thio)-2-chlorophenyl)amino)-2-oxoacetyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 29 | (2S,4R)-N-(2-((1-(3-((3-((5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 30 | (2S,4R)-N-(2-((1-(3-((3-((3-amino-5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 31 | (2S,4R)-N-(2-((1-(3-((3-((5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 32 | (2S,4R)-N-(2-((1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 33 | (2S,4R)-1-((S)-2-acetamido-3,3-dimethylbutanoyl)-N-(2-((1-(3-((3-((5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-4-hydroxypyrrolidine-2-carboxamide |
| 34 | (2S,4R)-N-(2-(2-(1-(3-((3-((5-(4-amino-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 35 | (2S,4R)-N-(2-(2-(1-(3-((3-((3-amino-5-(4-amino-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 36 | (2S,4R)-N-(2-(2-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 37 | (2S,4R)-N-(2-(2-(1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 38 | (2S,4R)-1-((S)-2-acetamido-3,3-dimethylbutanoyl)-N-(2-(2-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-4-hydroxypyrrolidine-2-carboxamide |
| 39 | (2S,4R)-N-(2-((1-(3-((3-((5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 40 | (2S,4R)-N-(2-(3-(1-(3-((3-((3-amino-5-(4-amino-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 41 | (2S,4R)-N-(2-(3-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 42 | (2S,4R)-N-(2-(3-(1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 43 | (2S,4R)-1-((S)-2-acetamido-3,3-dimethylbutanoyl)-N-(2-(3-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-4-hydroxypyrrolidine-2-carboxamide |
| 44 | (2S,4R)-N-(2-((1-(4-((3-((5-(4-(aminomethyl)-4-methylpiperidin-1-yl)pyrazin-2-yl)thio)-2-chlorophenyl)amino)-4-oxobutanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 45 | (2S,4R)-N-(2-(2-(1-(4-((3-((5-(4-(aminomethyl)-4-methylpiperidin-1-yl)pyrazin-2-yl)thio)-2-chlorophenyl)amino)-4-oxobutanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 46 | (2S,4R)-N-(2-((1-(1-((3-((5-(4-(aminomethyl)-4-methylpiperidin-1-yl)pyrazin-2-yl)thio)-2-chlorophenyl)carbamoyl)cyclopropane-1-carbonyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 47 | (2S,4R)-N-(2-(2-(1-(1-((3-((5-(4-(aminomethyl)-4-methylpiperidin-1-yl)pyrazin-2-yl)thio)-2-chlorophenyl)carbamoyl)cydopropane-1-carbonyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 48 | (2S,4R)-N-(2-(3-(1-(1-((3-((5-(4-(aminomethyl)-4-methylpiperidin-1-yl)pyrazin-2-yl)thio)-2-chlorophenyl)carbamoyl)cyclopropane-1-carbonyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 49 | (2S,4R)-N-(2-(2-(1-(3-((3-((5-(4-(aminomethyl)-4-methylpiperidin-1-yl)pyrazin-2-yl)thio)-2-chlorophenyl)amino)-2,2-dimethyl-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 50 | (2S,4R)-N-(2-((7-(3-((3-((5-(4-(aminomethyl)-4-methylpiperidin-1-yl)pyrazin-2-yl)thio)-2-chlorophenyl)amino)-3-oxopropanoyl)-7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 51 | (2S,4R)-N-(2-((7-(3-((3-((5-(4-(aminomethyl)-4-methylpiperidin-1-yl)pyrazin-2-yl)thio)-2-chlorophenyl)(methyl)amino)-3-oxopropanoyl)-7-azaspiro[3.5]nonan-2-yl)oxy)-4-(4-methylthiazol-5-yl)benzyl)-1-((S)-2-(1-fluorocydopropane-1-carboxamido)-3,3-dimethylbutanoyl)-4-hydroxypyrrolidine-2-carboxamide |
| 52 | (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 53 | (2S,4R)-N-(2-(2-(4-(3-((3-((5-(4-amino-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 54 | (2S,4R)-N-(2-(2-(4-(3-((3-((3-amino-5-(4-amino-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 55 | (2S,4R)-N-(2-(2-(4-(3-((3-((3-amino-5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 56 | (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(cyclopropanecarboxamido)-3,3-dimethylbutanoyl)-4-hydroxypyrrolidine-2-carboxamide |
| 57 | (2S,4R)-1-((S)-2-acetamido-3,3-dimethylbutanoyl)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-4-hydroxypyrrolidine-2-carboxamide |
| 58 | (2S,4R)-N-(2-(2-(4-(3-((3-((5-(4-(aminomethyl)-4-methylpiperidin-1-yl)pyrazin-2-yl)thio)-2-chlorophenyl)amino)-2-methyl-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 59 | (2S,4R)-N-(2-(2-(4-(3-((3-((5-(4-(aminomethyl)-4-methylpiperidin-1-yl)pyrazin-2-yl)thio)-2-chlorophenyl)amino)-2,2-dimethyl-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 60 | (2S,4R)-N-(2-(3-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 61 | (2S,4R)-1-((S)-2-acetamido-3,3-dimethylbutanoyl)-N-(2-(3-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)propoxy)-4-(4-methylthiazol-5-yl)benzyl)-4-hydroxypyrrolidine-2-carboxamide |
| 62 | (2S,4R)-N-(2-(2-((R)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 63 | (2S,4R)-N-(2-(2-((S)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 64 | (2S,4R)-N-(2-(3-((S)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 65 | ((2S,4R)-N-(2-(3-((R)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 66 | (S)-methyl 4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate |
| 67 | methyl (R)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate |
| 68 | (2S,4R)-N-(2-(2-(8-(3-((3-((5-(4-(aminomethyl)-4-methylpiperidin-1-yl)pyrazin-2-yl)thio)-2-chlorophenyl)amino)-3-oxopropanoyl)-3,8-diazabicyclo[3.2.1]octan-3-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 69 | (S)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid |
| 70 | (R)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylic acid |
| 71 | (2S,4R)-N-(2-(3-(1-(2-((3-((5-(4-(aminomethyl)-4-methylpiperidin-1-yl)pyrazin-2-yl)thio)-2-chlorophenyl)amino)-2-oxoethyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 72 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 73 | (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)piperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 74 | (2S,4R)-N-(2-(2-(1-(3-((3-((5-(4-(aminomethyl)-4-methylpiperidin-1-yl)pyrazin-2-yl)thio)-2-chlorophenyl)amino)-3-oxopropyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 75 | (2S,4R)-N-(2-(3-(1-(3-((3-((5-(4-(aminomethyl)-4-methylpiperidin-1-yl)pyrazin-2-yl)thio)-2-chlorophenyl)amino)-3-oxopropyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 76 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 77 | (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1 -yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 78 | (2S,4R)-N-(2-(4-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 79 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(cyclopropanecarboxamido)-3,3-dimethylbutanoyl)-4-hydroxypyrrolidine-2-carboxamide |
| 80 | (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)propoxy)-4-(4-methylthiazol-5-yl)benzyl)-1-((S)-2-(cyclopropanecarboxamido)-3,3-dimethylbutanoyl)-4-hydroxypyrrolidine-2-carboxamide |
| 81 | N-(3-((5-(4-(aminomethyl)-4-methylpiperidin-1-yl)pyrazin-2-yl)thio)-2-chlorophenyl)-5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)picolinamide |
| 82 | N-(3-((5-(4-(aminomethyl)-4-methylpiperidin-1-yl)pyrazin-2-yl)thio)-2-chlorophenyl)-6-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)nicotinamide |
| 83 | N-(3-((5-(4-(aminomethyl)-4-methylpiperidin-1-yl)pyrazin-2-yl)thio)-2-chlorophenyl)-5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-2-carboxamide |
| 84 | N-(3-((5-(4-(aminomethyl)-4-methylpiperidin-1-yl)pyrazin-2-yl)thio)-2-chlorophenyl)-2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-5-carboxamide |
| 85 | N-(3-((5-(4-(aminomethyl)-4-methylpiperidin-1-yl)pyrazin-2-yl)thio)-2-chlorophenyl)-6-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyridazine-3-carboxamide |
| 86 | N-(3-((5-(4-(aminomethyl)-4-methylpiperidin-1-yl)pyrazin-2-yl)thio)-2-chlorophenyl)-2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-4-carboxamide |
| 87 | N-(3-((5-(4-(aminomethyl)-4-methylpiperidin-1-yl)pyrazin-2-yl)thio)-2-chlorophenyl)-5-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)-7-azaspiro[3.5]nonan-7-yl)pyrimidine-2-carboxamide |
| 88 | N-(3-((5-(4-(aminomethyl)-4-methylpiperidin-1-yl)pyrazin-2-yl)thio)-2-chlorophenyl)-6-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)-7-azaspiro[3.5]nonan-7-yl)pyridazine-3-carboxamide |
| 89 | 2-(4-(4-((3-((5-(4-(aminomethyl)-4-methylpiperidin-1-yl)pyrazin-2-yl)thio)-2-chlorophenyl)amino)-4-oxobutyl)piperidin-1-yl)-N-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)pyrimidine-5-carboxamide |
| 90 | (2S,4R)-N-(2-(2-((2-(4-(4-((3-((5-(4-(aminomethyl)-4-methylpiperidin-1 -yl)pyrazin-2-yl)thio)-2-chlorophenyl)amino)-4-oxobutyl)piperidin-1-yl)pyrimidin-5-yl)oxy)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |

6. The compound of Claim 5 or a pharmaceutically acceptable salt thereof, wherein the compound is any one of the following compounds:
| Comp ound no. | IUPAC Name |
|---|---|
| 20 | ((2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-4-hydroxypiperidin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 29 | (2S,4R)-N-(2-((1-(3-((3-((5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 30 | (2S,4R)-N-(2-((1-(3-((3-((3-amino-5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 31 | (2S,4R)-N-(2-((1-(3-((3-((5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 32 | (2S,4R)-N-(2-((1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 33 | (2S,4R)-1-((S)-2-acetamido-3,3-dimethylbutanoyl)-N-(2-((1-(3-((3-((5-(4-(aminomethyl)-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-4-hydroxypyrrolidine-2-carboxamide |
| 34 | (2S,4R)-N-(2-(2-(1-(3-((3-((5-(4-amino-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 35 | (2S,4R)-N-(2-(2-(1-(3-((3-((3-amino-5-(4-amino-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 36 | (2S,4R)-N-(2-(2-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 37 | (2S,4R)-N-(2-(2-(1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 38 | (2S,4R)-1-((S)-2-acetamido-3,3-dimethylbutanoyl)-N-(2-(2-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)ethoxy)-4-(4-methylthiazol-5-yl)benzyl)-4-hydroxypyrrolidine-2-carboxamide |
| 39 | (2S,4R)-N-(2-((1-(3-((3-((5-(4-amino-4-methylpiperidin-1-yl)pyrazin-2-yl)thio)-2-chlorophenyl)amino)-3-oxopropanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 40 | (2S,4R)-N-(2-(3-(1-(3-((3-((3-amino-5-(4-amino-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 41 | (2S,4R)-N-(2-(3-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 42 | (2S,4R)-N-(2-(3-(1-(3-((3-((3-amino-5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 43 | (2S,4R)-1-((S)-2-acetamido-3,3-dimethylbutanoyl)-N-(2-(3-(1-(3-((3-((5-(4-(aminomethyl)-4-methylpiperidin-1-yl)pyrazin-2-yl)thio)-2-chlorophenyl)amino)-3-oxopropanoyl)piperidin-4-yl)propoxy)-4-(4-methylthiazol-5-yl)benzyl)-4-hydroxypyrrolidine-2-carboxamide |
| 44 | (2S,4R)-N-(2-((1-(4-((3-((5-(4-(aminomethyl)-4-methylpiperidin-1-yl)pyrazin-2-yl)thio)-2-chlorophenyl)amino)-4-oxobutanoyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 45 | (2S,4R)-N-(2-(2-(1-(4-((3-((5-(4-(aminomethyl)-4-methylpiperidin-1 -yl)pyrazin-2-yl)thio)-2-chlorophenyl)amino)-4-oxobutanoyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 50 | (2S,4R)-N-(2-((7-(3-((3-((5-(4-(aminomethyl)-4-methylpiperidin-1-yl)pyrazin-2-yl)thio)-2-chlorophenyl)amino)-3-oxopropanoyl)-7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 52 | (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 53 | (2S,4R)-N-(2-(2-(4-(3-((3-((5-(4-amino-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 54 | (2S,4R)-N-(2-(2-(4-(3-((3-((3-amino-5-(4-amino-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 55 | (2S,4R)-N-(2-(2-(4-(3-((3-((3-amino-5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 56 | (2S,4R)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-1-((S)-2-(cyclopropanecarboxamido)-3,3-dimethylbutanoyl)-4-hydroxypyrrolidine-2-carboxamide |
| 57 | (2S,4R)-1-((S)-2-acetamido-3,3-dimethylbutanoyl)-N-(2-(2-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)ethoxy)-4-(4-methylthiazol-5-yl)benzyl)-4-hydroxypyrrolidine-2-carboxamide |
| 58 | (2S,4R)-N-(2-(2-(4-(3-((3-((5-(4-(aminomethyl)-4-methylpiperidin-1-yl)pyrazin-2-yl)thio)-2-chlorophenyl)amino)-2-methyl-3-oxopropanoyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 60 | (2S,4R)-N-(2-(3-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)propoxy)-4-(4-methylthiazol-5-yl)benzyl)-1-((S)-2-(1-fluorocyclopropane-1- carboxamido)-3,3-dimethylbutanoyl)-4-hydroxypyrrolidine-2-carboxamide |
| 61 | (2S,4R)-1-((S)-2-acetamido-3,3-dimethylbutanoyl)-N-(2-(3-(4-(3-((3-((5-(4-(aminomethyl)-4-methylpiperidin-1-yl)pyrazin-2-yl)thio)-2-chlorophenyl)amino)-3-oxopropanoyl)piperazin-1-yl)propoxy)-4-(4-methylthiazol-5-yl)benzyl)-4-hydroxypyrrolidine-2-carboxamide |
| 62 | (2S,4R)-N-(2-(2-((R)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 63 | (2S,4R)-N-(2-(2-((S)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 64 | (2S,4R)-N-(2-(3-((S)-4-(3-((3-((5-(4-(aminomethyl)-4-methylpiperidin-1-yl)pyrazin-2-yl)thio)-2-chlorophenyl)amino)-3-oxopropanoyl)-3-methylpiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 66 | (S)-methyl 4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocydopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate |
| 67 | methyl (R)-4-(3-((3-((5-(4-(aminomethyl)-4-methylpiperidin-1-yl)pyrazin-2-yl)thio)-2-chlorophenyl)amino)-3-oxopropanoyl)-1-(2-(2-(((2S,4R)-1-((S)-2-(1-fluorocydopropane-1-carboxamido)-3,3-dimethylbutanoyl)-4-hydroxypyrrolidine-2-carboxamido)methyl)-5-(4-methylthiazol-5-yl)phenoxy)ethyl)piperazine-2-carboxylate |
| 68 | (2S,4R)-N-(2-(2-(8-(3-((3-((5-(4-(aminomethyl)-4-methylpiperidin-1-yl)pyrazin-2-yl)thio)-2-chlorophenyl)amino)-3-oxopropanoyl)-3,8-diazabicyclo[3.2.1]octan-3-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 76 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 77 | (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 78 | (2S,4R)-N-(2-(4-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)butoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| 79 | (2S,4R)-N-(2-(2-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)ethoxy)-4-(4-methylthiazol-5-yl)benzyl)-1-((S)-2-(cyclopropanecarboxamido)-3,3-dimethylbutanoyl)-4-hydroxypyrrolidine-2-carboxamide |
| 80 | (2S,4R)-N-(2-(3-(4-(2-((3-((5-(4-(aminomethyl)-4-methylpiperidin-1-yl)pyrazin-2-yl)thio)-2-chlorophenyl)amino)-2-oxoethyl)-3-oxopiperazin-1-yl)propoxy)-4-(4-methylthiazol-5-yl)benzyl)-1-((S)-2-(cyclopropanecarboxamido)-3,3-dimethylbutanoyl)-4-hydroxypyrrolidine-2-carboxamide |
| 83 | N-(3-((5-(4-(aminomethyl)-4-methylpiperidin-1-yl)pyrazin-2-yl)thio)-2-chlorophenyl)-5-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-2-carboxamide |
| 84 | N-(3-((5-(4-(aminomethyl)-4-methylpiperidin-1-yl)pyrazin-2-yl)thio)-2-chlorophenyl)-2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyrimidine-5-carboxamide |
| 85 | N-(3-((5-(4-(aminomethyl)-4-methylpiperidin-1-yl)pyrazin-2-yl)thio)-2-chlorophenyl)-6-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)pyridazine-3-carboxamide |

7. A composition comprising a compound of any one of Claims 1 to 6 or a pharmaceutically acceptable salt thereof, and a pharmaceutically acceptable carrier.

8. A pharmaceutical composition for degrading or inhibiting Src homology 2 domain-containing phosphatase (SHP2), comprising as an effective agent a compound of any one of Claims 1 to 6 or a pharmaceutically acceptable salt thereof..

9. The pharmaceutical composition of Claim 8, wherein the pharmaceutical composition is for treating or preventing a cancer.

10. The pharmaceutical composition of Claim 9, wherein the cancer is leukemia, lymphoma, lung cancer, head and neck cancer, esophageal cancer, stomach cancer, colorectal cancer, pancreatic cancer, liver cancer, breast cancer, ovarian cancer, cervical cancer, bladder cancer, melanoma, neuroblastoma, glioma, or sarcoma.

11. A method for treating or preventing a Src homology 2 domain-containing phosphataserelated disease, comprising administering to a subject in need thereof a therapeutically effective amount of a compound of any one of Claims 1 to 6 or pharmaceutically acceptable salt thereof.

12. The method of Claim 11, wherein the Src homology 2 domain-containing phosphataserelated disease is cancer.

13. The method of Claim 12, wherein the cancer is leukemia, lymphoma, lung cancer, head and neck cancer, esophageal cancer, stomach cancer, colorectal cancer, pancreatic cancer, liver cancer, breast cancer, ovarian cancer, cervical cancer, bladder cancer, melanoma, neuroblastoma, glioma, or sarcoma.
